# EUROPÄISCHE PATENTSCHRIFT

(11) **EP 0 485 329 B1**
(45) Veröffentlichungstag und Bekanntmachung des Hinweises auf die Patenterteilung: **05.11.1997**
(21) Anmeldenummer: 91810815.0
(22) Anmeldetag: 22.10.1991
(51) Int. Cl.: C07D 498/04, C09B 19/02, C09B 62/002, D06P 1/38, D06P 1/40

(54) **Triphendioxazinverbindungen, Verfahren zu ihrer Herstellung und ihre Verwendung**
Triphenedioxazine compounds, process for their preparation, and their use
Composés de triphènedioxazine, procédé pour leur préparation et leur utilisation

(30) Priorität: 30.10.1990 CH 3443/90
(43) Veröffentlichungstag der Anmeldung: 13.05.1992
(73) Patentinhaber: Ciba Specialty Chemicals Holding Inc., 4057 Basel (CH)
(72) Erfinder: Lauk, Urs, Dr., CH-8047 Zürich (CH)

(56) Entgegenhaltungen:
- EP-A- 0 299 328
- EP-A- 0 356 014
- EP-A- 0 361 186
- GB-A- 2 228 738

## Beschreibung

Die vorliegende Erfindung betrifft Triphendioxazinverbindungen, Verfahren zu ihrer Herstellung sowie ihre Verwendung zum Färben und Bedrucken von Fasermaterialien, insbesondere von textilen Fasermaterialien.

Gegenstand der vorliegenden Erfindung sind somit Verbindungen der Formel 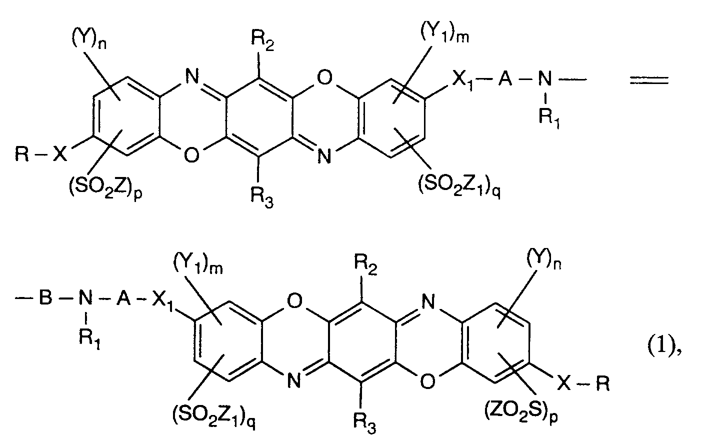 worin
R und R₁ unabhängig voneinander je Wasserstoff; unsubstituiertes oder durch Hydroxy, Sulfo, Sulfato, Chlor, Cyano, oder Acetoxy substituiertes und/oder mit Ausnahme von Methyl gegebenenfalls durch eine Gruppe -O- unterbrochenes C₁-C₄-Alkyl; unsubstituiertes oder durch 1 bis 3 Methylgruppen substituiertes Cyclopentyl oder Cyclohexyl; unsubstituiertes oder durch Sulfo, Nitro, Chlor, Methyl, Methoxy, N-Methyl- oder N-Ethylamino, N,N-Dimethyl- oder N,N-Diethylamino, Acetylamino, Propionylamino, Benzoylamino, Methoxycarbonyl, Ethoxycarbonyl, Carboxy oder Methylsulfonyl substituiertes Phenyl; unsubstituiertes oder durch Sulfo, Nitro und/oder Chlor substituiertes 1- oder 2-Naphthyl; oder unsubstituiertes oder durch Methyl, Methoxy, Sulfo und/oder Chlor substituiertes Benzyl bedeuten,
X die direkte Bindung, -O-, -S- oder -N(R₄)- ist, wobei R₄ die oben für R und R₁ angegebenen Bedeutungen hat,
X₁ -O-, -S- oder -N(R₄)- ist, wobei R₄ die oben für R und R₁ angegebenen Bedeutungen hat,
Y und Y₁ unabhängig voneinander je C₁-C₄-Alkyl, C₁-C₄-Alkoxy, Halogen, Sulfo, Carboxy, Carbamoyl, N-Mono- oder N,N-Di-C₁-C₄-Alkylcarbamoyl, N-Phenyl- oder N,N-Diphenylcarbamoyl, Sulfamoyl, N-Mono- oder N,N-Di-C₁-C₄-Alkylsulfamoyl oder N-Phenyl- oder N,N-Diphenylsulfamoyl bedeuten,
Z und Z₁ unabhängig voneinander je Hydroxy oder C₁-C₄-Alkyl sind,
R₂ und R₃ unabhängig voneinander je Wasserstoff, Halogen, Cyano, C₁-C₄-Alkyl, C₁-C₄-Alkoxy, gegebenenfalls durch C₁-C₄-Alkyl, C₁-C₄-Alkoxy, Acetylamino, Halogen, Nitro oder Sulfo substituiertes Phenyl, Benzyl, Benzoylamino oder Phenoxy, Sulfo, Carboxy, Carbamoyl, Phenylcarbamoyl oder C₂-C₅-Alkanoylamino bedeuten,
A unsubstituiertes oder durch Hydroxy, Sulfo, Sulfato, C₁-C₄-Alkoxy, Carboxy, Cyano, Halogen, Phenyl, Sulfophenyl oder C₂-C₅-Alkoxycarbonyl substituiertes und gegebenenfalls durch 1-2 Gruppen -O-, -N(R₅)-, worin R₅ C₁-C₄-Alkyl, Acetyl oder Wasserstoff bedeutet, -S- oder -SO₂- unterbrochenes C₂-C₆-Alkylen; oder unsubstituiertes oder einfach oder mehrfach durch C₁-C₃-Alkyl substituiertes Cyclopentylen oder Cyclohexylen; oder
unsubstituiertes oder durch C₁-C₄-Alkyl, C₁-C₄-Alkoxy, Sulfo, Halogen oder Carboxy substituiertes Phenylen, Biphenylen oder Naphthylen; oder
C₁-C₆-Alkylen-phenylen, Phenylen-C₁-C₆-alkylen-phenylen, C₁-C₃-Alkylen-phenylen-C₁-C₃-alkylen oder Methylen-naphthylen-methylen ist, wobei in diesen Aralkylenresten das Phenylen oder Naphthylen keine weiteren Substituenten oder 1 oder 2 Substituenten ausgewählt aus der Gruppe Sulfo, Carboxy, Sulfamoyl, Carbamoyl, Methyl, Ethyl, Methoxy, Ethoxy, Nitro, Chlor, Amino, N-Methyl- und N-Ethylamino, N,N-Dimethyl- und N,N-Diethylamino und Phenylamino enthält,
B ein Rest der Formel

   Q-E-Q (2'),

   ist, worin Q eine Gruppe der Formel 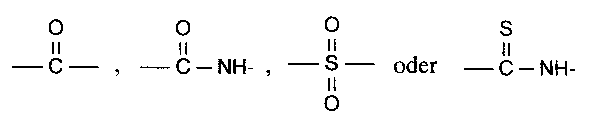 bedeutet, und
E für die direkte Bindung; unsubstituiertes oder durch Hydroxy, Sulfo, Sulfato, Methoxy, Carboxy, Phenyl oder Sulfophenyl substituiertes C₁-C₆-Alkylen oder C₂-C₆-Alkenylen; unsubstituiertes oder durch 1 bis 3 Methylgruppen substituiertes Cyclohexylen oder C₁-C₂-Alkylen-cyclohexylen; Piperazin-1,4-diyl; Thiophen-2,5-diyl; Biphenyl-4,4'-diyl; Stilben-4,4'-diyl; unsubstituiertes oder durch C₁-C₄-Alkyl, C₁-C₄-Alkoxy, Sulfo, Halogen oder Carboxy substituiertes Phenylen oder Naphthylen; oder für unsubstituiertes oder im Phenylteil durch Methyl, Methoxy, Chlor oder Sulfo substituiertes
C₁-C₃-Alkylen-phenylen oder C₁-C₂-Alkylen-phenylen-C₁-C₂-alkylen steht; und m, n, p und q unabhängig voneinander je die Zahl 0 oder 1 bedeuten.

R und R₁ stehen als Alkylrest unabhängig voneinander je für einen unsubstituierten oder durch Hydroxy, Sulfo, Sulfato, Chlor, Cyano oder Acetoxy substituierten und/oder mit Ausnahme von Methyl gegebenenfalls durch eine Gruppe -O- unterbrochenen C₁-C₄-Alkylrest.

Beispiele für geeignete Alkylreste R und R₁ sind somit Methyl, Ethyl, n-Propyl, iso-Propyl, n-, iso-, sec.- oder tert.-Butyl, β-Chlorethyl, β-Hydroxyethyl, β-Hydroxybutyl, β-Cyanethyl, Sulfomethyl, β-Sulfoethyl, β-Sulfatoethyl, β-Acetoxyethyl, β-Sulfatopropyl, γ-Sulfatopropyl oder der Rest der Formel -CH₂CH₂OCH₂CH₂OH, -CH₂CH₂NHCH₂CH₂OH oder -CH₂CH₂OCH₂CH₂OSO₃H.

Besonders bevorzugt ist für R und R₁ als Alkyl unabhängig voneinander je die Bedeutung eines unsubstituierten C₁-C₄-Alkylrestes, und sind insbesondere die Bedeutungen Methyl und Ethyl.

R und R₁ stehen als Cycloalkyl unabhängig voneinander je für unsubstituiertes oder durch 1 bis 3 Methylgruppen substituiertes Cyclopentyl oder Cyclohexyl und besonders bevorzugt für Cyclohexyl.

R und R₁ stehen als Aryl unabhängig voneinander je für unsubstituiertes oder durch Sulfo, Nitro, Chlor, Methyl, Methoxy, N-Methyl- oder N-Ethylamino, N,N-Dimethyl- oder N,N-Diethylamino, Acetylamino, Propionylamino, Benzoylamino, Methoxycarbonyl, Ethoxycarbonyl, Carboxy oder Methylsulfonyl substituiertes Phenyl oder für unsubstituiertes oder durch Sulfo, Nitro und/oder Chlor substituiertes 1- oder 2-Naphthyl.

Besonders bevorzugt ist für R und R₁ als Aryl unabhängig voneinander je die Bedeutung eines unsubstituierten oder durch Sulfo, Chlor, Methyl und/oder Methoxy substituierten Phenylrestes.

R und R₁ stehen als Aralkyl unabhängig voneinander je für unsubstituiertes oder durch Methyl, Sulfo, Chlor und/oder Methoxy substituiertes Benzyl und stellen besonders bevorzugt je den Benzylrest dar.

R und R₁ bedeuten unabhängig voneinander besonders bevorzugt je Wasserstoff, C₁-C₄-Alkyl, Cyclohexyl, unsubstituiertes oder durch Sulfo, Chlor, Methyl und/oder Methoxy substituiertes Phenyl oder Benzyl und insbesondere bevorzugt Wasserstoff, Methyl oder Ethyl. In einer besonders bevorzugten Ausführungsform der Erfindung stehen R und R₁ je für Wasserstoff.

Bei A als gegebenenfalls substituiertem Alkylenrest handelt es sich um einen unsubstituierten oder durch Hydroxy, Sulfo, Sulfato, C₁-C₄-Alkoxy, Carboxy, Cyano, Halogen, Phenyl, Sulfophenyl oder C₂-C₅-Alkoxycarbonyl substituierten und gegebenenfalls durch 1-2 Gruppen -O-, -N(R₅)-, worin R₅ C₁-C₄-Alkyl, Acetyl oder insbesondere Wasserstoff bedeutet, -S- oder -SO₂- unterbrochenen C₂-C₆-Alkylenrest.

Beispiele für geeignete Alkylenreste A sind 1,2-Ethylen, 1,2- und 1,3-Propylen, 1-Ethyl-1,2-ethylen, 2-Hydroxy-1,3-propylen, 2-Sulfato-1,3-propylen, 1- und 2-Phenyl-1,3-propylen, 2-(4'-Sulfophenyl)-1,3-propylen, 1,4-, 2,3- und 2,4-Butylen, 1,2-Dimethyl-1,2-ethylen, 1-Phenyl-1,2-ethylen, 2-Methyl-1,3-propylen, 2,2-Dimethyl-1,3-propylen, 1-Chlor-2,3-propylen, 1,5- und 2,4-pentylen, 2-Methyl-2,4-pentylen, 1-Carboxy-1,5-pentylen, 2,3-Diphenyl-1,4-butylen, 1-Methoxycarbonyl-1,5-pentylen, 1,6 und 2,5-Hexylen, 2-Carboxy-1,3-propylen, 2-Methoxy-1,3-propylen, ein Rest der Formel -CH₂-CH₂-Z'-CH₂-CH₂-, worin Z' -O-, -S-, -SO₂-, -NH- oder -N(CH₃)- bedeutet.

A steht als Alkylenrest besonders bevorzugt für einen unsubstituierten oder durch Hydroxy, Sulfo, Sulfato, Methoxy, Carboxy oder Sulfophenyl substituierten C₂-C₄-Alkylenrest oder -CH₂-CH₂-Z'-CH₂-CH₂-, worin Z' -O-, -S-, -SO₂-, -NH- oder -N(CH₃)- bedeutet und insbesondere für einen unsubstituierten oder durch Hydroxy, Sulfo, Sulfato, Methoxy, Carboxy oder Sulfophenyl substituierten C₂-C₄-Alkylenrest und insbesondere bevorzugt für einen unsubstituierten oder durch Hydroxy oder Sulfato substituierten 1,2-Ethylen oder 1,2- oder 1,3-Propylenrest. In einer besonders bevorzugten Ausführungsform der Erfindung steht A für 1,2-Ethylen, 1,2- oder 1,3-Propylen oder für 2-Sulfato-1,3-propylen.

Bei A als gegebenenfalls substituiertem Cycloalkylen handelt es sich um unsubstituiertes oder einfach oder mehrfach durch C₁-C₃-Alkyl substituiertes Cyclopentylen oder Cyclohexylen; besonders bevorzugt steht hierbei A für unsubstituiertes oder durch 1 bis 3 Methylgruppen substituiertes Cyclohexylen.

Beispiele für geeignete cycloaliphatische Reste A sind:
1,3- und 1,4-Cyclohexylen, 4-Methyl-1,3-cyclohexylen, 2-Methyl-1,3-cyclohexylen, 5,5-Dimethyl-1,3-cyclohexylen, 2-Methyl-1,4-cyclohexylen, 4,6-Dimethyl-1,3-cyclohexylen und 4-Methyl-1,2-cyclohexylen.

Steht A für einen bivalenten Arylrest, so ist dies ein unsubstituierter oder durch C₁-C₄-Alkyl, C₁-C₄-Alkoxy, Sulfo, Halogen oder Carboxy substituierter Phenylen-, Biphenylen oder Naphthylenrest.

Beispiele für Arylenreste A sind: 1,3- und 1,4-Phenylen, 2-Sulfo-1,4-phenylen, 2,5-Disulfo-1,4-phenylen, 4-Sulfo-1,3-phenylen, 2-Methyl-1,4-phenylen, 2-Carboxy-1,4-phenylen, 2-Methoxy-1,4-phenylen, 4,8-Disulfo-2,6-naphthylen, 8-Sulfo-2,6-naphthylen, 1,4-Naphthylen und 1,1'-Biphenyl-4,4'-diyl.

A steht als Arylenrest vorzugsweise für einen unsubstituierten oder durch Sulfo, Methyl, Methoxy oder Carboxy substituierten 1,3- oder 1,4-Phenylenrest oder für einen unsubstituierten oder durch Sulfo substituierten Naphthylenrest.

Bei A als Arylenrest handelt es sich besonders bevorzugt um unsubstituiertes oder durch Sulfo substituiertes 1,3- oder 1,4-Phenylen.

Bedeutet A einen gegebenenfalls substituierten Aralkylenrest, so ist dies ein C₁-C₆-Alkylen-phenylen-, ein Phenylen-C₁-C₆-alkylen-phenylen-, ein C₁-C₃-Alkylen-phenylen-C₁-C₃-alkylen- oder ein Methylen-naphthylen-methylenrest sein, wobei in diesen Aralkylenresten das Phenylen oder Naphthylen keine weiteren Substituenten oder 1 oder 2 Substituenten ausgewählt aus der Gruppe Sulfo, Carboxy, Sulfamoyl, Carbamoyl, Methyl, Ethyl, Methoxy, Ethoxy, Nitro, Chlor, Amino, N-Methyl- und N-Ethylamino, N,N-Dimethyl- und N,N-Diethylamino und Phenylamino tragen kann.

Beispiele für geeignete Aralkylenreste A sind

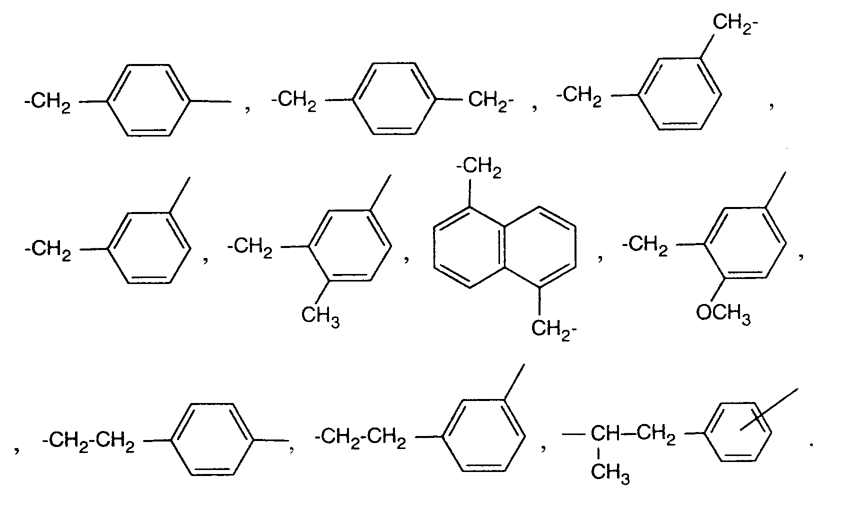

A stellt als Aralkylenrest bevorzugt einen C₁-C₃-Alkylen-phenylen- oder C₁-C₂-Alkylen-phenylen-C₁-C₂-alkylenrest dar, der im Phenylteil unsubstituiert oder z.B. durch Methyl, Methoxy, Chlor oder Sulfo substituiert ist.

A bedeutet vorzugsweise einen unsubstituierten oder durch Hydroxy, Sulfo, Sulfato, Methoxy, Carboxy, oder Sulfophenyl substituierten C₂-C₄-Alkylenrest, -CH₂-CH₂-Z'-CH₂-CH₂- worin Z' -O-, -S-, -SO₂-, -NH- oder -N(CH₃)- bedeutet, einen unsubstituierten oder durch 1 bis 3 Methylgruppen substituierten Cyclohexylenrest, einen unsubstituierten oder durch Sulfo substituierten 1,3- oder 1,4-Phenylenrest oder einen C₁-C₃-Alkylen-phenylen- oder C₁-C₂-Alkylen-phenylen-C₁-C₂-alkylenrest, worin das Phenylen jeweils unsubstituiert oder durch Methyl, Methoxy, Chlor oder Sulfo substituiert ist.

Steht R₄ für einen gegebenenfalls substituierten Alkyl-, Cycloalkyl-, Aryl- oder Aralkylrest, so gelten unabhängig die zuvor für R angegebenen Bedeutungen und Bevorzugungen.

R₄ bedeutet bevorzugt Wasserstoff, C₁-C₄-Alkyl, Cyclohexyl, unsubstituiertes oder durch Sulfo, Chlor, Methyl und/oder Methoxy substituiertes Phenyl oder Benzyl.

Besonders bevorzugte Bedeutungen von R₄ sind Wasserstoff und C₁-C₄-Alkyl, insbesondere Wasserstoff, Methyl und Ethyl und ganz besonders Wasserstoff.

X und X₁ stehen unabhängig voneinander je bevorzugt für eine Gruppe -N(R₄)-, worin für R₄ die zuvor angegebenen Bedeutungen und Bevorzugungen gelten. Besonders bevorzugt bedeuten X und X₁ je eine Gruppe -NH-.

Y und Y₁ bedeuten unabhängig voneinander vorzugsweise je Sulfo, C₁-C₄-Alkyl, C₁-C₄-Alkoxy oder Chlor und besonders bevorzugt Methoxy, Methyl, Chlor oder Sulfo; hierbei können Y und Y₁ eine verschiedene oder vorzugsweise die identische Bedeutung haben.

Die Variablen n und m stehen bevorzugt je für die Zahl 0.

Z und Z₁ stehen bevorzugt je für Methyl oder Ethyl und insbesondere bevorzugt je für Hydroxy.

p und q bedeuten vorzugsweise je die Zahl 1.

Bedeuten R₂ und/oder R₃ gegebenenfalls substituiertes Phenyl, Benzyl, Benzoylamino oder Phenoxy, so ist der Phenylring jeweils unsubstituiert oder durch C₁-C₄-Alkyl, C₁-C₄-Alkoxy, Acetylamino, Halogen, Nitro und/oder Sulfo substituiert; hierbei ist es jeweils bevorzugt, dass der Phenylring keine weiteren Substituenten trägt oder durch Chlor, Methyl, Methoxy, Acetylamino und/oder Sulfo weitersubstituiert ist.

Die Reste R₂ und R₃ können verschieden oder bevorzugt identisch sein.

R₂ und R₃ stehen vorzugsweise jedoch für Wasserstoff, Fluor, Chlor, Brom, Methyl, Methoxy, Acetylamino, Phenoxy oder Cyano und besonders bevorzugt für Brom oder Chlor.

Eine bevorzugte Ausführungsform der Erfindung betrifft Verbindungen der Formel (1), worin R₂ und R₃ jeweils Chlor bedeuten.

Bei dem bivalenten organischen Brückenglied B handelt es sich um einen Rest der Formel

-Q-E-Q- (2),

worin Q eine Gruppe der Formel 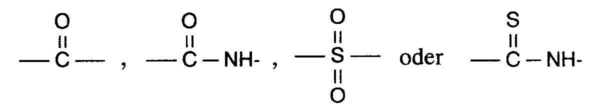 bedeutet, E für die direkte Bindung, unsubstituiertes oder durch Hydroxy, Sulfo, Sulfato, Methoxy, Carboxy, Phenyl oder Sulfophenyl substituiertes C₁-C₆-Alkylen oder C₂-C₆-Alkenylen, unsubstituiertes oder durch 1 bis 3 Methylgruppen substituiertes Cyclohexylen oder C₁-C₂-Alkylen-cyclohexylen, Piperazin-1,4-diyl, Thiophen-2,5-diyl, Biphenyl-4,4'-diyl, Stilben-4,4'-diyl, unsubstituiertes oder durch C₁-C₄-Alkyl, C₁-C₄-Alkoxy, Sulfo, Halogen oder Carboxy substituiertes Phenylen oder Naphthylen oder für unsubstituiertes oder im Phenylteil durch Methyl, Methoxy, Chlor oder Sulfo substituiertes C₁-C₃-Alkylen-phenylen oder C₁-C₂-Alkylen-phenylen-C₁-C₂-alkylen steht.

E als Alkylenrest ist bevorzugt unsubstituiertes oder durch Hydroxy, Sulfo, Sulfato, Methoxy, Carboxy, Phenyl oder Sulfophenyl substituiertes C₁-C₄-Alkylen und besonders bevorzugt Methylen, 1,2-Ethylen oder 1,2- oder 1,3-Propylen.

Besonders bevorzugte bivalente Brückenglieder B entsprechen hierbei der Formel 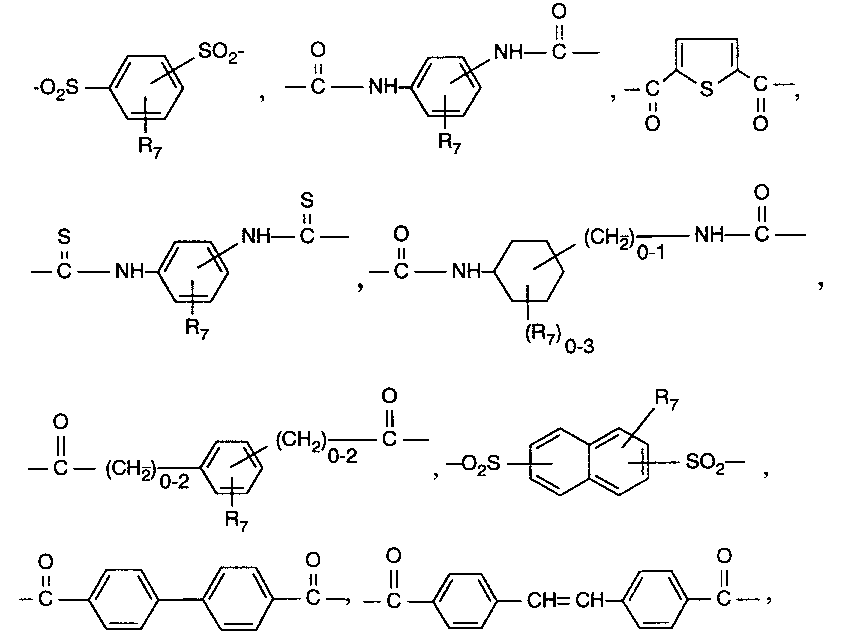 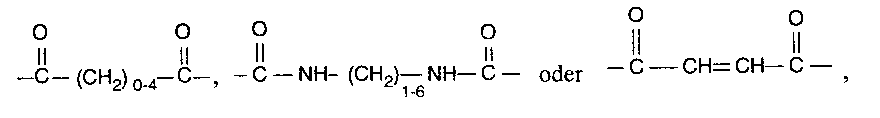 worin R₇ Sulfo, Methyl, Methoxy, Chlor, Carboxy oder vorzugsweise Wasserstoff bedeutet.

Eine besonders bevorzugte Ausführungsform der vorliegenden Erfindung betrifft Verbindungen der Formel (1), worin B ein Rest der Formel 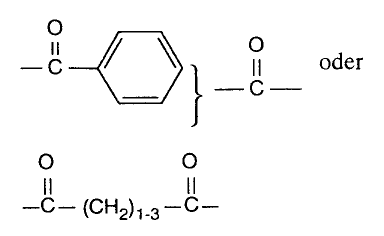 ist.

Von Bedeutung wegen ihrer guten färberischen Eigenschaften sind Verbindungen der zuvor angegebenen Formel (1), worin R und R₁ unabhängig voneinander je für Wasserstoff, unsubstituiertes oder durch Hydroxy, Sulfo, Sulfato, Chlor, Cyano, oder Acetoxy substituiertes und/oder mit Ausnahme von Methyl gegebenenfalls durch eine Gruppe -O-unterbrochenes C₁-C₄-Alkyl, unsubstituiertes oder durch 1 bis 3 Methylgruppen substituiertes Cyclopentyl oder Cyclohexyl, unsubstituiertes oder durch Sulfo, Nitro, Chlor, Methyl, Methoxy, N-Methyl- oder N-Ethylamino, N,N-Dimethyl- oder N,N-Diethylamino, Acetylamino, Propionylamino, Benzoylamino, Methoxycarbonyl, Ethoxycarbonyl, Carboxy oder Methylsulfonyl substituiertes Phenyl, unsubstituiertes oder durch Sulfo, Nitro und/oder Chlor substituiertes 1- oder 2-Naphthyl oder für unsubstituiertes oder durch Methyl, Methoxy, Sulfo und/oder Chlor substituiertes Benzyl stehen,
A für einen unsubstituierten oder durch Hydroxy, Sulfo, Sulfato, Methoxy, Carboxy oder Sulfophenyl substituierten C₂-C₄-Alkylenrest, -CH₂-CH₂-Z'-CH₂-CH₂- worin Z' -O-, -S-, -SO₂-, -NH- oder -N(CH₃)- bedeutet, einen unsubstituierten oder durch 1 bis 3 Methylgruppen substituierten Cyclohexylenrest, einen unsubstituierten oder durch Sulfo substituierten 1,3- oder 1,4-Phenylenrest oder für einen C₁-C₃-Alkylen-phenylen- oder C₁-C₂-Alkylen-phenylen-C₁-C₂-alkylenrest, worin das Phenylen jeweils unsubstituiert oder durch Methyl, Methoxy, Chlor oder Sulfo substituiert ist, steht,
X und X₁ unabhängig voneinander je eine Gruppe -N(R₄)-, worin R₄ Wasserstoff, C₁-C₄-Alkyl, Cyclohexyl, unsubstituiertes oder durch Sulfo, Chlor, Methyl und/oder Methoxy substituiertes Phenyl oder Benzyl bedeutet, sind,
Y und Y₁ unabhängig voneinander je Methoxy, Methyl, Chlor oder Sulfo sind, n und m unabhängig voneinander je für die Zahl 0 oder 1 stehen, Z und Z₁ unabhängig voneinander je Hydroxy, Methyl oder Ethyl bedeuten, p und q je die Zahl 1 darstellen, R₂ und R₃ jeweils Wasserstoff, Fluor, Chlor, Brom, Methyl, Methoxy, Acetylamino, Phenoxy oder Cyano bedeuten,
und das Brückenglied B die oben genannten Bedeutungen und Bevorzugungen hat.

Von besonderer Bedeutung wegen ihrer guten färberischen Eigenschaften sind Verbindungen der Formel 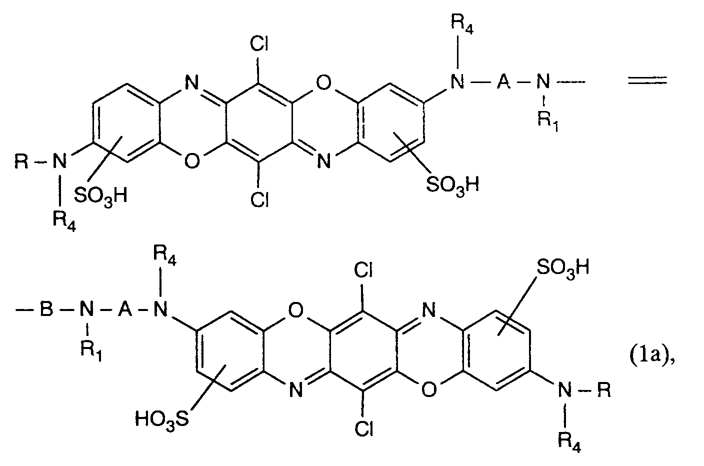 worin R und R₁ unabhängig voneinander je Wasserstoff, C₁-C₄-Alkyl, Cyclohexyl, unsubstituiertes oder durch Sulfo, Chlor, Methyl und/oder Methoxy substituiertes Phenyl oder Benzyl bedeuten,
A einen unsubstituierten oder durch Hydroxy, Sulfo, Sulfato, Methoxy, Carboxy oder Sulfophenyl substituierten C₂-C₄-Alkylenrest darstellt, R₄ Wasserstoff, Methyl oder Ethyl bedeutet, und B für einen Rest der Formel 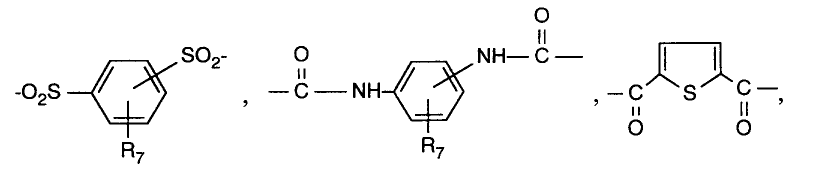 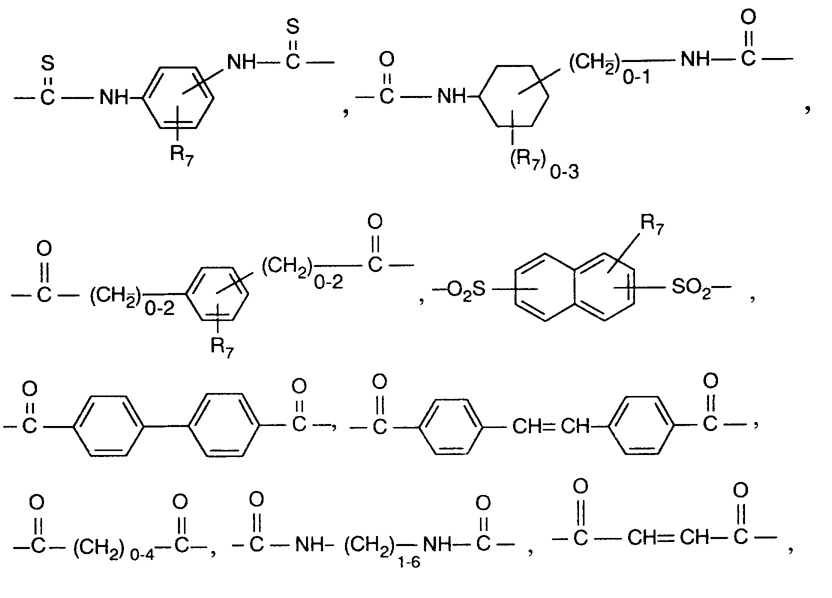 steht, worin R₇ Sulfo, Methyl, Methoxy, Chlor, Carboxy oder vorzugsweise Wasserstoff bedeutet.

Eine besonders bevorzugte Ausführungsform der vorliegenden Erfindung betrifft Verbindungen der Formel 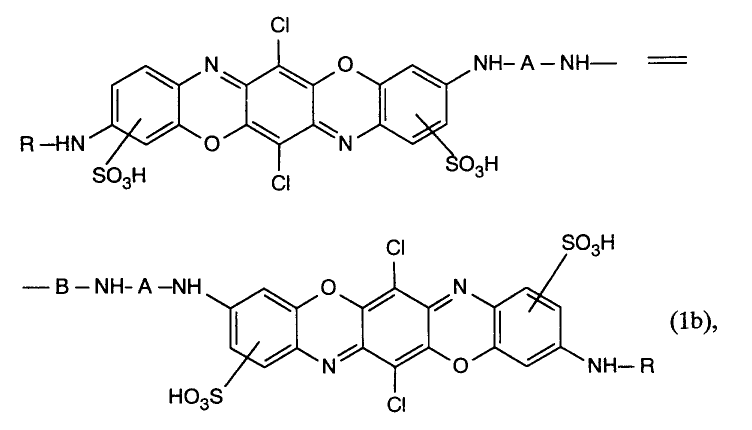 worin R Methyl, Ethyl, Benzyl oder insbesondere Wasserstoff bedeutet, A für einen unsubstituierten oder durch Hydroxy oder Sulfato substituierten 1,2-Ethylen- oder 1,2-oder 1,3-Propylenrest steht und B ein Rest der Formel 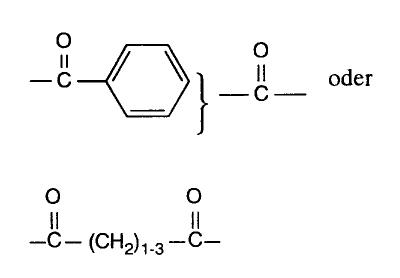 ist.

Die Verbindungen der Formel (1) können erhalten werden, z.B. indem man eine Verbindung der Formel 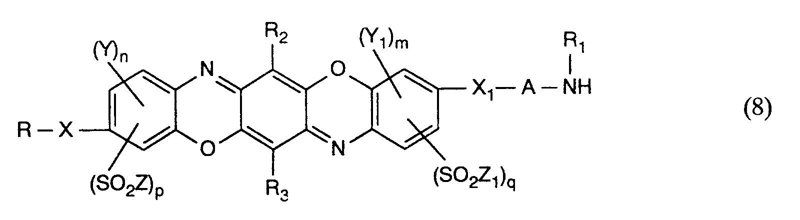 mit einer Verbindung der Formel

T-B-T (9)

kondensiert, worin A, B, R, R₁, R₂, R₃, X, X₁, Y, Y₁, Z, Z₁, m, n, p und q jeweils die zuvor angegebene Bedeutung haben und T Halogen, vorzugsweise Chlor, ist.

Die Kondensationsreaktion wird vorteilhaft in einem wässrigen oder wässrig-organischem Medium bei einer Temperatur von z.B. 0 bis 100°C und vorzugsweise 0 bis 50°C, durchgeführt. Man arbeitet zweckmässig bei einem neutralen bis alkalischen pH-Wert, d.h. bei einem pH-Wert von 7 bis 13 und vorzugsweise 8 bis 12; der pH-Wert kann durch Zugabe von Basen, z.B. Alkalimetallhydroxiden oder -carbonaten, Ammoniak oder organischen Aminen eingestellt und während der Kondensationsreaktionen konstant gehalten werden.

Die Verbindungen der Formeln (8) und (9) werden z.B. in einem molaren Verhältnis von 1,5:1 bis 2:1, vorzugsweise 1,7:1 bis 2:1, eingesetzt.

Die Verbindungen der Formel (8) sind z.B. aus der EP-A 0 356 014 bekannt oder können analog zu den dort angegebenen Herstellungsverfahren erhalten werden. Die Verbindungen der Formel (9) sind bekannt oder können in bekannter Weise erhalten werden.

Ein weiterer Gegenstand der vorliegenden Erfindung betrifft Farbstoffmischungen, die erhältlich sind, indem man eine Verbindung der zuvor angegebenen Formel (8) und eine Verbindung der Formel 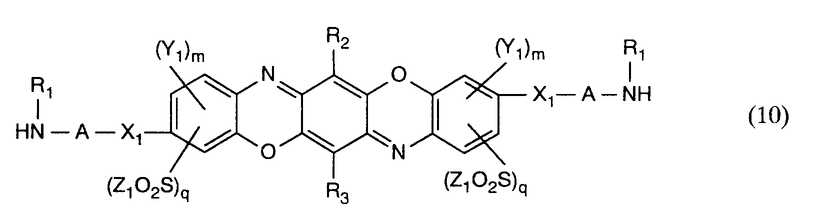 worin A, R₁, R₂, R₃, X₁, Y₁, Z₁, m und q jeweils die unter der Formel (1) angegebene Bedeutung haben, mit einer Verbindung der zuvor angegebenen Formel (9) kondensiert. Die Umsetzung verläuft hierbei im allgemeinen wie zuvor bei der Reaktion der Verbindungen der Formeln (8) und (9) beschrieben; anstelle einer Verbindung der Formel (8) verwendet man jedoch die äquimolare Menge eines Gemisches enthaltend je eine Verbindung der Formeln (8) und (10). Es hat sich als vorteilhaft erwiesen, die Verbindungen der Formeln (8) und (10) im Gewichtsverhältnis von z.B. 95:5 bis 50:50, vorzugsweise 90:10 bis 60:40 und besonders bevorzugt 80:20 bis 65:35, in das Verfahren einzusetzen.

Die Verbindungen der Formel (10) sind bekannt oder können in bekannter Weise erhalten werden.

Die Verbindungen der Formeln (8) und (10) können unabhängig voneinander hergestellt und in das Verfahren eingesetzt werden.

Mischungen von Verbindungen der Formeln (8) und (10) sind jedoch synthetisch auch direkt zugänglich, z.B. indem man eine Verbindung der Formel 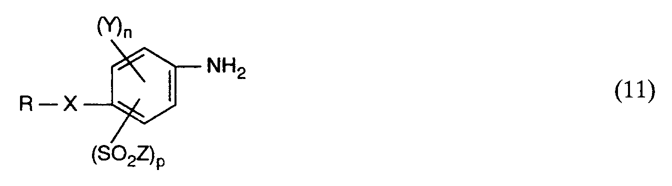 und eine Verbindung der Formel 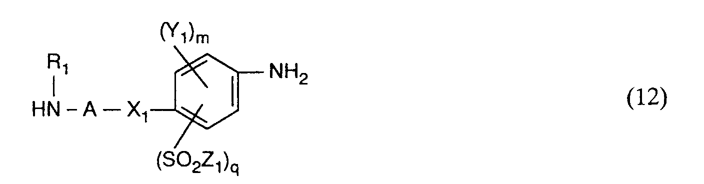 in an und für sich üblicher und zur Synthese von Triphendioxazin-Verbindungen bekannter Verfahrensweise mit einer 1,4-Benzochinon-Verbindung der Formel 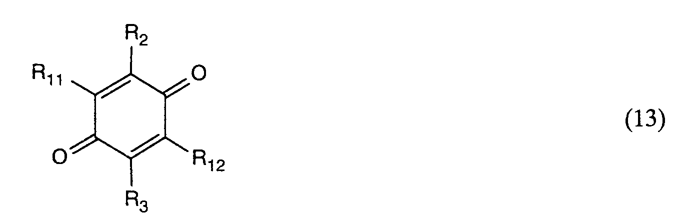 umsetzt, worin R, R₁, R₂, R₃, A, X, X₁, Y, Y₁, Z, Z₁, m, n, p und q jeweils die unter der Formel (1) angegebene Bedeutung haben und R₁₁ und R₁₂ unabhängig je eine der zuvor für R₂ angegebenen Bedeutungen haben.

Die Umsetzung der Verbindungen der Formeln (11), (12) und (13) miteinander führt zu einem Verbindungsgemisch, welches neben je einer Verbindung der Formeln (8) und (10) als weiteren Bestandteil noch eine Verbindung der Formel 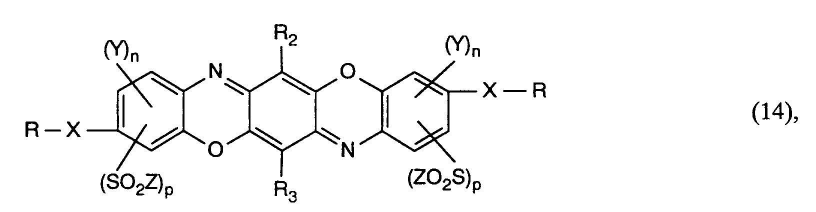 worin R, R₂, R₃, X, Y, Z, n und p jeweils die unter der Formel (1) angegebene Bedeutung haben, erhält; diese stört jedoch weder die Anwendbarkeit des erhaltenen Verbindungsgemisches für die weitere Umsetzung mit einer Verbindung der Formel (9) noch die Farbstoffeigenschaften des daraus resultierenden Farbstoffgemisches.

Die durch Umsetzung je einer Verbindung der Formeln (8), (9) und (10) erhältlichen Farbstoffgemische enthalten als Hauptkomponenten
a) eine Verbindung der zuvor angegebenen Formel (1),
b) oligomere Verbindungen der vermutlichen Formel 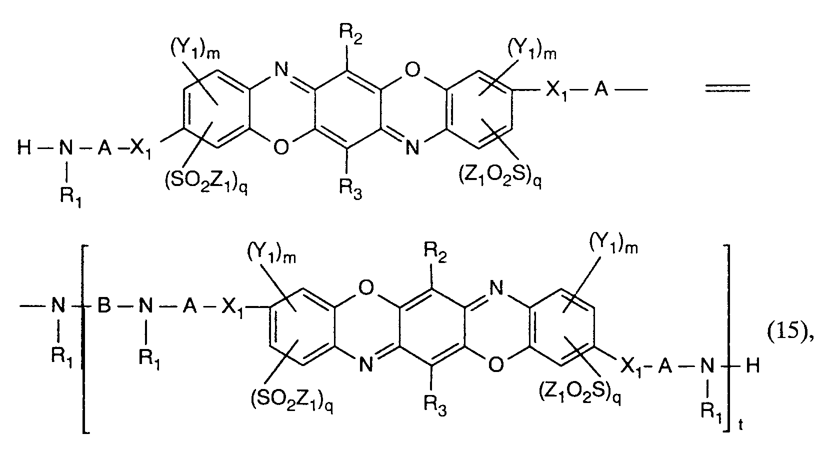 worin A, B, R₁, R₂, R₃, X₁, Y₁, Z₁, m und q jeweils die unter der Formel (1) angegebene Bedeutung haben und t für die Zahl 1, 2, 3, 4, 5 oder 6 steht, sowie
c) eine Verbindung der vermutlichen Formel 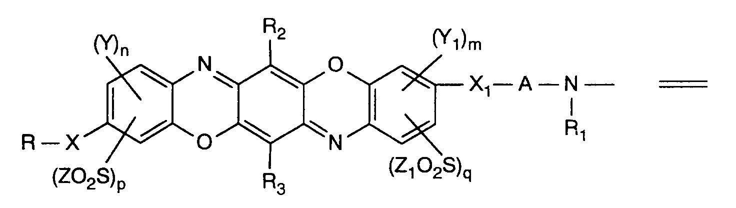 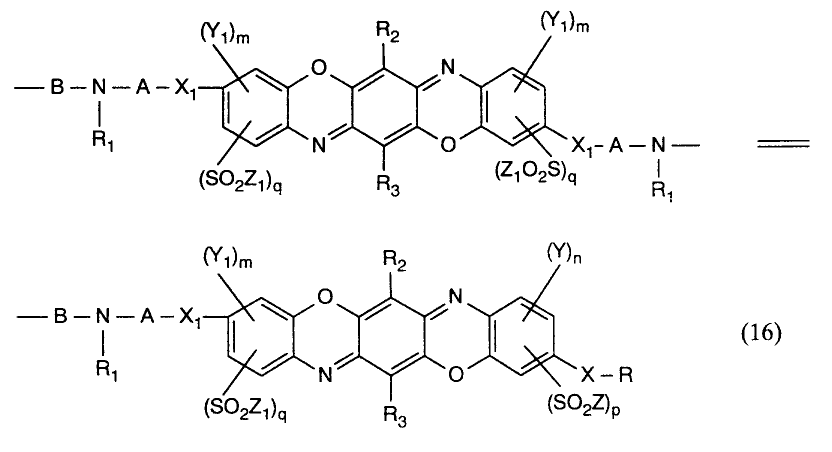 worin A, B, R, R₁, R₂, R₃, X, X₁, Y, Y₁, Z, Z₁, m, n, p und q jeweils die unter der Formel (1) angegebene Bedeutung haben.

Ein weiterer Gegenstand der Erfindung ist die Verwendung der Verbindungen der Formel (1) sowie der durch Umsetzung je einer Verbindung der Formeln (8), (9) und (10) erhältlichen Verbindungsgemische als Farbstoffe zum Färben oder Bedrucken von stickstoffhaltigen und insbesondere hydroxylgruppenhaltigen Fasermaterialien.

Die erfindungsgemässen Verbindungen der Formel (1) sowie die erfindungsgemäss erhältlichen Farbstoffgemische eignen sich also zum Färben und Bedrucken von stickstoffhaltigen oder insbesondere von cellulosischen Fasermaterialien, vorzugsweise von textilen Fasermaterialien, aus Seide, Wolle oder synthetischen Polyamiden, sowie bevorzugt aus den cellulosischen Fasern, wie Rayon, Baumwolle oder Hanf.

Bezüglich ihrer färberischen Eigenschaften können sie als direktziehende oder Direktfarbstoffe (C.I. direct dyes) bezeichnet werden.

Ebenfalls können textile Fasermaterialien aus Mischfasern, wie z.B. aus Wolle/Baumwoll-, Polyamid/Baumwoll-, Polyacryl/Baumwoll- oder insbesondere Polyester/Baumwoll-Mischfasern durch Einbad-Färbeverfahren und in Gegenwart von Farbstoffen für die jeweils anderen Fasertypen gefärbt werden.

Die textilen Fasermaterialien können in den verschiedensten Verarbeitungszuständen vorliegen, wie z.B. als Faser, Garn, Gewebe oder Gewirke. Neben den textilen Substraten können auch Leder und Papier mit den erfindungsgemässen Farbstoffen und Farbstoffgemischen gefärbt werden.

Man erhält egale Färbungen in blauen Farbtönen mit guten Allgemeinechtheiten, insbesondere guten Reib-, Nass-, Nassreib-, Schweiss- und Lichtechtheiten. Sofern nötig, kann man die Nassechtheiten, insbesondere die Waschechtheit, der erhaltenen Direktfärbungen und -drucke durch eine Nachbehandlung mit sog. Fixiermitteln noch wesentlich verbessern.

Die erfindungsgemässen Farbstoffe und Farbstoffgemische sind gut mit anderen Farbstoffen, insbesondere Dispersionsfarbstoffen kombinierbar. Die erfindungsgemässen Farbstoffe und Farbstoffgemische weisen eine ausreichende Hochtemperatur-Stabilität auf und lassen sich so unter den Färbebedingungen für Polyesterfasern, d.h. bei Temperaturen im Bereich von etwa 100 bis 150°C, vorzugsweise von 100 bis 130°C, aus wässriger Flotte und bei einem pH-Wert von 4 bis 7,5, vorzugsweise 5 bis 7, färben.

Damit ist es möglich, übliche Dispersionsfarbstoffe zusammen mit den erfindungsgemässen Farbstoffen und Farbstoffgemischen in einem einstufigen, einbadigen Verfahren zum Färben von Polyester/Baumwoll-Mischfasern (Mischgewebe) einzusetzen, wobei beide Faserarten gleichmässig und echt durch den jeweiligen Farbstoff angefärbt werden. Verwendet man einen Dispersionsfarbstoff mit gleicher Nuance wie der erfindungsgemässe Farbstoff bzw. das Farbstoffgemisch sie aufweisen, so ist es auch möglich Ton-in-Ton-Färbungen zu erhalten.

Mit der Bereitstellung der erfindungsgemässen Farbstoffe und Farbstoffgemische kann man das Färben von textilen Mischfasern (Mischgeweben), z.B. solchen aus Polyester- und Cellulosefasern, wesentlich vereinfachen. Die an sich übliche Färbung jeder Faserart einer Fasermischung in einem separaten Arbeitsgang unter Anwendung unterschiedlicher Färbebedingungen ist damit nicht mehr nötig.

Die erfindungsgemässen Verbindungen der Formel (1) sowie die erfindungsgemässen Verbindungsgemische eignen sich auch zur Herstellung von wässrigen Tinten für den Tintenstrahldruck (ink-jet printing).

Die folgenden Beispiele dienen zur Erläuterung der Erfindung. Teile und Prozente beziehen sich auf das Gewicht, sofern nicht anders angegeben. Gewichtsteile und Volumenteile stehen in gleicher Beziehung zueinander wie Kilogramm und Liter. Die Temperaturen sind in Celsiusgraden angegeben.

### Beispiel 1: 118 Teile der Verbindung der Formel

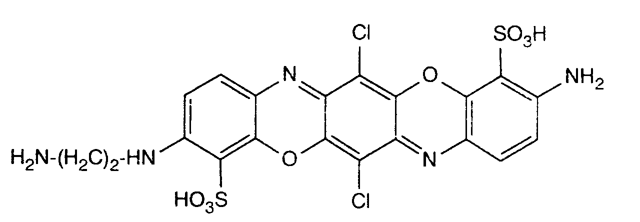

(Herstellung gemäss EP 0 356 014, Beispiel 68)
werden in 3000 Teilen Wasser suspendiert und der pH-Wert der Suspension mit Natriumhydroxidlösung auf ca. 11-12 eingestellt. Man tropft innerhalb von ca. 30 Minuten 22,5 Teile Terephthaloyldichlorid in 200 Teilen Tetrahydrofuran bei 0 bis 5° zu und hält dabei den pH-Wert durch Zugabe von Natriumhydroxidlösung bei 11 bis 12. Anschliessend lässt man ca. 2 Stunden bei Raumtemperatur und pH 11 bis 12 ausreagieren, wobei weitere Natriumhydroxidlösung verbraucht wird; dann stellt man den pH-Wert mit Salzsäure in etwa neutral ein, salzt aus, lässt weitere 30 Minuten rühren und filtriert dann das Produkt ab, welches der Formel (1) entspricht, worin R und R₁ je Wasserstoff, X und X₁ je -NH-, Z und Z₁ je Hydroxy, n und m je 0, p und q je 1, R₂ und R₃ je Chlor, A 1,2-Ethylen und B ein Rest der Formel

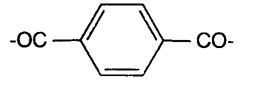

bedeuten, und welches Baumwolle in einer blauen Nuance mit guten Allgemeinechtheiten färbt.

### Beispiel 1a: Verfährt man wie im Beispiel 1 beschrieben und verwendet anstelle von Terephthaloyldichlorid die äquivalente Menge Isophthaloyldichlorid, erhält man einen analogen Farbstoff, der Baumwolle in einer blauen Nuance mit guten Allgemeinechtheiten färbt.

### Beispiel 2:

a) 17,9 Teile 1,4-Diaminobenzol-2-sulfonsäure (52,5%) und 38,1 Teile 4-(3-Aminopropylamino)-anilin-3-sulfonsäure (96,5%) werden in 1500 Teilen Wasser und 300 Teilen Isopropanol suspendiert, die Suspension durch Zugabe von Phosphatpuffergemisch und Natriumhydroxidlösung auf pH 5,5-6,0 eingestellt und unter Rühren auf 50° erwärmt. In die warme Suspension werden 49,2 Teile Chloranil eingetragen und dabei der pH-Wert durch Zugabe von Kaliumhydrogencarbonatlösung bei 6 gehalten. Man lässt ca. 4 Stunden weiterreagieren, kühlt auf Raumtemperatur ab und filtriert den ausgefallenen Feststoff ab, der anschliessend intensiv mit Wasser und dann mit Aceton gewaschen wird.
   Man erhält ein Stoffgemisch bestehend aus 1,2-1,4 Teilen der Verbindung der Formel 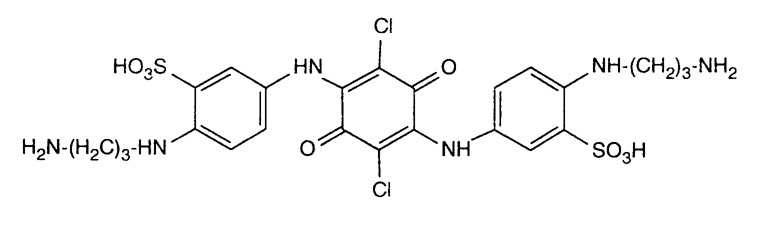 und 1 Teil der Verbindung der Formel 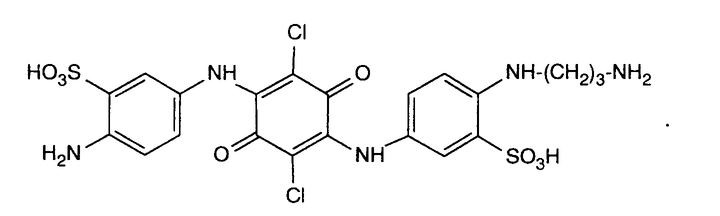 (Eine Verbindung der mutmasslichen Formel 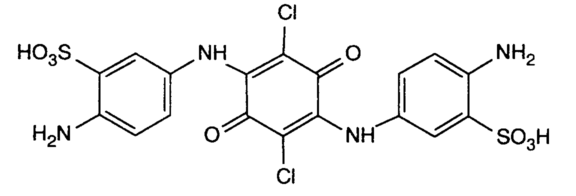 befindet sich im Filtrat.)
b) 55 Teile des gemäss a) erhaltenen Anilgemisches werden bei 0 bis 5° innerhalb von 3 Stunden in kleinen Portionen unter Rühren zu 200 Teilen 25%igem Oleum zugegeben. Dann werden weitere 100 Teile 25%iges Oleum zugetropft und 30 Minuten bei Raumtemperatur gerührt. Anschliessend gibt man innerhalb von ca. 80 Minuten 52,3 Teile Kaliumperoxodisulfat in kleinen Portionen so zu, dass die Temperatur 30° nicht übersteigt, und lässt das Ganze ca. 30 Minuten ausreagieren. Danach wird das Reaktionsgemisch in ein Eis/Wasser-Gemisch gegeben, ca. 15 Minuten rühren gelassen und dann der Feststoff abfiltriert. Der erhaltene Niederschlag wird intensiv mit Wasser und mit Ethanol gewaschen und getrocknet. Man erhält die Dioxazinverbindungen der Formeln 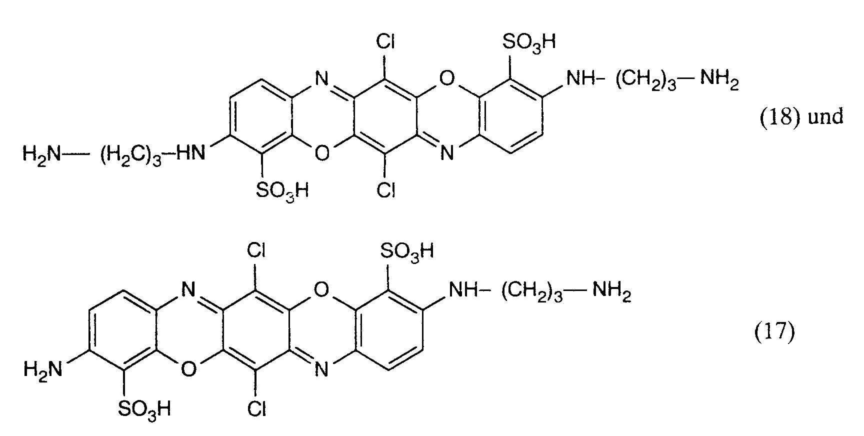 im Verhältnis von ca. 1,2-1,4:1.
c) 76,8 Teile des gemäss b) erhaltenen Dioxazinverbindungsgemisches werden in 750 Teilen Wasser suspendiert und unter Zugabe von Natriumhydroxidlösung vollständig gelöst. Zu dieser Lösung, die einen pH-Wert von ca. 11,5 aufweist, werden innerhalb von ca. 30 Minuten 7,5 Teile Terephthaloyldichlorid in 60 Teilen Tetrahydrofuran (THF) zugetropft und der pH-Wert durch Zugabe von Natriumhydroxidlösung bei 11,5 gehalten. Anschliessend lässt man ca. 2 Stunden bei Raumtemperatur und pH 11,5 ausreagieren, wobei weitere Natriumhydroxidlösung verbraucht wird; dann wird der pH-Wert mit Essigsäure neutral gestellt und nach Zugabe von Natriumchlorid und Ethanol noch ca. 30 Minuten gerührt. Das ausgesalzene Produkt wird abfiltriert und getrocknet; es enthält als Hauptkomponenten je eine Verbindung der Formeln (1) und (16) sowie oligomere Verbindungen der Formel (15), worin A 1,3-Propylen, B ein Rest der Formel 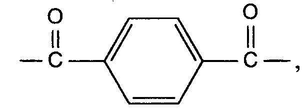 R und R₁ je Wasserstoff, R₂ und R₃ je Chlor, X und X₁ je -NH-, Z und Z₁ je Hydroxy, p und q je die Zahl 1, m und n je die Zahl 0 und t die Zahl 1, 2 oder 3 bedeutet, und färbt Baumwolle in einer brillanten blauen Nuance mit guten Allgemeinechtheiten.

### Beispiel 3:

a) 80 Teile der Verbindung der Formel 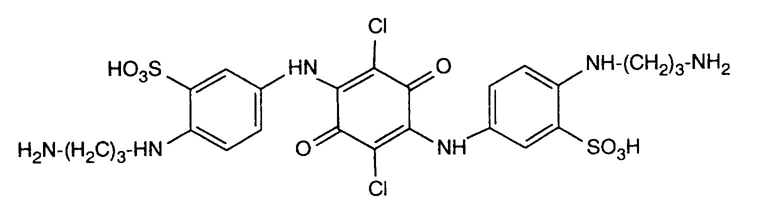 werden bei Raumtemperatur innerhalb von 40 Minuten unter gutem Rühren in 820 Teile Oleum (25%) eingetragen. Die Temperatur steigt dabei von anfänglich 20 bis 25° auf 40 bis 43° an, wobei sich eine olivbraune Lösung bildet. Anschliessend wird innerhalb von 15 Minuten auf 65° erwärmt und das Reaktionsgemisch 45 Minuten bei dieser Temperatur gehalten, wobei sich die Mischung allmählich tiefblau verfärbt. Dann wird im Eisbad auf 20° abgekühlt und der ganze Ansatz (unter externer Eiskühlung) vorsichtig auf 4000 Teile Eis ausgetragen. Die Temperatur bleibt hierbei unterhalb eines Wertes von 30°. Zum Schluss werden 2500 Teile einer wässrigen Natriumhydroxid-Lösung (30%) innerhalb von ca. einer Stunde zugetropft. Die Temperatur wird hierbei durch Kühlung bei einem Wert von 35° gehalten. Anschliessend wird unter gutem Rühren innerhalb von 30 Minuten auf 20° abgekühlt und das ausgefallene Produkt abgenutscht. Nach zweimaligem Waschen mit je 1000 Teilen Wasser und Trockensaugen erhält man 172 Teile eines noch feuchten Rohproduktes, welches in Form der freien Säure dem Gemisch bestehend aus 70 Gew.-% der Verbindung der Formel 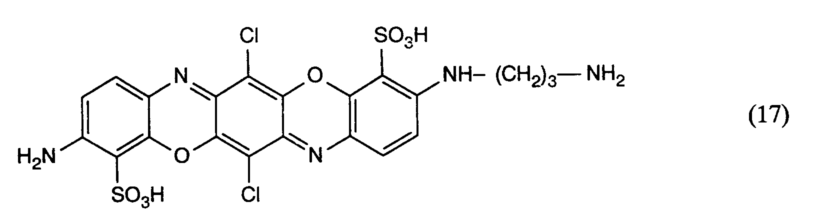 und 30 Gew.-% der Verbindung der Formel 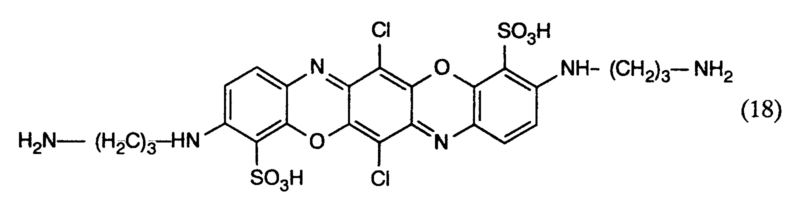 entspricht.
b) 25,6 Teile des wie oben angegeben erhaltenen Gemisches bestehend aus 70 Gew.-% der Verbindung der Formel (17) und 30 Gew.-% der Verbindung der Formel (18) werden in 250 Teilen Wasser suspendiert und unter Zugabe von Natriumhydroxidlösung vollständig gelöst. Zu dieser Lösung, die einen pH-Wert von ca. 11,5 aufweist, werden innerhalb von ca. 30 Minuten 2,5 Teile Terephthaloyldichlorid in 20 Teilen Tetrahydrofuran (THF) zugetropft und der pH-Wert durch Zugabe von Natriumhydroxidlösung bei 11,5 gehalten. Anschliessend lässt man ca. 2 Stunden bei Raumtemperatur und pH 11,5 ausreagieren, wobei weitere Natriumhydroxidlösung verbraucht wird; dann wird der pH-Wert mit Essigsäure neutral gestellt und nach Zugabe von Natriumchlorid und Ethanol noch ca. 30 Minuten gerührt. Das ausgesalzene Produkt wird abfiltriert und getrocknet; es enthält als Hauptkomponenten je eine Verbindung der Formeln (1) und (16) sowie oligomere Verbindung der Formel (15), worin A 1,3-Propylen, B ein Rest der Formel 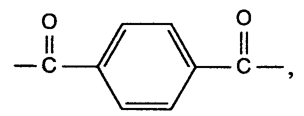 R und R₁ je Wasserstoff, R₂ und R₃ je Chlor, X und X₁ je -NH-, Z und Z₁ je Hydroxy, p und q je die Zahl 1, m und n je die Zahl 0 und t die Zahl 1, 2 oder 3 bedeutet, und färbt Baumwolle in einer brillanten blauen Nuance mit guten Allgemeinechtheiten.

Beispiele 4 bis 13: Verfährt man wie in Beispiel 2 angegeben, verwendet jedoch in Schritt a) des Beispiels 2 anstelle von 38,1 Teile 4-(3-Aminopropylamino)-anilin-3-sulfonsäure eine äquimolare Menge einer der in der folgenden Tabelle 1 in Spalte 2 angegebenen Anilinoverbindungen, so erhält man analoge Gemische von Farbstoffen, die Baumwolle in einer brillanten blauen Nuance mit guten Allgemeinechtheiten färben.

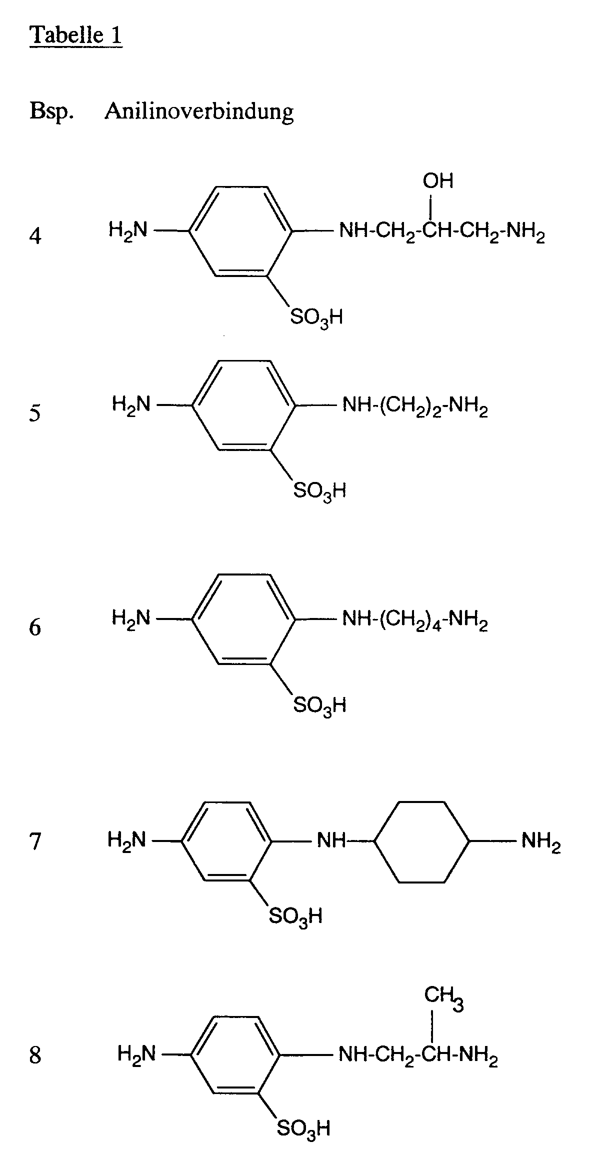

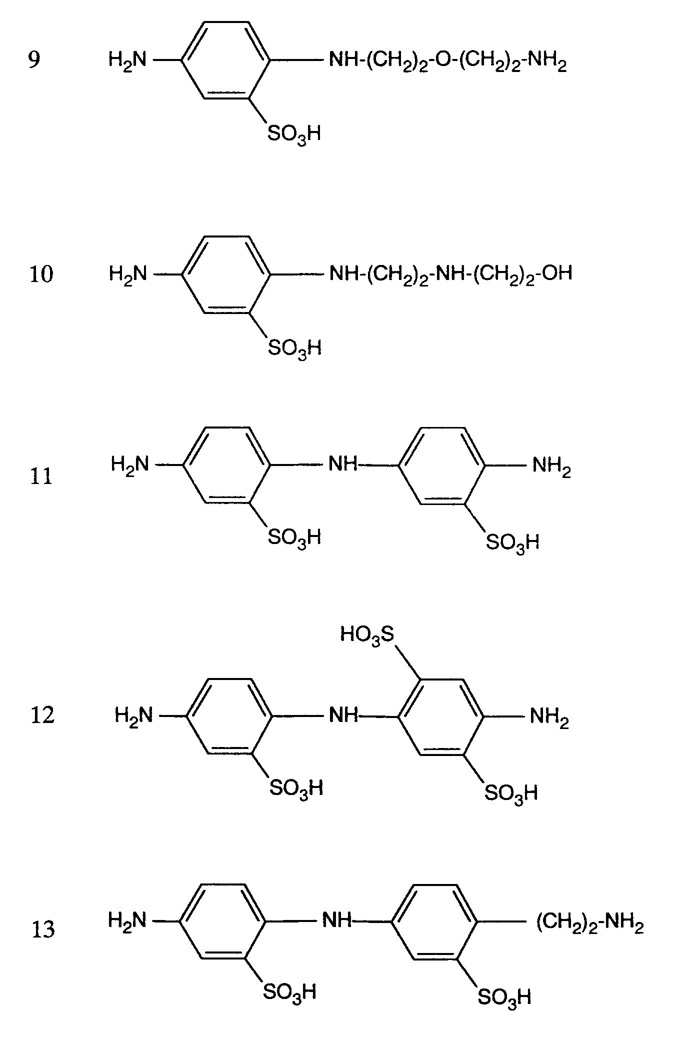

Beispiele 14 bis 25: Verfährt man wie in Beispiel 2 angegeben, verwendet jedoch in Schritt c) des Beispiels 2 anstelle von 7,5 Teilen Terephthaloyldichlorid eine äquimolare Menge einer der in der folgenden Tabelle 2 in Spalte 2 angegebenen Dicarbonylchlorid- bzw. Disulfonylchloridverbindungen, so erhält man analoge Gemische von Farbstoffen, die Baumwolle in einer brillanten blauen Nuance mit guten Allgemeinechtheiten färben.

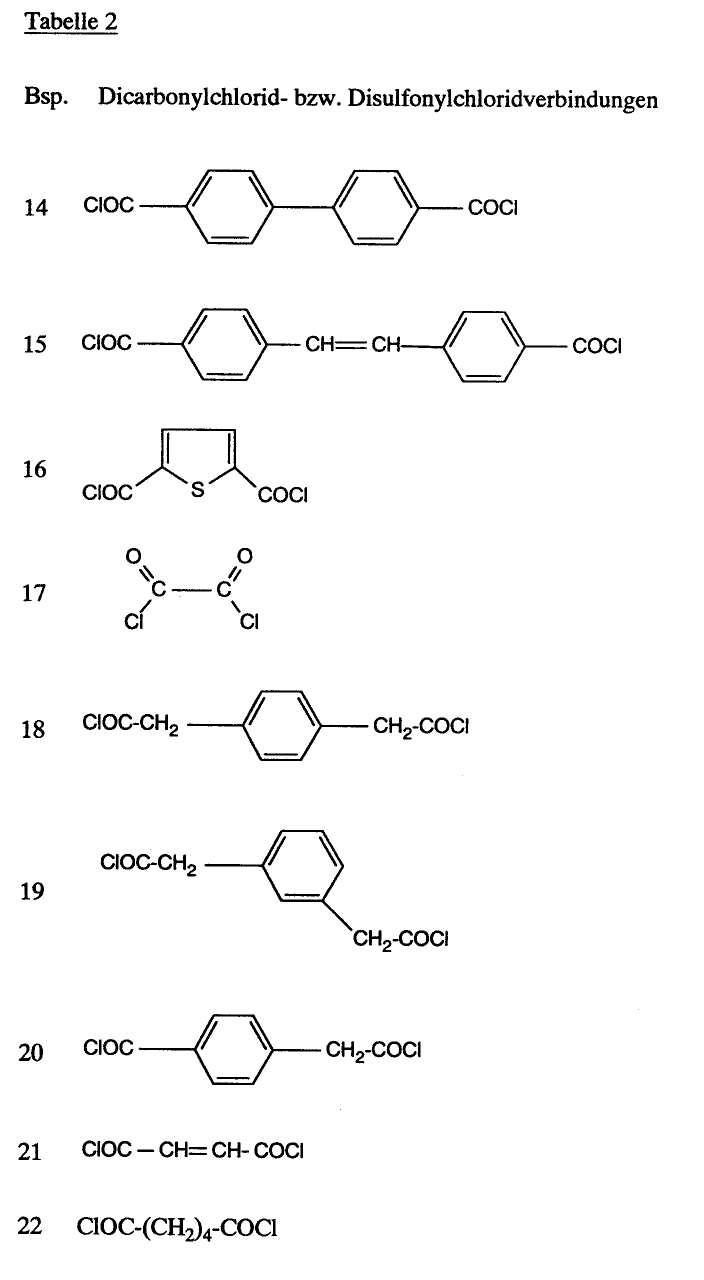

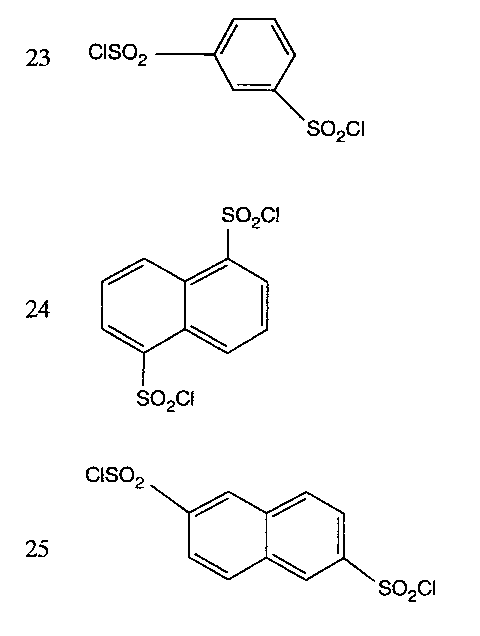

Beispiel 26: 12,65 Teile des gemäss Schritt a) des Beispiels 3 erhältlichen Gemisches der Verbindungen der Formeln (17) und (18) (Gehalt von 49,2%) werden in 140 Teile Wasser suspendiert und mit 1,57 Teilen Lithiumhydroxid x H₂O bei Raumtemperatur innerhalb von 2,5 Stunden unter Rühren in Lösung gebracht. Anschliessend werden 1,26 Teile Toluylen-2,4-diisocyanat, gelöst in 5 Teilen Dioxan, bei einer Temperatur von 10° innerhalb von einer Stunde zugetropft. Nach beendeter Kondensation wird durch Zugabe von Salzsäure sauergestellt und das ausgefallene Produkt abgenutscht. Man erhält 7 Teile eines blauen Pulvers. Das erhaltene Produkt enthält als Hauptkomponenten je eine Verbindung der Formeln (1) und (16) sowie oligomere Verbindungen der Formel (15), worin A 1,3-Propylen, B ein Rest der Formel 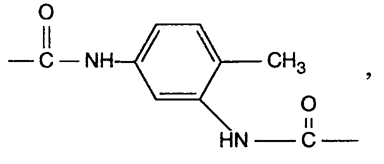 R und R₁ je Wasserstoff, R₂ und R₃ je Chlor, X und X₁ je -NH-, Z und Z₁ je Hydroxy, p und q je die Zahl 1, m und n je die Zahl 0 und t die Zahl 1, 2 oder 3 bedeutet, und färbt Baumwolle in einer brillanten blauen Nuance mit guten Allgemeinechtheiten.

Beispiele 27 bis 32: Verfährt man wie in Beispiel 26 angegeben, verwendet jedoch anstelle von 1,26 Teilen Toluylen-2,4-diisocyanat eine äquimolare Menge einer der in der folgenden Tabelle 3 in Spalte 2 angegebenen Isocyano- bzw. Isothiocyanoverbindungen, so erhält man analoge Gemische von Farbstoffen, die Baumwolle in einer brillanten blauen Nuance mit guten Allgemeinechtheiten färben.

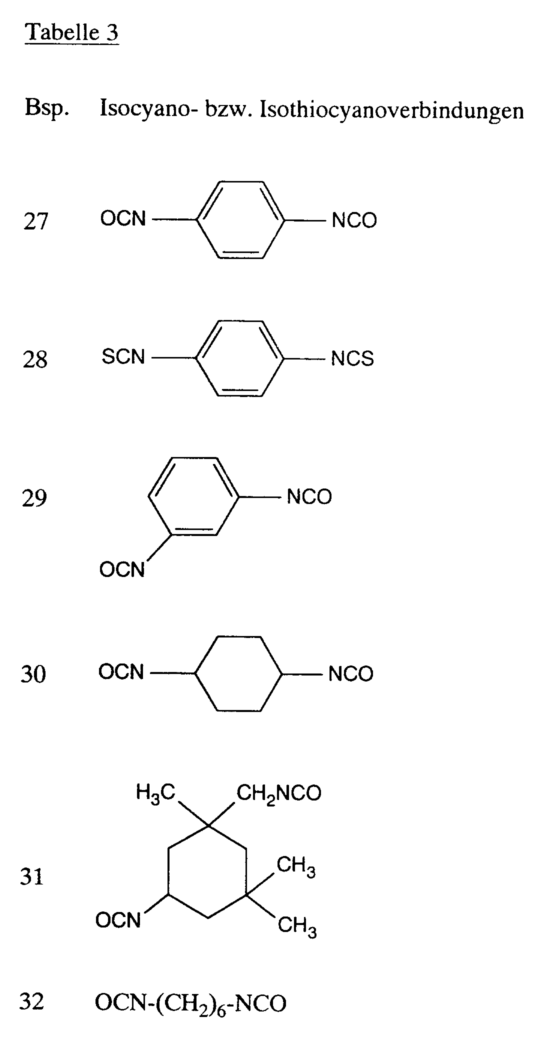

### Färbevorschrift I

12,5 Teile eines nicht mercerisierten, ungebleichten Baumwollgewebes werden mit einem Teil eines nichtionogenen Netzmittels bei einer Temperatur von 80° eingenetzt. Das Baumwollgewebe wird in eine Farbstofflösung, welche 2% des gemäss Schritt b) des Beispiels 3 erhaltenen Farbstoffgemisches und 2 g/l Natriumsulfat enthält, bei einem Flottenverhältnis von 1:20 eingetragen. Anschliessend wird das Färbebad innerhalb von 30 Minuten auf eine Temperatur von 95° erhitzt, es werden 8 g/l Natriumsulfat zugegeben, das Färbebad 45 Minuten bei der Temperatur von 95° belassen, innerhalb von 15 Minuten auf eine Temperatur von 80° abgekühlt und weitere 15 Minuten bei dieser Temperatur belassen. Nach Spülung mit Wasser und Trocknung erhält man ein in einer reinblauen Nuance gefärbtes Baumwollgewebe, welches gute Allgemeinechtheiten aufweist.

### Färbevorschrift II

12,5 Teile Polyamid-6.6-Gewebe werden bei einer Temperatur von 40° in ein Färbebad, welches durch Zugabe von 2 g/l eines Phosphatpuffers auf pH 6 gestellt ist, gegeben. Das Flottenverhältnis beträgt 1:20. Nach 10 Minuten werden 2% des gemäss Schritt b) des Beispiels 3 erhaltenen Farbstoffgemisches zugegeben und das Färbebad innerhalb von 45 Minuten zum Sieden erhitzt und 45 Minuten bei dieser Temperatur belassen. Nach Spülung mit Wasser und Trocknung erhält man ein in einer reinblauen Nuance gefärbtes Polyamid-6.6-Gewebe, welches gute Allgemeinechtheiten aufweist.

## Patentansprüche (Patentansprüche für folgende(n) Vertragsstaat(en): BE, CH, DE, FR, GB, IT, LI)

1. Verbindungen der Formel 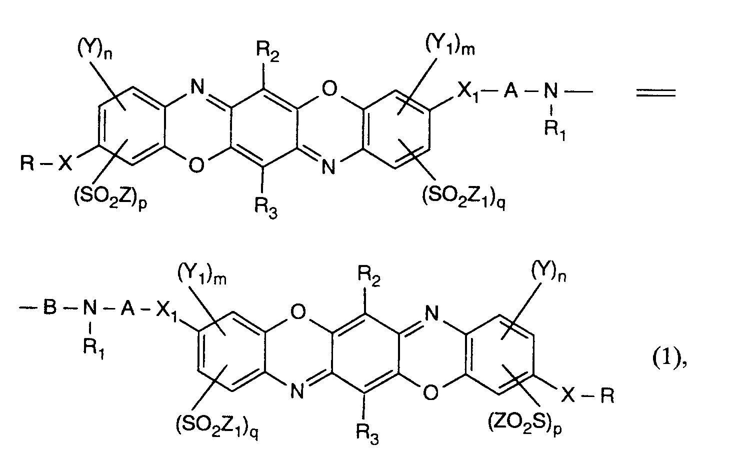 worin
R und R₁ unabhängig voneinander je Wasserstoff; unsubstituiertes oder durch Hydroxy, Sulfo, Sulfato, Chlor, Cyano, oder Acetoxy substituiertes und/oder mit Ausnahme von Methyl gegebenenfalls durch eine Gruppe -O- unterbrochenes C₁-C₄-Alkyl;
unsubstituiertes oder durch 1 bis 3 Methylgruppen substituiertes Cyclopentyl oder Cyclohexyl; unsubstituiertes oder durch Sulfo, Nitro, Chlor, Methyl, Methoxy, N-Methyl- oder N-Ethylamino, N,N-Dimethyl- oder N,N-Diethylamino, Acetylamino, Propionylamino, Benzoylamino, Methoxycarbonyl, Ethoxycarbonyl, Carboxy oder Methylsulfonyl substituiertes Phenyl; unsubstituiertes oder durch Sulfo, Nitro und/oder Chlor substituiertes 1- oder 2-Naphthyl; oder unsubstituiertes oder durch Methyl, Methoxy, Sulfo und/oder Chlor substituiertes Benzyl bedeuten,
X die direkte Bindung, -O-, -S- oder -N(R₄)- ist, wobei R₄ die oben für R und R₁ angegebenen Bedeutungen hat,
X₁ -O-, -S- oder -N(R₄)- ist, wobei R₄ die oben für R und R₁ angegebenen Bedeutungen hat,
Y und Y₁ unabhängig voneinander je C₁-C₄-Alkyl, C₁-C₄-Alkoxy, Halogen, Sulfo, Carboxy, Carbamoyl, N-Mono- oder N,N-Di-C₁-C₄-Alkylcarbamoyl, N-Phenyl- oder N,N-Diphenylcarbamoyl, Sulfamoyl, N-Mono- oder N,N-Di-C₁-C₄-Alkylsulfamoyl oder N-Phenyl- oder N,N-Diphenylsulfamoyl bedeuten,
Z und Z₁ unabhängig voneinander je Hydroxy oder C₁-C₄-Alkyl sind,
R₂ und R₃ unabhängig voneinander je Wasserstoff, Halogen, Cyano, C₁-C₄-Alkyl, C₁-C₄-Alkoxy, gegebenenfalls durch C₁-C₄-Alkyl, C₁-C₄-Alkoxy, Acetylamino, Halogen, Nitro oder Sulfo substituiertes Phenyl, Benzyl, Benzoylamino oder Phenoxy, Sulfo, Carboxy, Carbamoyl, Phenylcarbamoyl oder C₂-C₅-Alkanoylamino bedeuten,
A unsubstituiertes oder durch Hydroxy, Sulfo, Sulfato, C₁-C₄-Alkoxy, Carboxy, Cyano, Halogen, Phenyl, Sulfophenyl oder C₂-C₅-Alkoxycarbonyl substituiertes und gegebenenfalls durch 1-2 Gruppen -O-, -N(R₅)-, worin R₅ C₁-C₄-Alkyl, Acetyl oder Wasserstoff bedeutet, -S- oder -SO₂- unterbrochenes C₂-C₆-Alkylen; oder unsubstituiertes oder einfach oder mehrfach durch C₁-C₃-Alkyl substituiertes Cyclopentylen oder Cyclohexylen; oder
unsubstituiertes oder durch C₁-C₄-Alkyl, C₁-C₄-Alkoxy, Sulfo, Halogen oder Carboxy substituiertes Phenylen, Biphenylen oder Naphthylen; oder
C₁-C₆-Alkylen-phenylen, Phenylen-C₁-C₆-alkylen-phenylen, C₁-C₃-Alkylen-phenylen-C₁-C₃-alkylen oder Methylen-naphthylen-methylen ist, wobei in diesen Aralkylenresten das Phenylen oder Naphthylen keine weiteren Substituenten oder 1 oder 2 Substituenten ausgewählt aus der Gruppe Sulfo, Carboxy, Sulfamoyl, Carbamoyl, Methyl, Ethyl, Methoxy, Ethoxy, Nitro, Chlor, Amino, N-Methyl- und N-Ethylamino, N,N-Dimethyl- und N,N-Diethylamino und Phenylamino enthält,
B ein Rest der Formel
Q-E-Q (2'),
ist, worin Q eine Gruppe der Formel 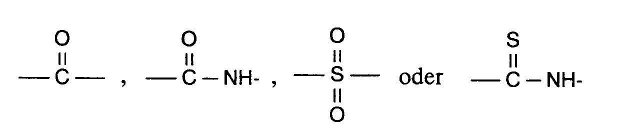 bedeutet, und
E für die direkte Bindung; unsubstituiertes oder durch Hydroxy, Sulfo, Sulfato, Methoxy, Carboxy, Phenyl oder Sulfophenyl substituiertes C₁-C₆-Alkylen oder C₂-C₆-Alkenylen; unsubstituiertes oder durch 1 bis 3 Methylgruppen substituiertes Cyclohexylen oder C₁-C₂-Alkylen-cyclohexylen; Piperazin-1,4-diyl; Thiophen-2,5-diyl; Biphenyl-4,4'-diyl; Stilben-4,4'-diyl; unsubstituiertes oder durch C₁-C₄-Alkyl, C₁-C₄-Alkoxy, Sulfo, Halogen oder Carboxy substituiertes Phenylen oder Naphthylen; oder für unsubstituiertes oder im Phenylteil durch Methyl, Methoxy, Chlor oder Sulfo substituiertes C₁-C₃-Alkylen-phenylen oder C₁-C₂-Alkylen-phenylen-C₁-C₂-alkylen steht; und m, n, p und q unabhängig voneinander je die Zahl 0 oder 1 bedeuten.

2. Verbindungen gemäss Anspruch 1, dadurch gekennzeichnet, dass R und R₁ unabhängig voneinander je Wasserstoff, C₁-C₄-Alkyl, Cyclohexyl, unsubstituiertes oder durch Sulfo, Chlor, Methyl und/oder Methoxy substituiertes Phenyl oder Benzyl bedeuten.

3. Verbindungen gemäss einem der Ansprüche 1 und 2, dadurch gekennzeichnet, dass R und R₁ je Wasserstoff sind.

4. Verbindungen gemäss einem der Ansprüche 1 bis 3, dadurch gekennzeichnet, dass A einen unsubstituierten oder durch Hydroxy, Sulfo, Sulfato, Methoxy, Carboxy oder Sulfophenyl substituierten C₂-C₄-Alkylenrest, -CH₂-CH₂-Z'-CH₂-CH₂- worin Z' -O-, -S-, -SO₂-, -NH- oder -N(CH₃)- bedeutet, einen unsubstituierten oder durch 1 bis 3 Methylgruppen substituierten Cyclohexylenrest, einen unsubstituierten oder durch Sulfo substituierten 1,3- oder 1,4-Phenylenrest oder einen C₁-C₃-Alkylen-phenylen oder C₁-C₂-Alkylen-phenylen-C₁-C₂-alkylenrest, worin das Phenylen jeweils unsubstituiert oder durch Methyl, Methoxy, Chlor oder Sulfo substituiert ist, bedeutet.

5. Verbindungen gemäss einem der Ansprüche 1 bis 4, dadurch gekennzeichnet, dass A unsubstituiertes oder durch Hydroxy, Sulfo, Sulfato, Methoxy, Carboxy oder Sulfophenyl substituiertes C₂-C₄-Alkylen bedeutet.

6. Verbindungen gemäss einem der Ansprüche 1 bis 5, dadurch gekennzeichnet, dass A unsubstituiertes oder durch Hydroxy oder Sulfato substituiertes 1,2-Ethylen oder 1,2- oder 1,3-Propylen bedeutet.

7. Verbindungen gemäss einem der Ansprüche 1 bis 6, dadurch gekennzeichnet, dass X für die Gruppe -N(R₄)-, worin R₄ Wasserstoff, C₁-C₄-Alkyl, Cyclohexyl, unsubstituiertes oder durch Sulfo, Chlor, Methyl und/oder Methoxy substituiertes Phenyl oder Benzyl bedeutet, steht.

8. Verbindungen gemäss Anspruch 7, dadurch gekennzeichnet, dass R₄ Wasserstoff oder C₁-C₄-Alkyl, besonders Wasserstoff, ist.

9. Verbindungen gemäss einem der Ansprüche 1 bis 8, dadurch gekennzeichnet, dass m und n je für die Zahl 0 stehen.

10. Verbindungen gemäss einem der Ansprüche 1 bis 9, dadurch gekennzeichnet, dass p und q je für die Zahl 1 stehen und Z und Z₁ je Hydroxy oder C₁-C₄-Alkyl, besonders Hydroxy, bedeuten.

11. Verbindungen gemäss einem der Ansprüche 1 bis 10, dadurch gekennzeichnet, dass R₂ und R₃ jeweils Wasserstoff, Fluor, Chlor, Brom, Methyl, Methoxy, Acetylamino, Phenoxy oder Cyano bedeuten.

12. Verbindungen gemäss einem der Ansprüche 1 bis 11, dadurch gekennzeichnet, dass R₂ und R₃ je Chlor bedeuten.

13. Verbindungen gemäss einem der Ansprüche 1 bis 12, dadurch gekennzeichnet, dass B ein Brückenglied der Formel 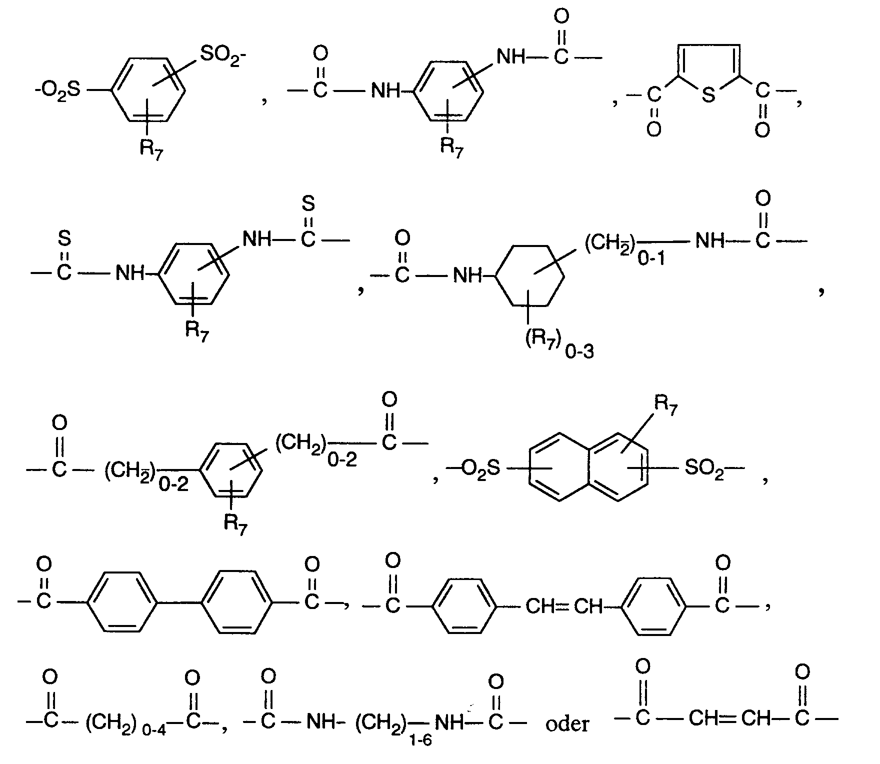 ist, worin R₇ Sulfo, Methyl, Methoxy, Chlor, Carboxy oder vorzugsweise Wasserstoff bedeutet.

14. Verbindungen gemäss einem der Ansprüche 1 bis 13, dadurch gekennzeichnet, dass B einen Rest der Formel 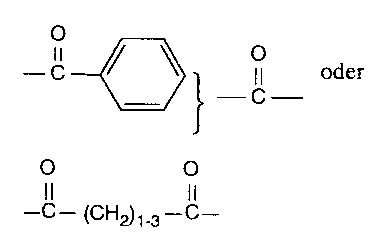 bedeutet.

15. Verbindungen gemäss Anspruch 1 der Formel (1), worin
R und R₁ unabhänging voneinander je für Wasserstoff, unsubstituiertes oder durch Hydroxy, Sulfo, Sulfato, Chlor, Cyano, oder Acetoxy substituiertes und/oder mit Ausnahme von Methyl gegebenenfalls durch eine Gruppe -O- unterbrochenes C₁-C₄-Alkyl;
unsubstituiertes oder durch 1 bis 3 Methylgruppen substituiertes Cyclopentyl oder Cyclohexyl;
unsubstituiertes oder durch Sulfo, Nitro, Chlor, Methyl, Methoxy, N-Methyl- oder N-Ethylamino, N,N-Dimethyl- oder N,N-Diethylamino, Acetylamino, Propionylamino, Benzoylamino, Methoxycarbonyl, Ethoxycarbonyl, Carboxy oder Methylsulfonyl substituiertes Phenyl;
unsubstituiertes oder durch Sulfo, Nitro und/oder Chlor substituiertes 1- oder 2-Naphthyl; oder
für unsubstituiertes oder durch Methyl, Methoxy, Sulfo und/oder Chlor substituiertes Benzyl stehen,
A für einen unsubstituierten oder durch Hydroxy, Sulfo, Sulfato, Methoxy, Carboxy oder Sulfophenyl substituierten C₂-C₄-Alkylenrest;
-CH₂-CH₂-Z'-CH₂-CH₂- worin Z' -O-, -S-, -SO₂-, -NH- oder -N(CH₃)- bedeutet; einen unsubstituierten oder durch 1 bis 3 Methylgruppen substituierten Cyclohexylenrest; einen unsubstituierten oder durch Sulfo substituierten 1,3- oder 1,4-Phenylenrest; oder für einen C₁-C₃-Alkylen-phenylen- oder C₁-C₂-Alkylen-phenylen-C₁-C₂-alkylenrest, worin das Phenylen jeweils unsubstituiert oder durch Methyl, Methoxy, Chlor oder Sulfo substituiert ist; steht,
X und X₁ unabhängig voneinander je eine Gruppe -N(R₄)-, worin R₄ Wasserstoff, C₁-C₄-Alkyl, Cyclohexyl, unsubstituiertes oder durch Sulfo, Chlor, Methyl und/oder Methoxy substituiertes Phenyl oder Benzyl bedeutet, sind,
Y und Y₁ unabhängig voneinander je Methoxy, Methyl, Chlor oder Sulfo sind, n und m unabhängig voneinander je für die Zahl 0 oder 1 stehen,
Z und Z₁ unabhängig voneinander je Hydroxy, Methyl oder Ethyl bedeuten, p und q je die Zahl 1 darstellen, und
R₂ und R₃ jeweils Wasserstoff, Fluor, Chlor, Brom, Methyl, Methoxy, Acetylamino, Phenoxy oder Cyano bedeuten.

16. Verbindungen gemäss Anspruch 1 der Formel 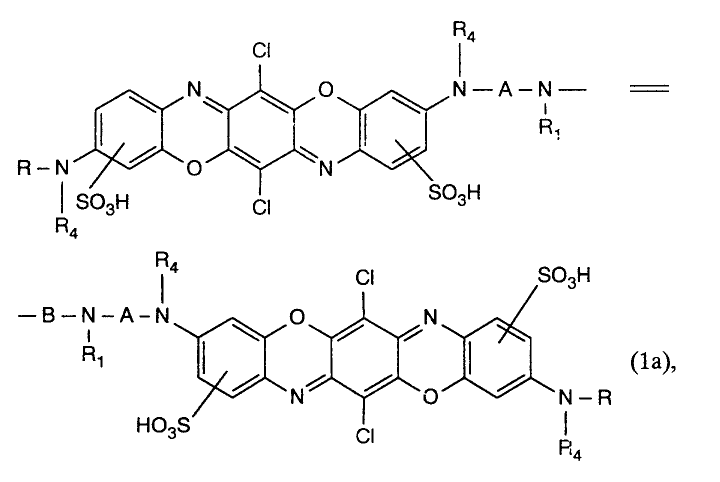 worin
R und R₁ unabhängig voneinander je Wasserstoff, C₁-C₄-Alkyl, Cyclohexyl, unsubstituiertes oder durch Sulfo, Chlor, Methyl und/oder Methoxy substituiertes Phenyl oder Benzyl bedeutet,
A einen unsubstituierten oder durch Hydroxy, Sulfo, Sulfato, Methoxy, Carboxy oder Sulfophenyl substituierten C₂-C₄-Alkylenrest darstellt,
R₄ Wasserstoff, Methyl oder Ethyl bedeutet, und
B für einen Rest der Formel 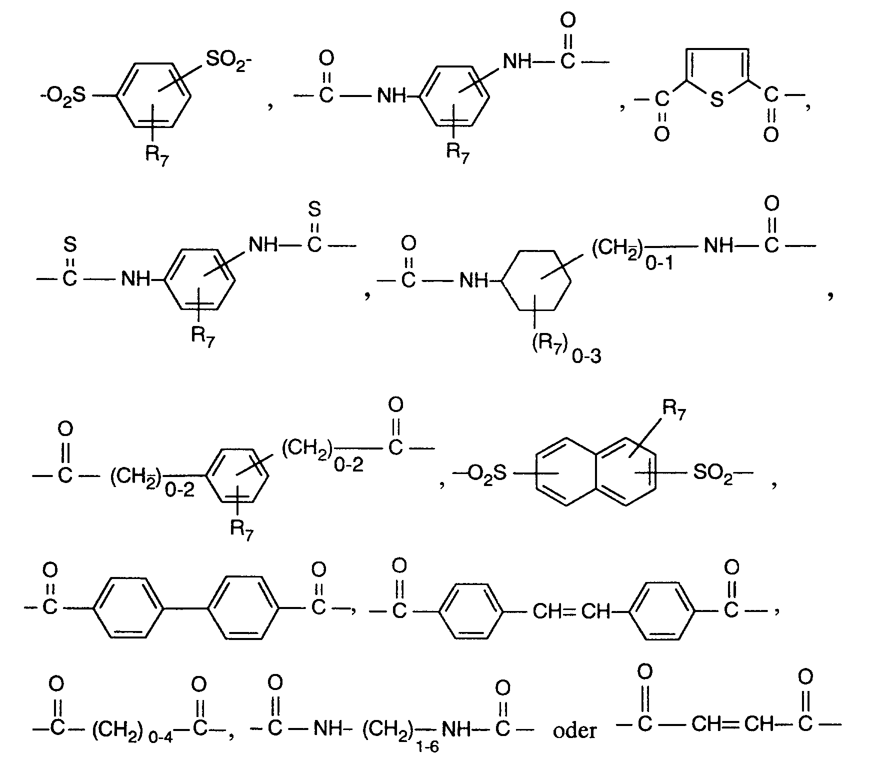 steht, worin R₇ Sulfo, Methyl, Methoxy, Chlor, Carboxy oder vorzugsweise Wasserstoff bedeutet.

17. Verbindungen gemäss Anspruch 1 der Formel 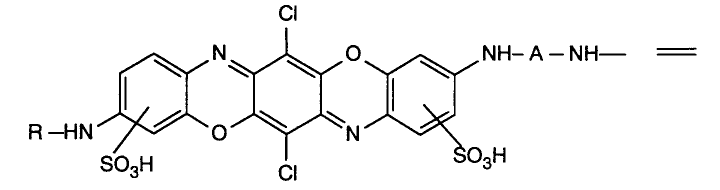 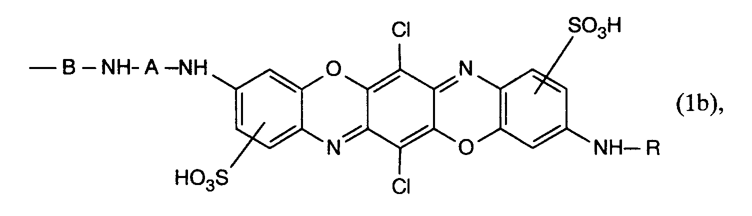 worin
R Methyl, Ethyl, Benzyl oder insbesondere Wasserstoff bedeutet,
A für einen unsubstituierten oder durch Hydroxy oder Sulfato substituierten 1,2-Ethylen- oder 1,2- oder 1,3-Propylenrest steht und
B ein Rest der Formel 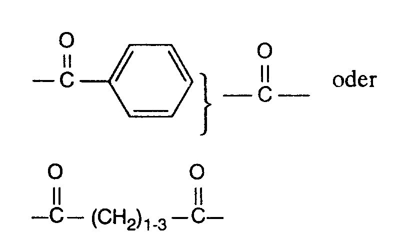 ist.

18. Verfahren zur Herstellung von Verbindungen der Formel (1), dadurch gekennzeichnet, dass man eine Verbindung der Formel 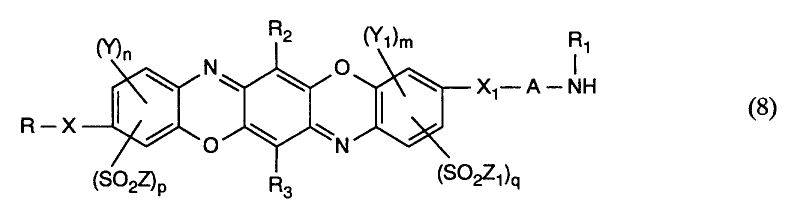 mit einer Verbindung der Formel
T-B-T (9)
kondensiert, worin A, B, R, R₁, R₂, R₃, X, X₁, Y, Y₁, Z, Z₁, m, n, p und q jeweils die im Anspruch 1 angegebene Bedeutung haben und T Halogen, vorzugsweise Chlor, ist.

19. Farbstoffmischungen, erhältlich indem man eine Verbindung der Formel (8) gemäss Anspruch 18 und eine Verbindung der Formel 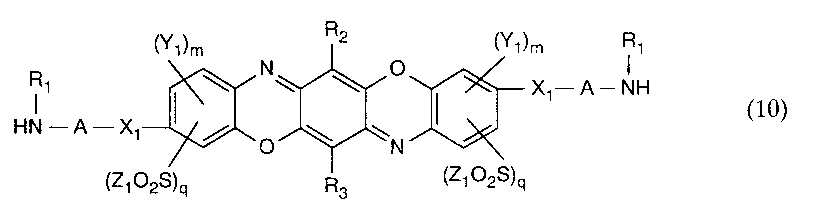 worin A, R₁, R₂, R₃, X₁, Y₁, Z₁, m und q jeweils die im Anspruch 1 angegebene Bedeutung haben, mit einer Verbindung der Formel (9) gemäss Anspruch 18 kondensiert.

20. Farbstoffmischungen gemäss Anspruch 19, dadurch gekennzeichnet, dass man die Verbindungen der Formeln (8) und (10) im Gewichtsverhältnis von 90:10 bis 60:40, vorzugsweise 80:20 bis 65:35 einsetzt.

21. Verwendung der Verbindungen der Formel (1) sowie der durch Umsetzung der Verbindungen der Formeln (8), (9) und (10) gemäss Anspruch 19 erhältlichen Verbindungsgemische als Farbstoffe zum Färben und Bedrucken von stickstoffhaltigen und insbesondere cellulosischen Fasermaterialien.

22. Verwendung nach Anspruch 21, dadurch gekennzeichnet, dass man Fasergemische aus Synthesefasern und cellulosischen Fasermaterialien, insbesondere Polyester/Baumwoll-Mischgewebe, in Gegenwart eines Dispersionsfarbstoffes für die Polyesterfasern unter den Färbebedingungen für Polyesterfasern färbt.

23. Verfahren zum Färben von Polyester/Baumwoll-Mischgeweben mit Dispersions- und Direktfarbstoffen, dadurch gekennzeichnet, dass man in einem einstufigen, einbadigen Verfahren neben den Dispersionsfarbstoffen Verbindungen der Formel (1) gemäss Anspruch 1 oder gemäss Anspruch 19 erhältliche Verbindungsgemische als Farbstoffe verwendet und aus wässriger Flotte bei Temperaturen im Bereich von 100 bis 150°C, vorzugsweise 120 bis 130°C, und einem pH-Wert zwischen 4 und 7,5 färbt.

## Patentansprüche (Patentansprüche für folgende(n) Vertragsstaat(en): ES)

1. Verfahren zur Herstellung von Verbindungen der Formel 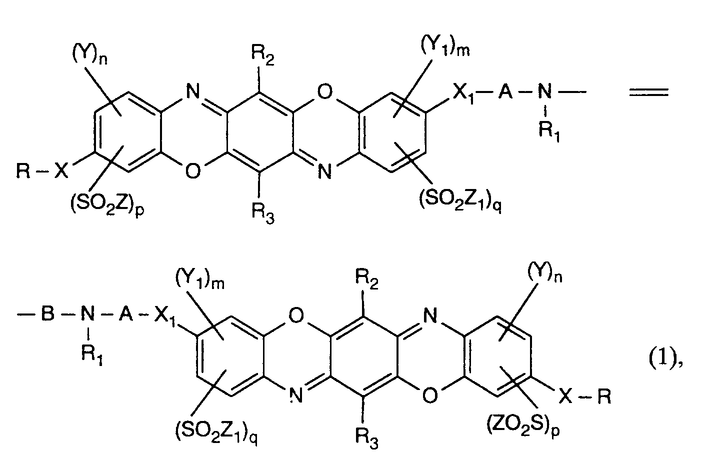 worin
R und R₁ unabhängig voneinander je Wasserstoff; unsubstituiertes oder durch Hydroxy, Sulfo, Sulfato, Chlor, Cyano, oder Acetoxy substituiertes und/oder mit Ausnahme von Methyl gegebenenfalls durch eine Gruppe -O- unterbrochenes C₁-C₄-Alkyl;
unsubstituiertes oder durch 1 bis 3 Methylgruppen substituiertes Cyclopentyl oder Cyclohexyl; unsubstituiertes oder durch Sulfo, Nitro, Chlor, Methyl, Methoxy, N-Methyl- oder N-Ethylamino, N,N-Dimethyl- oder N,N-Diethylamino, Acetylamino, Propionylamino, Benzoylamino, Methoxycarbonyl, Ethoxycarbonyl, Carboxy oder Methylsulfonyl substituiertes Phenyl; unsubstituiertes oder durch Sulfo, Nitro und/oder Chlor substituiertes 1- oder 2-Naphthyl; oder unsubstituiertes oder durch Methyl, Methoxy, Sulfo und/oder Chlor substituiertes Benzyl bedeuten,
X die direkte Bindung, -O-, -S- oder -N(R₄)- ist, wobei R₄ die oben für R und R₁ angegebenen Bedeutungen hat,
X₁ -O-, -S- oder -N(R₄)- ist, wobei R₄ die oben für R und R₁ angegebenen Bedeutungen hat,
Y und Y₁ unabhängig voneinander je C₁-C₄-Alkyl, C₁-C₄-Alkoxy, Halogen, Sulfo, Carboxy, Carbamoyl, N-Mono- oder N,N-Di-C₁-C₄-Alkylcarbamoyl, N-Phenyl- oder N,N-Diphenylcarbamoyl, Sulfamoyl, N-Mono- oder N,N-Di-C₁-C₄-Alkylsulfamoyl oder N-Phenyl- oder N,N-Diphenylsulfamoyl bedeuten,
Z und Z₁ unabhängig voneinander je Hydroxy oder C₁-C₄-Alkyl sind,
R₂ und R₃ unabhängig voneinander je Wasserstoff, Halogen, Cyano, C₁-C₄-Alkyl, C₁-C₄-Alkoxy, gegebenenfalls durch C₁-C₄-Alkyl, C₁-C₄-Alkoxy, Acetylamino, Halogen, Nitro oder Sulfo substituiertes Phenyl, Benzyl, Benzoylamino oder Phenoxy, Sulfo, Carboxy, Carbamoyl, Phenylcarbamoyl oder C₂-C₅-Alkanoylamino bedeuten,
A unsubstituiertes oder durch Hydroxy, Sulfo, Sulfato, C₁-C₄-Alkoxy, Carboxy, Cyano, Halogen, Phenyl, Sulfophenyl oder C₂-C₅-Alkoxycarbonyl substituiertes und gegebenenfalls durch 1-2 Gruppen -O-, -N(R₅)-, worin R₅ C₁-C₄-Alkyl, Acetyl oder Wasserstoff bedeutet, -S- oder -SO₂- unterbrochenes C₂-C₆-Alkylen; oder unsubstituiertes oder einfach oder mehrfach durch C₁-C₃-Alkyl substituiertes Cyclopentylen oder Cyclohexylen; oder
unsubstituiertes oder durch C₁-C₄-Alkyl, C₁-C₄-Alkoxy, Sulfo, Halogen oder Carboxy substituiertes Phenylen, Biphenylen oder Naphthylen; oder
C₁-C₆-Alkylen-phenylen, Phenylen-C₁-C₆-alkylen-phenylen, C₁-C₃-Alkylen-phenylen-C₁-C₃-alkylen oder Methylen-naphthylen-methylen ist, wobei in diesen Aralkylenresten das Phenylen oder Naphthylen keine weiteren Substituenten oder 1 oder 2 Substituenten ausgewählt aus der Gruppe Sulfo, Carboxy, Sulfamoyl, Carbamoyl, Methyl, Ethyl, Methoxy, Ethoxy, Nitro, Chlor, Amino, N-Methyl- und N-Ethylamino, N,N-Dimethyl- und N,N-Diethylamino und Phenylamino enthält,
B ein Rest der Formel
Q-E-Q (2'),
ist, worin Q eine Gruppe der Formel 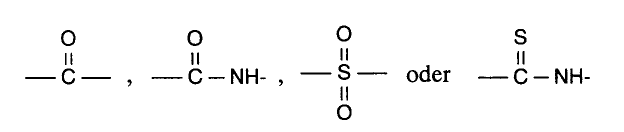 bedeutet, und
E für die direkte Bindung; unsubstituiertes oder durch Hydroxy, Sulfo, Sulfato, Methoxy, Carboxy, Phenyl oder Sulfophenyl substituiertes C₁-C₆-Alkylen oder C₂-C₆-Alkenylen; unsubstituiertes oder durch 1 bis 3 Methylgruppen substituiertes Cyclohexylen oder C₁-C₂-Alkylen-cyclohexylen; Piperazin-1,4-diyl; Thiophen-2,5-diyl; Biphenyl-4,4'-diyl; Stilben-4,4'-diyl; unsubstituiertes oder durch C₁-C₄-Alkyl, C₁-C₄-Alkoxy, Sulfo, Halogen oder Carboxy substituiertes Phenylen oder Naphthylen; oder für unsubstituiertes oder im Phenylteil durch Methyl, Methoxy, Chlor oder Sulfo substituiertes
C₁-C₃-Alkylen-phenylen oder C₁-C₂-Alkylen-phenylen-C₁-C₂-alkylen steht; und m, n, p und q unabhängig voneinander je die Zahl 0 oder 1 bedeuten, dadurch gekennzeichnet, dass man eine Verbindung der Formel 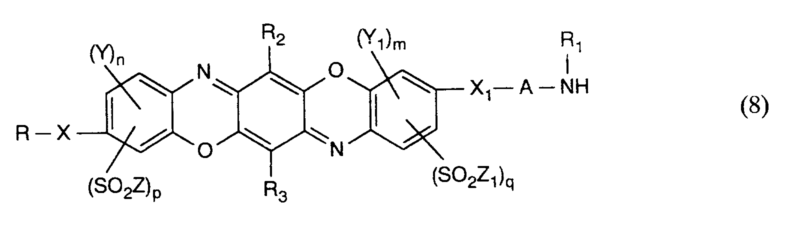 mit einer Verbindung der Formel
T-B-T (9)
kondensiert, worin A, B, R, R₁, R₂, R₃, X, X₁, Y, Y₁, Z, Z₁, m, n, p und q jeweils die unter Formel 1 angegebene Bedeutung haben und T Halogen, vorzugsweise Chlor, ist.

2. Verfahren gemäss Anspruch 1, dadurch gekennzeichnet, dass man eine Verbindung der Formel (8) verwendet, worin R und R₁ unabhängig voneinander je Wasserstoff, C₁-C₄-Alkyl, Cyclohexyl, unsubstituiertes oder durch Sulfo, Chlor, Methyl und/oder Methoxy substituiertes Phenyl oder Benzyl bedeuten.

3. Verfahren gemäss einem der Ansprüche 1 und 2, dadurch gekennzeichnet, dass man eine Verbindung der Formel (8) verwendet, worin R und R₁ je Wasserstoff sind.

4. Verfahren gemäss einem der Ansprüche 1 bis 3, dadurch gekennzeichnet, dass man eine Verbindung der Formel (8) verwendet, worin
A einen unsubstituierten oder durch Hydroxy, Sulfo, Sulfato, Methoxy, Carboxy oder Sulfophenyl substituierten C₂-C₄-Alkylenrest;
-CH₂-CH₂-Z'-CH₂-CH₂- worin Z' -O-, -S-, -SO₂-, -NH- oder -N(CH₃)- bedeutet; einen unsubstituierten oder durch 1 bis 3 Methylgruppen substituierten Cyclohexylenrest; einen unsubstituierten oder durch Sulfo substituierten 1,3- oder 1,4-Phenylenrest; oder einen C₁-C₃-Alkylen-phenylen oder C₁-C₂-Alkylen-phenylen-C₁-C₂-alkylenrest, worin das Phenylen jeweils unsubstituiert oder durch Methyl, Methoxy, Chlor oder Sulfo substituiert ist, bedeutet.

5. Verfahren gemäss einem der Ansprüche 1 bis 4, dadurch gekennzeichnet, dass man eine Verbindung der Formel (8) verwendet, worin A unsubstituiertes oder durch Hydroxy, Sulfo, Sulfato, Methoxy, Carboxy oder Sulfophenyl substituiertes C₂-C₄-Alkylen bedeutet.

6. Verfahren gemäss einem der Ansprüche 1 bis 5, dadurch gekennzeichnet, dass man eine Verbindung der Formel (8) verwendet, worin A unsubstituiertes oder durch Hydroxy oder Sulfato substituiertes 1,2-Ethylen oder 1,2- oder 1,3-Propylen bedeutet.

7. Verfahren gemäss einem der Ansprüche 1 bis 6, dadurch gekennzeichnet, dass man eine Verbindung der Formel (8) verwendet, worin X für die Gruppe -N(R₄)-, worin R₄ Wasserstoff, C₁-C₄-Alkyl, Cyclohexyl, unsubstituiertes oder durch Sulfo, Chlor, Methyl und/oder Methoxy substituiertes Phenyl oder Benzyl bedeutet, steht.

8. Verfahren gemäss Anspruch 7, dadurch gekennzeichnet, dass man eine Verbindung der Formel (8) verwendet, worin R₄ Wasserstoff oder C₁-C₄-Alkyl, besonders Wasserstoff, ist.

9. Verfahren gemäss einem der Ansprüche 1 bis 8, dadurch gekennzeichnet, dass man eine Verbindung der Formel (8) verwendet, worin m und n je für die Zahl 0 stehen.

10. Verfahren gemäss einem der Ansprüche 1 bis 9, dadurch gekennzeichnet, dass man eine Verbindung der Formel (8) verwendet, worin p und q je für die Zahl 1 stehen und Z und Z₁ je Hydroxy oder C₁-C₄-Alkyl, besonders Hydroxy, bedeuten.

11. Verfahren gemäss einem der Ansprüche 1 bis 10, dadurch gekennzeichnet, dass man eine Verbindung der Formel (8) verwendet, worin R₂ und R₃ jeweils Wasserstoff, Fluor, Chlor, Brom, Methyl, Methoxy, Acetylamino, Phenoxy oder Cyano bedeuten.

12. Verfahren gemäss einem der Ansprüche 1 bis 11, dadurch gekennzeichnet, dass man eine Verbindung der Formel (8) verwendet, worin R₂ und R₃ je Chlor bedeuten.

13. Verfahren gemäss einem der Ansprüche 1 bis 12, dadurch gekennzeichnet, dass man eine Verbindung der Formel (9) verwendet, worin B ein Brückenglied der Formel 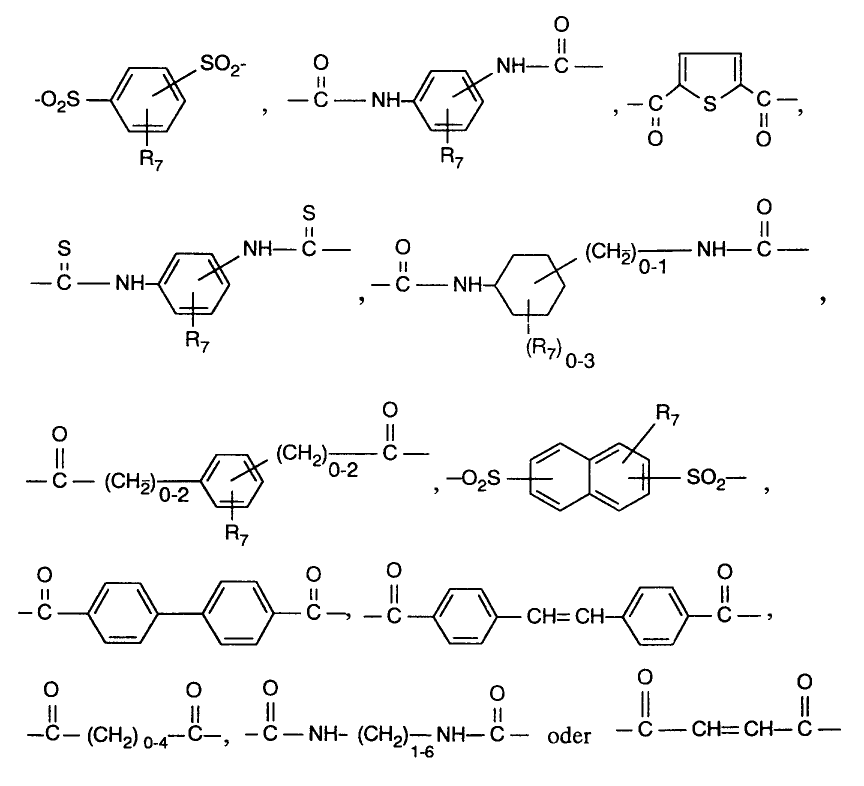 ist, worin R₇ Sulfo, Methyl, Methoxy, Chlor, Carboxy oder vorzugsweise Wasserstoff bedeutet.

14. Verfahren gemäss einem der Ansprüche 1 bis 13, dadurch gekennzeichnet, dass man eine Verbindung der Formel (9) verwendet, worin B einen Rest der Formel 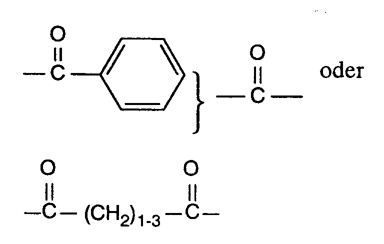 bedeutet.

15. Verfahren gemäss Anspruch 1, dadurch gekennzeichnet, dass man eine Verbindung der Formel (8), worin
R und R₁ unabhänging voneinander je für Wasserstoff;
unsubstituiertes oder durch Hydroxy, Sulfo, Sulfato, Chlor, Cyano, oder Acetoxy substituiertes und/oder mit Ausnahme von Methyl gegebenenfalls durch eine Gruppe -O-unterbrochenes C₁-C₄-Alkyl;
unsubstituiertes oder durch 1 bis 3 Methylgruppen substituiertes Cyclopentyl oder Cyclohexyl;
unsubstituiertes oder durch Sulfo, Nitro, Chlor, Methyl, Methoxy, N-Methyl- oder N-Ethylamino, N,N-Dimethyl- oder N,N-Diethylamino, Acetylamino, Propionylamino, Benzoylamino, Methoxycarbonyl, Ethoxycarbonyl, Carboxy oder Methylsulfonyl substituiertes Phenyl;
unsubstituiertes oder durch Sulfo, Nitro und/oder Chlor substituiertes 1- oder 2-Naphthyl; oder
für unsubstituiertes oder durch Methyl, Methoxy, Sulfo und/oder Chlor substituiertes Benzyl stehen,
A für einen unsubstituierten oder durch Hydroxy, Sulfo, Sulfato, Methoxy, Carboxy oder Sulfophenyl substituierten C₂-C₄-Alkylenrest;
-CH₂-CH₂-Z'-CH₂-CH₂- worin Z' -O-, -S-, -SO₂-, -NH- oder -N(CH₃)- bedeutet; einen unsubstituierten oder durch 1 bis 3 Methylgruppen substituierten Cyclohexylenrest; einen unsubstituierten oder durch Sulfo substituierten 1,3- oder 1,4-Phenylenrest; oder für einen C₁-C₃-Alkylen-phenylen- oder C₁-C₂-Alkylen-phenylen-C₁-C₂-alkylenrest, worin das Phenylen jeweils unsubstituiert oder durch Methyl, Methoxy, Chlor oder Sulfo substituiert ist; steht,
X und X₁ unabhängig voneinander je eine Gruppe -N(R₄)-, worin R₄ Wasserstoff, C₁-C₄-Alkyl, Cyclohexyl, unsubstituiertes oder durch Sulfo, Chlor, Methyl und/oder Methoxy substituiertes Phenyl oder Benzyl bedeutet, sind,
Y und Y₁ unabhängig voneinander je Methoxy, Methyl, Chlor oder Sulfo sind,
n und m unabhängig voneinander je für die Zahl 0 oder 1 stehen,
Z und Z₁ unabhängig voneinander je Hydroxy, Methyl oder Ethyl bedeuten,
p und q je die Zahl 1 darstellen und
R₂ und R₃ jeweils Wasserstoff, Fluor, Chlor, Brom, Methyl, Methoxy, Acetylamino, Phenoxy oder Cyano bedeuten.

16. Verfahren gemäss Anspruch 1, dadurch gekennzeichnet, dass man eine Verbindung der Formel 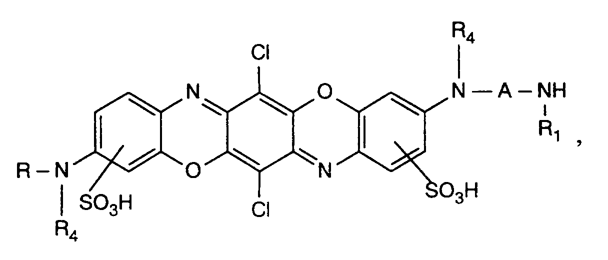 worin
R und R₁ unabhängig voneinander je Wasserstoff, C₁-C₄-Alkyl, Cyclohexyl, unsubstituiertes oder durch Sulfo, Chlor, Methyl und/oder Methoxy substituiertes Phenyl oder Benzyl bedeutet,
A einen unsubstituierten oder durch Hydroxy, Sulfo, Sulfato, Methoxy, Carboxy oder Sulfophenyl substituierten C₂-C₄-Alkylenrest darstellt, und
R₄ Wasserstoff, Methyl oder Ethyl bedeutet,
mit einer Verbindung der Formel (9) kondensiert, worin B für einen Rest der Formel 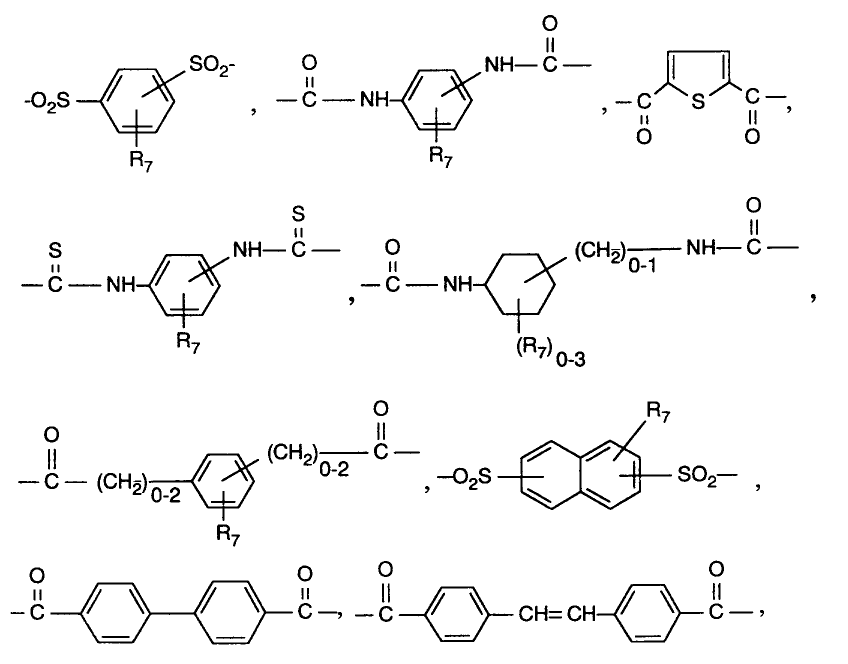 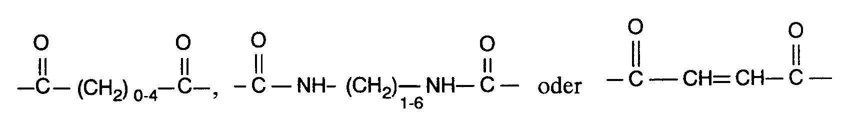 steht, worin R₇ Sulfo, Methyl, Methoxy, Chlor, Carboxy oder vorzugsweise Wasserstoff bedeutet.

17. Verfahren gemäss Anspruch 1, dadurch gekennzeichnet, dass man eine Verbindung der Formel 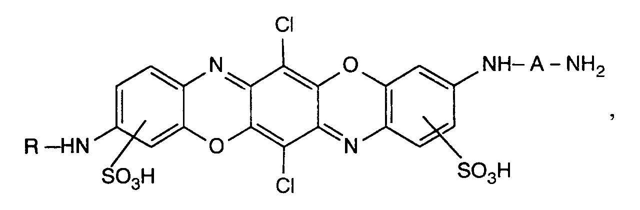 worin R Methyl, Ethyl, Benzyl oder insbesondere Wasserstoff bedeutet und
A für einen unsubstituierten oder durch Hydroxy oder Sulfato substituierten 1,2-Ethylen- oder 1,2- oder 1,3-Propylenrest steht,
mit einer Verbindung der Formel (9) kondensiert, worin B ein Rest der Formel 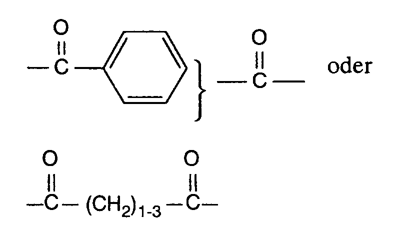 ist.

18. Verfahren zur Herstellung von Farbstoffmischungen, dadurch gekennzeichnet, dass man eine Verbindung der Formel (8) gemäss Anspruch 1 und eine Verbindung der Formel 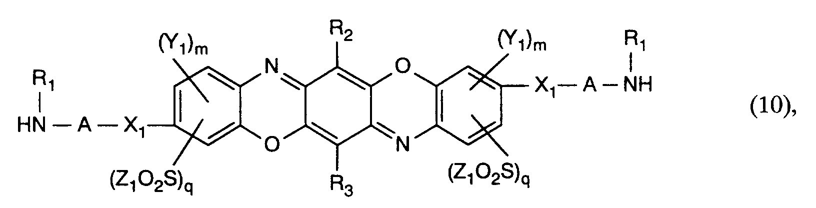 worin A, R₁, R₂, R₃, X₁, Y₁, Z₁, m und q jeweils die im Anspruch 1 angegebene Bedeutung haben, mit einer Verbindung der Formel (9) gemäss Anspruch 1 kondensiert.

19. Verfahren zur Herstellung von Farbstoffmischungen gemäss Anspruch 18, dadurch gekennzeichnet, dass man die Verbindungen der Formeln (8) und (10) im Gewichtsverhältnis von 90:10 bis 60:40, vorzugsweise 80:20 bis 65:35 einsetzt.

20. Verbindungen der Formel 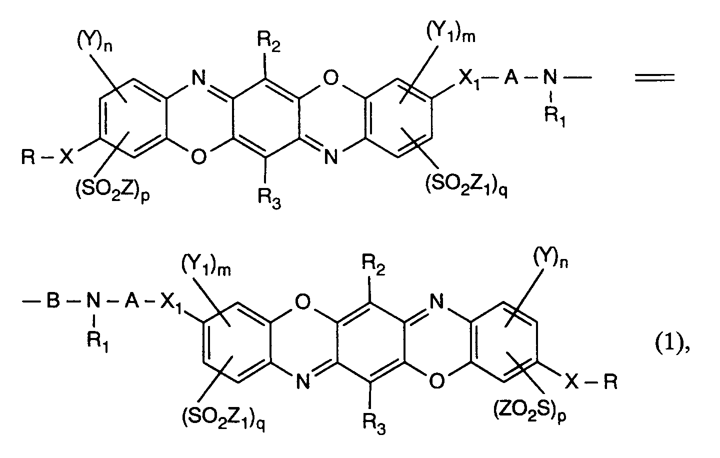 worin
R und R₁ unabhängig voneinander je Wasserstoff; unsubstituiertes oder durch Hydroxy, Sulfo, Sulfato, Chlor, Cyano, oder Acetoxy substituiertes und/oder mit Ausnahme von Methyl gegebenenfalls durch eine Gruppe -O- unterbrochenes C₁-C₄-Alkyl;
unsubstituiertes oder durch 1 bis 3 Methylgruppen substituiertes Cyclopentyl oder Cyclohexyl; unsubstituiertes oder durch Sulfo, Nitro, Chlor, Methyl, Methoxy, N-Methyl- oder N-Ethylamino, N,N-Dimethyl- oder N,N-Diethylamino, Acetylamino, Propionylamino, Benzoylamino, Methoxycarbonyl, Ethoxycarbonyl, Carboxy oder Methylsulfonyl substituiertes Phenyl; unsubstituiertes oder durch Sulfo, Nitro und/oder Chlor substituiertes 1- oder 2-Naphthyl; oder unsubstituiertes oder durch Methyl, Methoxy, Sulfo und/oder Chlor substituiertes Benzyl bedeuten,
X die direkte Bindung, -O-, -S- oder -N(R₄)- ist, wobei R₄ die oben für R und R₁ angegebenen Bedeutungen hat,
X₁ -O-, -S- oder -N(R₄)- ist, wobei R₄ die oben für R und R₁ angegebenen Bedeutungen hat,
Y und Y₁ unabhängig voneinander je C₁-C₄-Alkyl, C₁-C₄-Alkoxy, Halogen, Sulfo, Carboxy, Carbamoyl, N-Mono- oder N,N-Di-C₁-C₄-Alkylcarbamoyl, N-Phenyl- oder N,N-Diphenylcarbamoyl, Sulfamoyl, N-Mono- oder N,N-Di-C₁-C₄-Alkylsulfamoyl oder N-Phenyl- oder N,N-Diphenylsulfamoyl bedeuten,
Z und Z₁ unabhängig voneinander je Hydroxy oder C₁-C₄-Alkyl sind,
R₂ und R₃ unabhängig voneinander je Wasserstoff, Halogen, Cyano, C₁-C₄-Alkyl, C₁-C₄-Alkoxy, gegebenenfalls durch C₁-C₄-Alkyl, C₁-C₄-Alkoxy, Acetylamino, Halogen, Nitro oder Sulfo substituiertes Phenyl, Benzyl, Benzoylamino oder Phenoxy, Sulfo, Carboxy, Carbamoyl, Phenylcarbamoyl oder C₂-C₅-Alkanoylamino bedeuten,
A unsubstituiertes oder durch Hydroxy, Sulfo, Sulfato, C₁-C₄-Alkoxy, Carboxy, Cyano, Halogen, Phenyl, Sulfophenyl oder C₂-C₅-Alkoxycarbonyl substituiertes und gegebenenfalls durch 1-2 Gruppen -O-, -N(R₅)-, worin R₅ C₁-C₄-Alkyl, Acetyl oder Wasserstoff bedeutet, -S- oder -SO₂- unterbrochenes C₂-C₆-Alkylen; oder unsubstituiertes oder einfach oder mehrfach durch C₁-C₃-Alkyl substituiertes Cyclopentylen oder Cyclohexylen; oder
unsubstituiertes oder durch C₁-C₄-Alkyl, C₁-C₄-Alkoxy, Sulfo, Halogen oder Carboxy substituiertes Phenylen, Biphenylen oder Naphthylen; oder
C₁-C₆-Alkylen-phenylen, Phenylen-C₁-C₆-alkylen-phenylen, C₁-C₃-Alkylen-phenylen-C₁-C₃-alkylen oder Methylen-naphthylen-methylen ist, wobei in diesen Aralkylenresten das Phenylen oder Naphthylen keine weiteren Substituenten oder 1 oder 2 Substituenten ausgewählt aus der Gruppe Sulfo, Carboxy, Sulfamoyl, Carbamoyl, Methyl, Ethyl, Methoxy, Ethoxy, Nitro, Chlor, Amino, N-Methyl- und N-Ethylamino, N,N-Dimethyl- und N,N-Diethylamino und Phenylamino enthält,
B ein Rest der Formel
Q-E-Q (2'),
ist, worin Q eine Gruppe der Formel 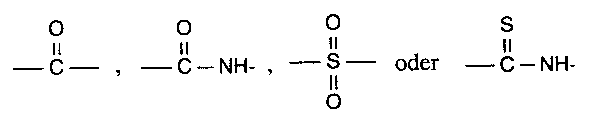 bedeutet, und
E für die direkte Bindung; unsubstituiertes oder durch Hydroxy, Sulfo, Sulfato, Methoxy, Carboxy, Phenyl oder Sulfophenyl substituiertes C₁-C₆-Alkylen oder C₂-C₆-Alkenylen;
unsubstituiertes oder durch 1 bis 3 Methylgruppen substituiertes Cyclohexylen oder C₁-C₂-Alkylen-cyclohexylen; Piperazin-1,4-diyl; Thiophen-2,5-diyl; Biphenyl-4,4'-diyl; Stilben-4,4'-diyl; unsubstituiertes oder durch C₁-C₄-Alkyl, C₁-C₄-Alkoxy, Sulfo, Halogen oder Carboxy substituiertes Phenylen oder Naphthylen; oder für unsubstituiertes oder im Phenylteil durch Methyl, Methoxy, Chlor oder Sulfo substituiertes
C₁-C₃-Alkylen-phenylen oder C₁-C₂-Alkylen-phenylen-C₁-C₂-alkylen steht; und
m, n, p und q unabhängig voneinander je die Zahl 0 oder 1 bedeuten.

21. Farbstoffmischungen, erhältlich indem man eine Verbindung der Formel (8) gemäss Anspruch 1 und eine Verbindung der Formel 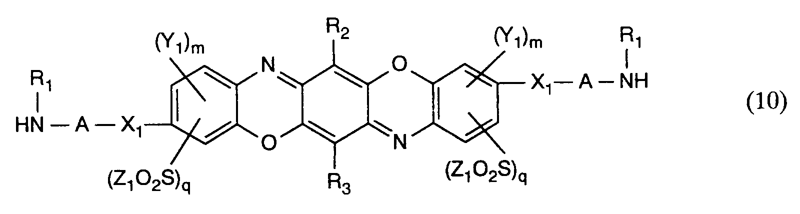 worin A, R₁, R₂, R₃, X₁, Y₁, Z₁, m und q jeweils die im Anspruch 1 angegebene Bedeutung haben, mit einer Verbindung der Formel (9) gemäss Anspruch 1 kondensiert.

22. Verwendung der gemäss Anspruch 1 erhältlichen Verbindungen der Formel (1) sowie der durch Umsetzung der Verbindungen der Formeln (8), (9) und (10) gemäss Anspruch 18 erhältlichen Verbindungsgemische als Farbstoffe zum Färben und Bedrucken von stickstoffhaltigen und insbesondere cellulosischen Fasermaterialien.

23. Verwendung nach Anspruch 22, dadurch gekennzeichnet, dass man Fasergemische aus Synthesefasern und cellulosischen Fasermaterialien, insbesondere Polyester/Baumwoll-Mischgewebe, in Gegenwart eines Dispersionsfarbstoffes für die Polyesterfasern unter den Färbebedingungen für Polyesterfasern färbt.

24. Verfahren zum Färben von Polyester/Baumwoll-Mischgeweben mit Dispersions- und Direktfarbstoffen, dadurch gekennzeichnet, dass man in einem einstufigen, einbadigen Verfahren neben den Dispersionsfarbstoffen gemäss Anspruch 1 erhältliche Verbindungen der Formel (1) oder gemäss Anspruch 18 erhältliche Verbindungsgemische als Farbstoffe verwendet und aus wässriger Flotte bei Temperaturen im Bereich von 100 bis 150°C, vorzugsweise 120 bis 130°C, und einem pH-Wert zwischen 4 und 7,5 färbt.

## Claims (Claims for the following Contracting State(s): BE, CH, DE, FR, GB, IT, LI)

1. A compound of formula 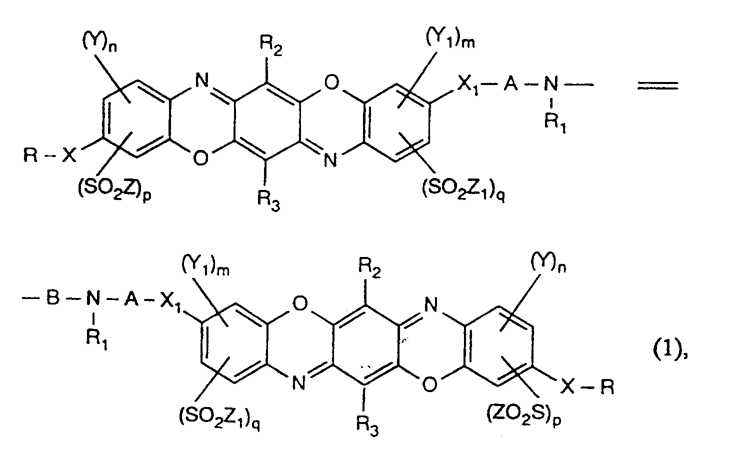
wherein R and R₁ are each independently of the other hydrogen;
unsubstituted C₁-C₄alkyl or C₁-C₄alkyl which is substituted by hydroxy, sulfo, sulfato, chloro, cyano or acetoxy and/or, with the exception of methyl, may be interrupted by a group -O-; cyclopentyl or cyclohexyl which are unsubstituted or substituted by 1 to 3 methyl groups;
unsubstituted phenyl or phenyl which is substituted by sulfo, nitro, chloro, methyl, methoxy, N-methylamino or N-ethylamino, N,N-dimethyl-amino or N,N-diethylamino, acetylamino, propionylamino, benzoylamino, methoxycarbonyl, ethoxycarbonyl, carboxy or methylsulfonyl;
unsubstituted 1- or 2-naphthyl or 1- or 2-naphthyl which is substituted by sulfo, nitro and/or chloro; or unsubstituted benzyl or benzyl which is substituted by methyl, methoxy, sulfo and/or chloro,
X is a direct bond, -O-, -S- or -N(R₄)-, wherein R₄ is as defined above for R and R₁,
X₁ is -O-, -S- or -N(R₄)-, wherein R₄ is as defined above for R and R₁,
Y and Y₁ are each independently of the other C₁-C₄alkyl, C₁-C₄alkoxy, halogen, sulfo, carboxy, carbamoyl, N-mono- or N,N-di-C₁-C₄alkylcarbamoyl, N-phenyl- or N,N-diphenylcarbamoyl, sulfamoyl, N-mono- or N,N-di-C₁-C₄alkylsulfamoyl or N-phenyl- or N,N-diphenylsulfamoyl,
Z and Z₁ are each independently of the other hydroxy or C₁-C₄alkyl,
R₂ and R₃ are each independently of the other hydrogen, halogen, cyano, C₁-C₄alkyl, C₁-C₄alkoxy, unsubstituted or C₁-C₄alkyl-, C₁-C₄alkoxy, acetylamino-, halogen-, nitro- or sulfo-substituted phenyl, benzyl, benzoylamino or phenoxy, sulfo, carboxy, carbamoyl, phenylcarbamoyl or C₂-C₅alkanoylamino,
A is C₂-C₆alkylene which is unsubstituted or substituted by hydroxy, sulfo, sulfato, C₁-C₄alkoxy, carboxy, cyano, halogen, phenyl, sulfophenyl or C₂-C₅alkoxycarbonyl and is uninterrupted or interrupted by 1-2 groups -O-, -N(R₅)- wherein R₅ is C₁-C₄alkyl, acetyl or hydrogen, -S- or -SO₂-; or is cyclohexylene or cyclopentylene which are unsubstituted or substituted one or more times by C₁-C₃alkyl; or is
naphthylene, biphenylene or phenylene which are unsubstituted or substituted by C₁-C₄alkyl, C₁-C₄alkoxy, sulfo, halogen or carboxy; or is C₁-C₆alkylene-phenylene, phenylene-C₁-C₆alkylene-phenylene, C₁-C₃alkylene-phenylene-C₁-C₃alkylene or methylene-naphthylene-methylene, in which aralkylene radicals the phenylene or naphthylene contains no further substituents or contains 1 or 2 substituents selected from the group consisting of sulfo, carboxy, sulfamoyl, carbamoyl, methyl, ethyl, methoxy, ethoxy, nitro, chloro, amino, N-methylamino and N-ethylamino, N,N-dimethylamino and N,N-diethylamino and phenylamino;
B is a radical of the formula
Q-E-Q (2'),
wherein Q is a group of the formula, 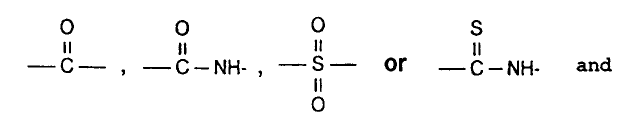
E is a direct bond; C₁-C₆alkylene or C₂-C₆alkenylene which are unsubstituted or substituted by hydroxy, sulfo, sulfato, methoxy, carboxy, phenyl or sulfophenyl; cyclohexylene or C₁-C₂alkylene-cyclohexylene which are unsubstituted or substituted by 1 to 3 methyl groups; piperazine-1,4-diyl; thiophene-2,5-diyl; biphenyl-4,4'-diyl; stilbene-4,4'-diyl; unsubstituted phenylene or naphthylene, or phenylene or naphthylene which are substituted by C₁-C₄-alkyl, C₁-C₄alkoxy, sulfo, halogen or carboxy; or is C₁-C₃alkylene-phenylene or C₁-C₂ alkylene-phenylene-C₁-C₂ alkylene which are unsubstitued or subsituted in the phenyl moiety by methyl, methoxy, chloro or sulfo; and m, n, p and q are each independently of one another 0 or 1.

2. A compound according to claim 1, wherein R and R₁ are each independently of the other hydrogen, C₁-C₄alkyl, cyclohexyl,
unsubstituted phenyl or benzyl, or phenyl or benzyl which are substituted by sulfo, chloro, methyl and/or methoxy.

3. A compound according to one of claims 1 and 2, wherein R and R₁ are each hydrogen.

4. A compound according to one of claims 1 to 3, wherein A is a C₂-C₄alkylene radical which is unsubstituted or substituted by hydroxy, sulfo, sulfato, methoxy, carboxy or sulfophenyl, -CH₂-CH₂-Z'-CH₂-CH₂-, wherein Z' is -O-, -S-, -SO₂-, -NH- or -N(CH₃)-, a cyclohexylene radical which is unsubstituted or substituted by 1 to 3 methyl groups, an unsubstituted or sulfo-substituted 1,3- or 1,4-phenylene radical, or a C₁-C₃alkylene-phenylene or C₁-C₂alkylene-phenylene-C₁-C₂alkylene radical, wherein the phenylene is in each case unsubstituted or substituted by methyl, methoxy, chloro or sulfo.

5. A compound according to one of claims 1 to 4, wherein A is C₂-C₄alkylene which is unsubstituted or substituted by hydroxy, sulfo, sulfato, methoxy, carboxy or sulfophenyl.

6. A compound according to one of claims 1 to 5, wherein A is 1,2-ethylene or 1,2- or 1,3-propylene which is unsubstituted or substituted by hydroxy or sulfato.

7. A compound according to one of claims 1 to 6, wherein X is the group -N(R₄)-, wherein R₄ is hydrogen, C₁-C₄alkyl, cyclohexyl, unsubstituted phenyl or benzyl, or phenyl or benzyl which are substituted by sulfo, chloro, methyl and/or methoxy.

8. A compound according to claim 7, wherein R₄ is hydrogen or C₁-C₄alkyl, especially hydrogen.

9. A compound according to one of claims 1 to 8, wherein m and n are each 0.

10. A compound according to one of claims 1 to 9, wherein p and q are each 1 and Z and Z₁ are each hydroxy or C₁-C₄alkyl, especially hydroxy.

11. A compound according to one of claims 1 to 10, wherein R₂ and R₃ are each hydrogen, fluoro, chloro, bromo, methyl, methoxy, acetylamino, phenoxy or cyano.

12. A compound according to one of claims 1 to 11, wherein R₂ and R₃ are each chloro.

13. A compound according to one of claims 1 to 12, wherein B is a linking group of the formula 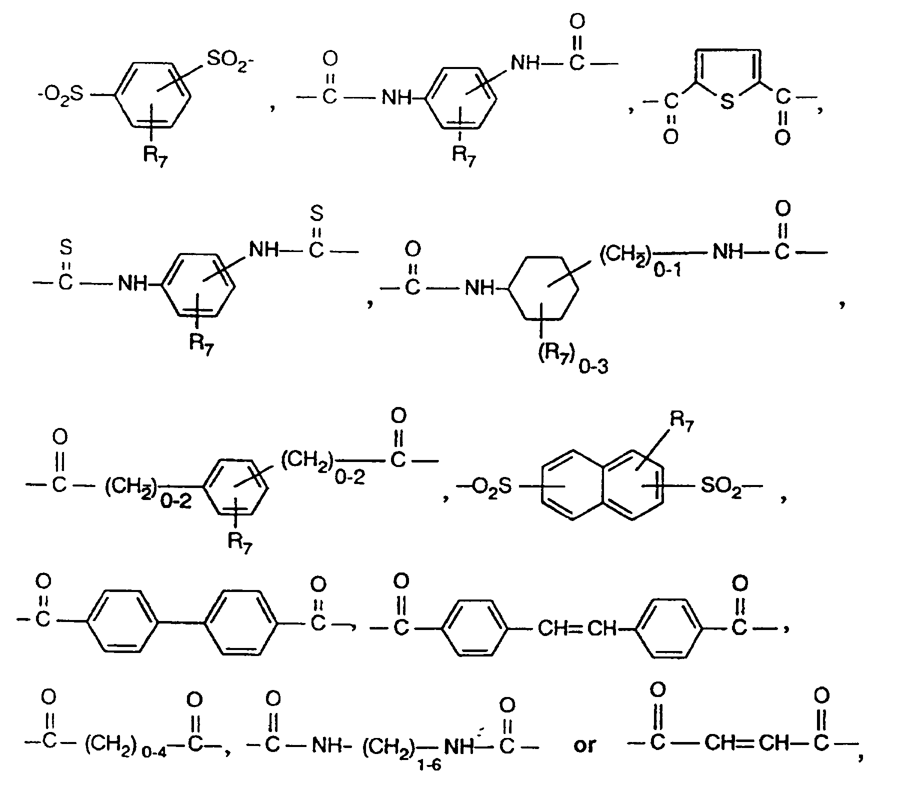 wherein R₇ is sulfo, methyl, methoxy, chloro, carboxy or, preferably, hydrogen.

14. A compound according to one of claims 1 to 13, wherein B is a radical of the formula

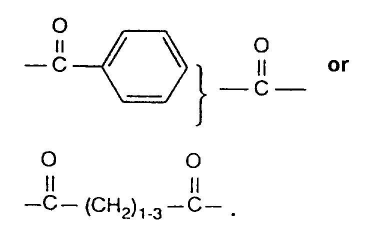

15. A compound according to claim 1 of formula (1), wherein R and R₁ are each independently of the other hydrogen, unsubstituted C₁-C₄alkyl or C₁-C₄alkyl which is substituted by hydroxy, sulfo, sulfato, chloro, cyano, or acetoxy and/or, with the exception of methyl, may be interrupted by a group -O-; unsubstituted cyclopentyl or cyclohexyl, or cyclopentyl or cyclohexyl which are substituted by 1 to 3 methyl groups;
unsubstituted phenyl or phenyl which is substituted by sulfo, nitro, chloro, methyl, methoxy, N-methylamino or N-ethylamino, N,N-dimethylamino or N,N-diethylamino, acetylamino, propionylamino, benzoylamino, methoxycarbonyl, ethoxycarbonyl, carboxy or methylsulfonyl; unsubstituted 1- or 2-naphthyl or 1- or 2-naphthyl which is substituted by sulfo, nitro and/or chloro; or is unsubstituted benzyl or benzyl which is substituted by methyl, methoxy, sulfo and/or chloro,
A is a C₂-C₄alkylene radical which is unsubstituted or substituted by hydroxy, sulfo, sulfato, methoxy, carboxy or sulfophenyl; -CH₂-CH₂-Z'-CH₂-CH₂-, wherein Z' is -O-, -S-, -SO₂-, -NH- or -N(CH₃)-; a cyclohexylene radical which is unsubstituted or substituted by 1 to 3 methyl groups;
an unsubstituted or sulfo-substituted 1,3- or 1,4-phenylene radical; or is a C₁-C₃alkylene-phenylene or C₁-C₂alkylene-phenylene-C₁-C₂alkylene radical, wherein the phenylene is in each case unsubstituted or substituted by methyl, methoxy, chloro or sulfo;
X and X₁ are each independently of the other a group -N(R₄)-, wherein R₄ is hydrogen, C₁-C₄alkyl, cyclohexyl, unsubstituted phenyl or benzyl or phenyl or benzyl which are substituted by sulfo, chloro, methyl and/or methoxy,
Y and Y₁ are each independently of the other methoxy, methyl, chloro or sulfo, n and m are each independently of the other 0 or 1, Z and Z₁ are each independently of the other hydroxy, methyl or ethyl, p and q are each 1, and R₂ and R₃ are each hydrogen, fluoro, chloro, bromo, methyl, methoxy, acetylamino, phenoxy or cyano.

16. A compound according to claim 1 of formula 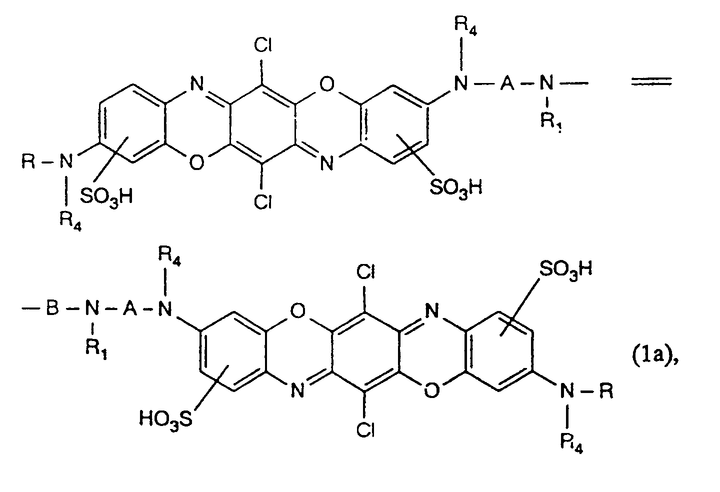
wherein R and R₁ are each independently of the other hydrogen, C₁-C₄alkyl, cyclohexyl, unsubstituted phenyl or benzyl, or phenyl or benzyl which are substituted by sulfo, chloro, methyl and/or methoxy,
A is a C₂-C₄alkylene radical which is unsubstituted or substituted by hydroxy, sulfo, sulfato, methoxy, carboxy or sulfophenyl, R₄ is hydrogen, methyl or ethyl, and B is a radical of formula 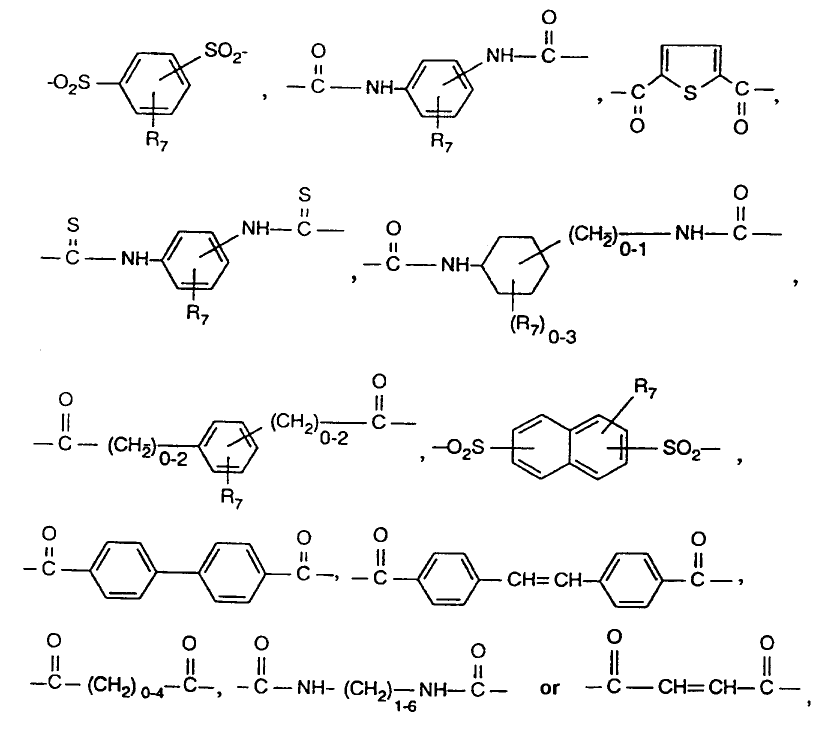
wherein R₇ is sulfo, methyl, methoxy, chloro, carboxy or, preferably, hydrogen.

17. A compound according to claim 1 of formula 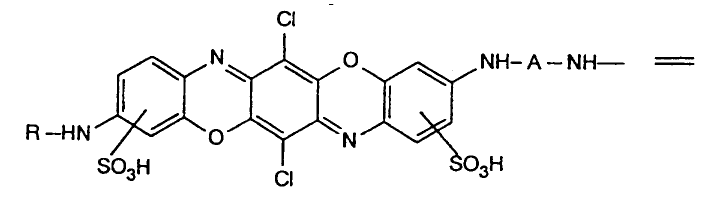 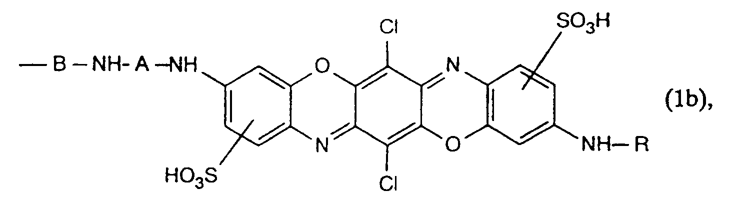
wherein R is methyl, ethyl, benzyl, or, in particular, hydrogen,
A is a 1,2-ethylene or 1,2- or 1,3-propylene radical which is unsubstituted or substituted by hydroxy or sulfato, and B is a radical of formula

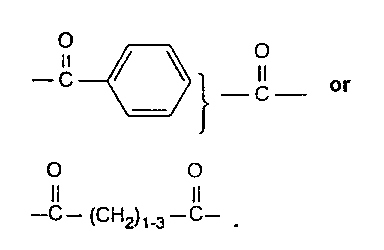

18. A process for the preparation of a compound of formula (1), which comprises condensing a compound of formula 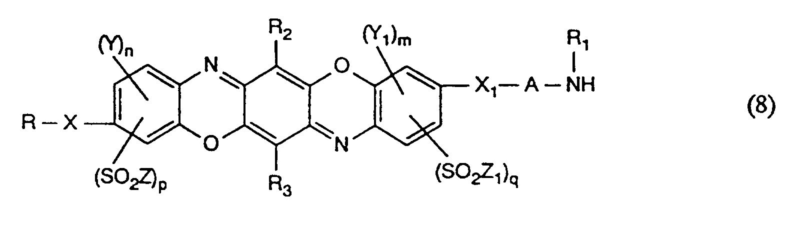 with a compound of formula
T-B-T (9)
wherein A, B, R, R₁, R₂, R₃, X, X₁, Y, Y₁, Z, Z₁, m, n, p and q are each as defined in claim 1 and T is halogen, preferably chloro.

19. A dye mixture obtainable by condensing a compound of formula (8) according to claim 18 and a compound of formula 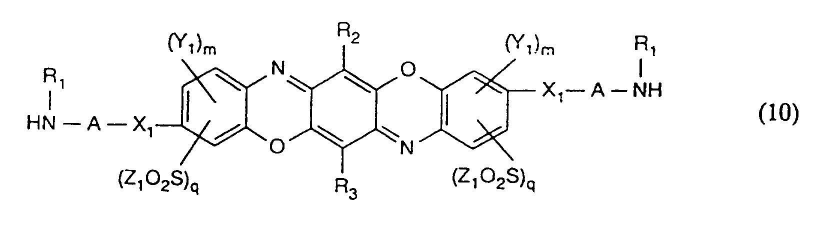 wherein A, R₁, R₂, R₃, X₁, Y₁, Z₁, m and q are each as defined in claim 1 with a compound of formula (9) according to claim 18.

20. A dye mixture according to claim 19, wherein the compounds of formulae (8) and (10) are used in the weight ratio of 90:10 to 60:40, preferably 80:20 to 65:35.

21. Use of a compound of formula (1) or of a mixture of compounds obtainable by reaction of compounds of formulae (8), (9) and (10) according to claim 19, as dye for dyeing or printing nitrogen-containing and, more particularly, cellulosic fibre materials.

22. Use according to claim 21, which comprises dyeing blends of synthetic fibres and cellulosic fibre materials, in particular polyester/cotton blends, in the presence of a disperse dye for the polyester fibres, under the dyeing conditions for polyester fibres.

23. A process for dyeing polyester/cotton blends with disperse and direct dyes, which comprises using a compound of formula (1) according to claim 1 or a mixture of compounds obtainable according to claim 19 as dye, in a one-step, single-bath process and in addition to the disperse dyes, and dyeing from an aqueous liquor at temperatures in the range from 100 to 150°C, preferably from 120 to 130°C, and at a pH of between 4 and 7.5.

## Claims (Claims for the following Contracting State(s): ES)

1. A process for the preparation of a compound of formula 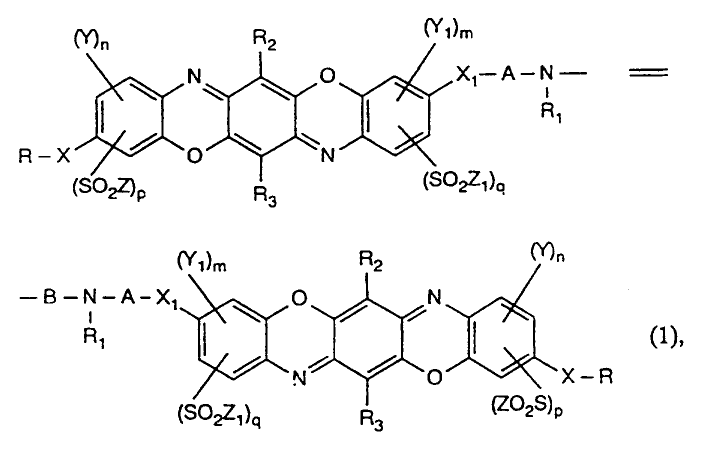
wherein R and R₁ are each independently of the other hydrogen; unsubstituted C₁-C₄alkyl or C₁-C₄alkyl which is substituted by hydroxy, sulfo, sulfato, chloro, cyano or acetoxy and/or, with the exception of methyl, may be interrupted by a group -O-; cyclopentyl or cyclohexyl which are unsubstituted or substituted by 1 to 3 methyl groups;
unsubstituted phenyl or phenyl which is substituted by sulfo, nitro, chloro, methyl, methoxy, N-methylamino or N-ethylamino, N,N-dimethyl-amino or N,N-diethylamino, acetylamino, propionylamino, benzoylamino, methoxycarbonyl, ethoxycarbonyl, carboxy or methylsulfonyl;
unsubstituted 1- or 2-naphthyl or 1- or 2-naphthyl which is substituted by sulfo, nitro and/or chloro; or unsubstituted benzyl or benzyl which is substituted by methyl, methoxy, sulfo and/or chloro,
X is a direct bond, -O-, -S- or -N(R₄)-, wherein R₄ is as defined above for R and R₁,
X₁ is -O-, -S- or -N(R₄)-, wherein R₄ is as defined above for R and R₁,
Y and Y₁ are each independently of the other C₁-C₄alkyl, C₁-C₄alkoxy, halogen, sulfo, carboxy, carbamoyl, N-mono- or N,N-di-C₁-C₄alkylcarbamoyl, N-phenyl- or N,N-diphenylcarbamoyl, sulfamoyl, N-mono- or N,N-di-C₁-C₄alkylsulfamoyl or N-phenyl- or N,N-diphenylsulfamoyl,
Z and Z₁ are each independently of the other hydroxy or C₁-C₄alkyl,
R₂ and R₃ are each independently of the other hydrogen, halogen, cyano, C₁-C₄alkyl, C₁-C₄alkoxy, unsubstituted or C₁-C₄alkyl-, C₁-C₄alkoxy, acetylamino-, halogen-, nitro- or sulfo-substituted phenyl, benzyl, benzoylamino or phenoxy, sulfo, carboxy, carbamoyl, phenylcarbamoyl or C₂-C₅alkanoylamino,
A is C₂-C₆alkylene which is unsubstituted or substituted by hydroxy, sulfo, sulfato, C₁-C₄alkoxy, carboxy, cyano, halogen, phenyl, sulfophenyl or C₂-C₅alkoxycarbonyl and is uninterrupted or interrupted by 1-2 groups -O-, -N(R₅)- wherein R₅ is C₁-C₄alkyl, acetyl or hydrogen, -S- or -SO₂-; or is cyclohexylene or cyclopentylene which are unsubstituted or substituted one or more times by C₁-C₃alkyl; or is
naphthylene, biphenylene or phenylene which are unsubstituted or substituted by C₁-C₄alkyl, C₁-C₄alkoxy, sulfo, halogen or carboxy; or is C₁-C₆alkylene-phenylene, phenylene-C₁-C₆alkylene-phenylene, C₁-C₃alkylene-phenylene-C₁-C₃alkylene or methylene-naphthylene-methylene, in which aralkylene radicals the phenylene or naphthylene contains no further substituents or contains 1 or 2 substituents selected from the group consisting of sulfo, carboxy, sulfamoyl, carbamoyl, methyl, ethyl, methoxy, ethoxy, nitro, chloro, amino, N-methylamino and N-ethylamino, N,N-dimethylamino and N,N-diethylamino and phenylamino;
B is a radical of the formula
Q-E-Q (2'),
wherein Q is a group of the formula, 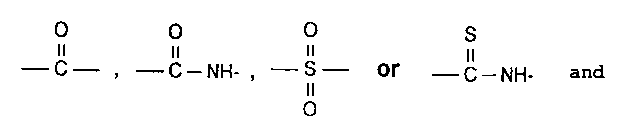
E is a direct bond; C₁-C₆alkylene or C₂-C₆alkenylene which are unsubstituted or substituted by hydroxy, sulfo, sulfato, methoxy, carboxy, phenyl or sulfophenyl; cyclohexylene or C₁-C₂alkylene-cyclohexylene which are unsubstituted or substituted by 1 to 3 methyl groups, piperazine-1,4-diyl; thiophene-2,5-diyl; biphenyl-4,4'-diyl; stilbene-4,4'-diyl; unsubstituted phenylene or naphthylene, or phenylene or naphthylene which are substitued by C₁-C₄-alkyl, C₁-C₄alkoxy, sulfo, halogen or carboxy; or is C₁-C₃alkylene-phenylene or C₁-C₂alkylene-phenylene-C₁-C₂alkylene which are unsubstitued or subsituted in the phenyl moiety by methyl, methoxy, chloro or sulfo; and m, n, p and q are each independently of one another 0 or 1 which comprises condensing a compound of formula 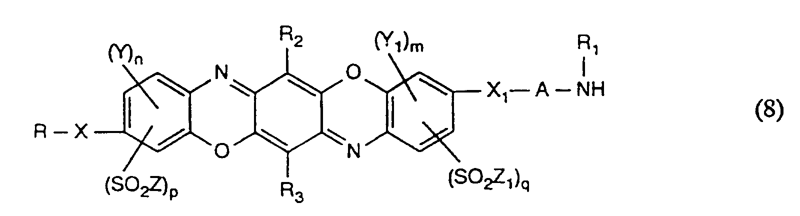 with a compound of formula
T-B-T (9)
wherein A, B, R, R₁, R₂, R₃, X, X₁, Y, Y₁, Z, Z₁, m, n, p and q are each as defined under formula 1 and T is halogen, preferably chloro.

2. A process according to claim 1, wherein R and R₁ are each independently of the other hydrogen, C₁-C₄alkyl, cyclohexyl, unsubstituted phenyl or benzyl, or phenyl or benzyl which are substituted by sulfo, chloro, methyl and/or methoxy.

3. A process according to one of claims 1 and 2, wherein R and R₁ are each hydrogen.

4. A compound according to one of claims 1 to 3, wherein a compound of the formula (8) is used in which A is a C₂-C₄alkylene radical which is unsubstituted or substituted by hydroxy, sulfo, sulfato, methoxy, carboxy or sulfophenyl, -CH₂-CH₂-Z'-CH₂-CH₂-, wherein Z' is -O-, -S-, -SO₂-, -NH- or -N(CH₃)-, a cyclohexylene radical which is unsubstituted or substituted by 1 to 3 methyl groups, an unsubstituted or sulfo-substituted 1,3- or 1,4-phenylene radical, or a C₁-C₃alkylene-phenylene or C₁-C₂alkylene-phenylene-C₁-C₂alkylene radical, wherein the phenylene is in each case unsubstituted or substituted by methyl, methoxy, chloro or sulfo.

5. A process according to one of claims 1 to 4, wherein a compound of the formula (8) is used in which A is C₂-C₄alkylene which is unsubstituted or substituted by hydroxy, sulfo, sulfato, methoxy, carboxy or sulfophenyl.

6. A process according to one of claims 1 to 5, wherein a compound of the formula (8) is used in which A is 1,2-ethylene or 1,2- or 1,3-propylene which is unsubstituted or substituted by hydroxy or sulfato.

7. A process according to one of claims 1 to 6, wherein a compound of the formula (8) is used in which X is the group -N(R₄)-, wherein R₄ is hydrogen, C₁-C₄alkyl, cyclohexyl, unsubstituted phenyl or benzyl, or phenyl or benzyl which are substituted by sulfo, chloro, methyl and/or methoxy.

8. A process according to claim 7, wherein a compound of the formula (8) is used in which R₄ is hydrogen or C₁-C₄alkyl, especially hydrogen.

9. A process according to one of claims 1 to 8, wherein a compound of the formula (8) is used in which m and n are each 0.

10. A process according to one of claims 1 to 9, wherein a compound of the formula (8) is used in which p and q are each 1 and Z and Z₁ are each hydroxy or C₁-C₄alkyl, especially hydroxy.

11. A process according to one of claims 1 to 10, wherein a compound of the formula (8) is used in which R₂ and R₃ are each hydrogen, fluoro, chloro, bromo, methyl, methoxy, acetylamino, phenoxy or cyano.

12. A process according to one of claims 1 to 11, wherein a compound of the formula (8) is used in which R₂ and R₃ are each chloro.

13. A process according to one of claims 1 to 12, wherein B is a linking group of the formula 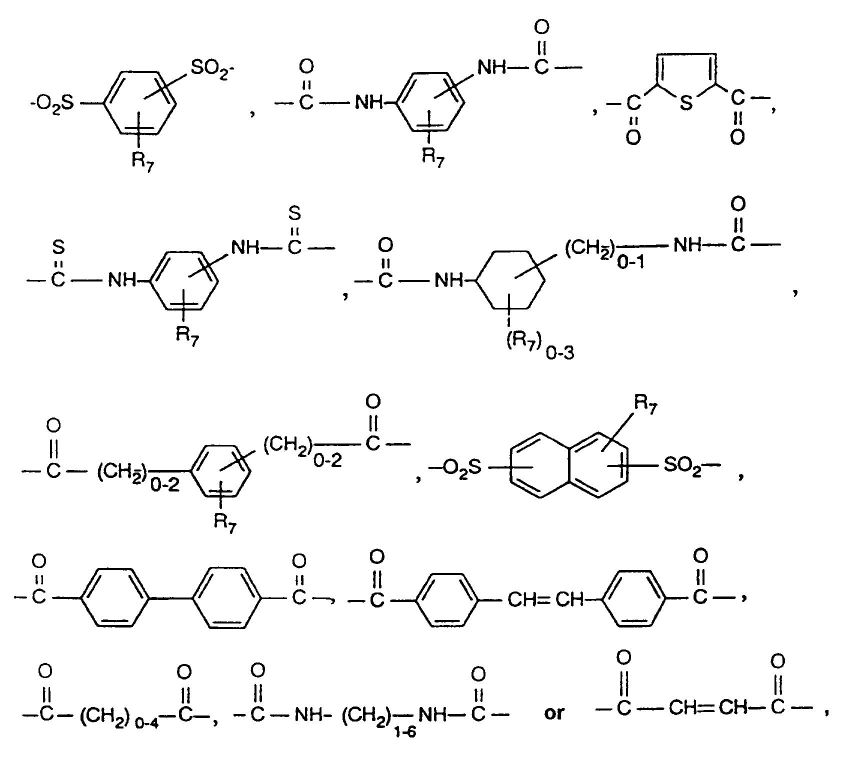 wherein R₇ is sulfo, methyl, methoxy, chloro, carboxy or, preferably, hydrogen.

14. A process according to one of claims 1 to 13, wherein a compound of the formula (9) is used in which B is a radical of the formula

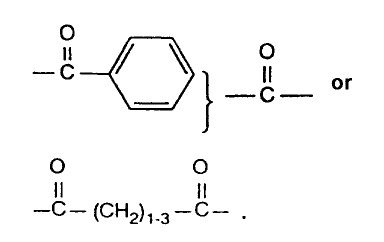

15. A process according to claim 1, wherein a compound of the formula (8) is used in which R and R₁ are each independently of the other hydrogen, unsubstituted C₁-C₄alkyl or C₁-C₄alkyl which is substituted by hydroxy, sulfo, sulfato, chloro, cyano, or acetoxy and/or, with the exception of methyl, may be interrupted by a group -O-; unsubstituted cyclopentyl or cyclohexyl, or cyclopentyl or cyclohexyl which are substituted by 1 to 3 methyl groups; unsubstituted phenyl or phenyl which is substituted by sulfo, nitro, chloro, methyl, methoxy, N-methylamino or N-ethylamino, N,N-dimethylamino or N,N-diethylamino, acetylamino, propionylamino, benzoylamino, methoxycarbonyl, ethoxycarbonyl, carboxy or methylsulfonyl; unsubstituted 1- or 2-naphthyl or 1- or 2-naphthyl which is substituted by sulfo, nitro and/or chloro; or is unsubstituted benzyl or benzyl which is substituted by methyl, methoxy, sulfo and/or chloro,
A is a C₂-C₄alkylene radical which is unsubstituted or substituted by hydroxy, sulfo, sulfato, methoxy, carboxy or sulfophenyl; -CH₂-CH₂-Z'-CH₂-CH₂-, wherein Z' is -O-, -S-, -SO₂-, -NH- or -N(CH₃)-; a cyclohexylene radical which is unsubstituted or substituted by 1 to 3 methyl groups; an unsubstituted or sulfo-substituted 1,3- or 1,4-phenylene radical; or is a C₁-C₃alkylene-phenylene or C₁-C₂alkylene-phenylene-C₁-C₂alkylene radical, wherein the phenylene is in each case unsubstituted or substituted by methyl, methoxy, chloro or sulfo;
X and X₁ are each independently of the other a group -N(R₄)-, wherein R₄ is hydrogen, C₁-C₄alkyl, cyclohexyl, unsubstituted phenyl or benzyl or phenyl or benzyl which are substituted by sulfo, chloro, methyl and/or methoxy,
Y and Y₁ are each independently of the other methoxy, methyl, chloro or sulfo, n and m are each independently of the other 0 or 1, Z and Z₁ are each independently of the other hydroxy, methyl or ethyl, p and q are each 1, and R₂ and R₃ are each hydrogen, fluoro, chloro, bromo, methyl, methoxy, acetylamino, phenoxy or cyano.

16. A process according to claim 1, wherein a compound of formula 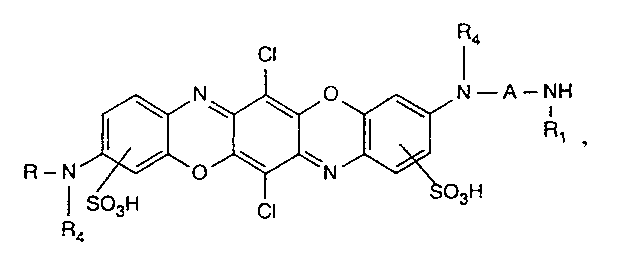
wherein R and R₁ are each independently of the other hydrogen, C₁-C₄alkyl, cyclohexyl, unsubstituted phenyl or benzyl, or phenyl or benzyl which are substituted by sulfo, chloro, methyl and/or methoxy,
A is a C₂-C₄alkylene radical which is unsubstituted or substituted by hydroxy, sulfo, sulfato, methoxy, carboxy or sulfophenyl, and R₄ is hydrogen, methyl or ethyl, is condensed with a compound of the formula (9) in which B is a radical of formula 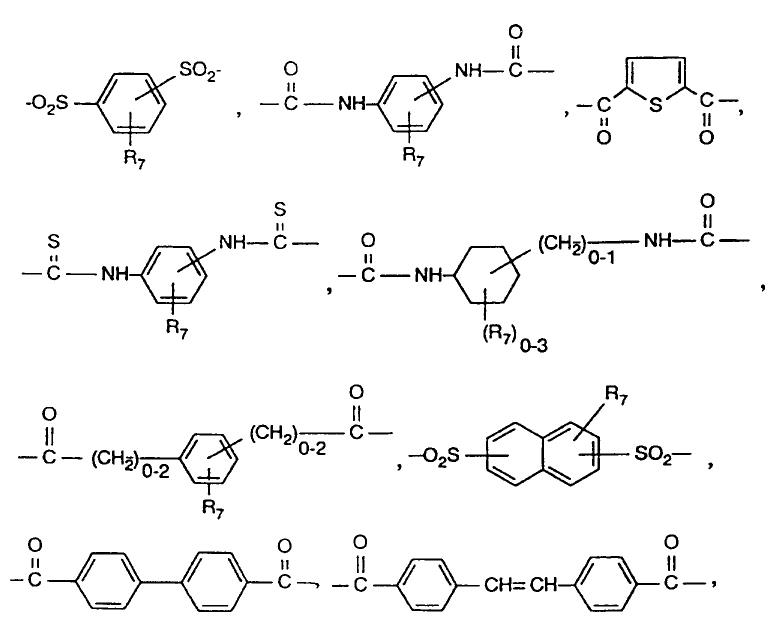 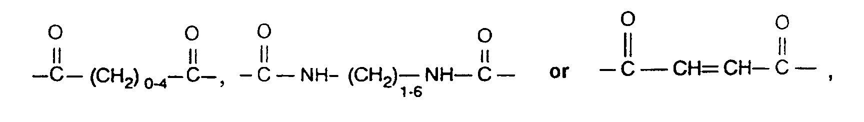 wherein R₇ is sulfo, methyl, methoxy, chloro, carboxy or, preferably, hydrogen.

17. A process according to claim 1, wherein a compound of the formula 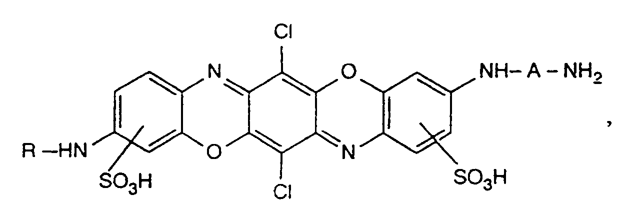 wherein R is methyl, ethyl, benzyl, or, in particular, hydrogen, and A is a 1,2-ethylene or 1,2- or 1,3-propylene radical which is unsubstituted or substituted by hydroxy or sulfato, is condensed with a compound of the formula (9) in which B is a radical of formula

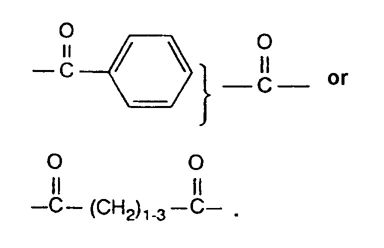

18. A process for preparing a dye mixture, which comprises condensing a compound of formula (8) according to claim 21 and a compound of formula 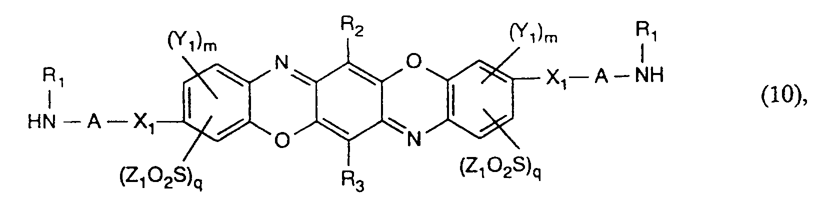 wherein A, R₁, R₂, R₃, X₁, Y₁, Z₁, m and q are each as defined in claim 1, with a compound of formula (9) according to claim 1.

19. A process for preparing a dye mixture according to claim 18, wherein the compounds of formulae (8) and (10) are used in the weight ratio of 90:10 to 60:40, preferably 80:20 to 65:35.

20. A compound of the formula 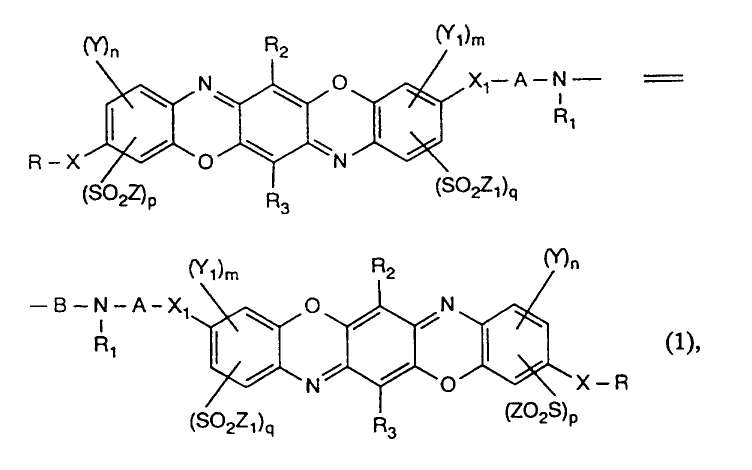
wherein R and R₁ are each independently of the other hydrogen; unsubstituted C₁-C₄alkyl or C₁-C₄alkyl which is substituted by hydroxy, sulfo, sulfato, chloro, cyano or acetoxy and/or, with the exception of methyl, may be interrupted by a group -O-; cyclopentyl or cyclohexyl which are unsubstituted or substituted by 1 to 3 methyl groups; unsubstituted phenyl or phenyl which is substituted by sulfo, nitro, chloro, methyl, methoxy, N-methylamino or N-ethylamino, N,N-dimethyl-amino or N,N-diethylamino, acetylamino, propionylamino, benzoylamino, methoxycarbonyl, ethoxycarbonyl, carboxy or methylsulfonyl;
unsubstituted 1- or 2-naphthyl or 1- or 2-naphthyl which is substituted by sulfo, nitro and/or chloro; or unsubstituted benzyl or benzyl which is substituted by methyl, methoxy, sulfo and/or chloro, X is a direct bond, -O-, -S- or -N(R₄)-, wherein R₄ is as defined above for R and R₁,
X₁ is -O-, -S- or -N(R₄)-, wherein R₄ is as defined above for R and R₁,
Y and Y₁ are each independently of the other C₁-C₄alkyl, C₁-C₄alkoxy, halogen, sulfo, carboxy, carbamoyl, N-mono- or N,N-di-C₁-C₄alkylcarbamoyl, N-phenyl- or N,N-diphenylcarbamoyl, sulfamoyl, N-mono- or N,N-di-C₁-C₄alkylsulfamoyl or N-phenyl- or N,N-diphenylsulfamoyl,
Z and Z₁ are each independently of the other hydroxy or C₁-C₄alkyl,
R₂ and R₃ are each independently of the other hydrogen, halogen, cyano, C₁-C₄alkyl, C₁-C₄alkoxy, unsubstituted or C₁-C₄alkyl-, C₁-C₄alkoxy, acetylamino-, halogen-, nitro- or sulfo-substituted phenyl, benzyl, benzoylamino or phenoxy, sulfo, carboxy, carbamoyl, phenylcarbamoyl or C₂-C₅alkanoylamino,
A is C₂-C₆alkylene which is unsubstituted or substituted by hydroxy, sulfo, sulfato, C₁-C₄alkoxy, carboxy, cyano, halogen, phenyl, sulfophenyl or C₂-C₅alkoxycarbonyl and is uninterrupted or interrupted by 1-2 groups -O-, -N(R₅)- wherein R₅ is C₁-C₄alkyl, acetyl or hydrogen, -S- or -SO₂-; or is cyclohexylene or cylcopentylene which are unsubstituted or substituted one or more times by C₁-C₃alkyl; or is
naphthylene, biphenylene or phenylene which are unsubstituted or substituted by C₁-C₄alkyl, C₁-C₄alkoxy, sulfo, halogen or carboxy; or is C₁-C₆alkylene-phenylene, phenylene-C₁-C₆alkylene-phenylene, C₁-C₃alkylene-phenylene-C₁-C₃alkylene or methylene-naphthylene-methylene, in which aralkylene radicals the phenylene or naphthylene contains no further substituents or contains 1 or 2 substituents selected from the group consisting of sulfo, carboxy, sulfamoyl, carbamoyl, methyl, ethyl, methoxy, ethoxy, nitro, chloro, amino, N-methylamino and N-ethylamino, N,N-dimethylamino and N,N-diethylamino and phenylamino;
B is a radical of the formula
Q-E-Q (2'),
wherein Q is a group of the formula, 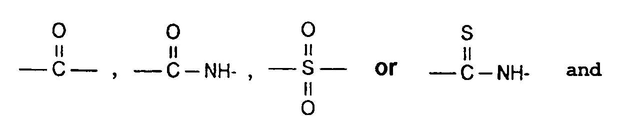
E is a direct bond; C₁-C₆alkylene or C₂-C₆alkenylene which are unsubstituted or substituted by hydroxy, sulfo, sulfato, methoxy, carboxy, phenyl or sulfophenyl; cyclohexylene or C₁-C₂alkylene-cyclohexylene which are unsubstituted or substituted by 1 to 3 methyl groups, piperazine-1,4-diyl; thiophene-2,5-diyl; biphenyl-4,4'-diyl; stilbene-4,4'-diyl; unsubstituted phenylene or naphthylene, or phenylene or naphthylene which are substitued by C₁-C₄-alkyl, C₁-C₄alkoxy, sulfo, halogen or carboxy; or is C₁-C₃alkylene-phenylene or C₁-C₂alkylene-phenylene-C₁-C₂alkylene which are unsubstitued or subsituted in the phenyl moiety by methyl, methoxy, chloro or sulfo; and
m, n, p and q are each independently of one another 0 or 1.

21. A dye mixture obtainable by condensing a compound of the formula (8) according to claim 1 and a compound of the formula 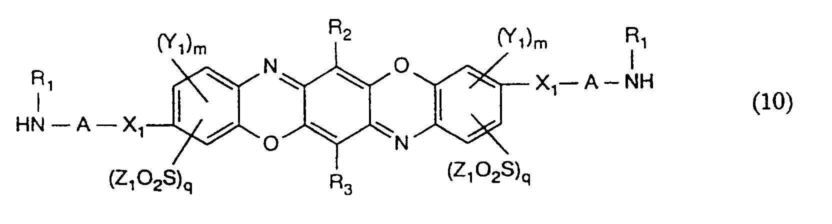 wherein A, R₁, R₂, R₃, X₁, Y₁, Z₁, m and q are each as defined in claim 1 with a compound of the formula (9) according to claim 1.

22. Use of a compound of formula (1) obtainable according to claim 1 or of a mixture of compounds obtainable by reaction of compounds of formulae (8), (9) and (10) according to claim 18 as dye for dyeing or printing nitrogen-containing and, more particularly, cellulosic fibre materials.

23. Use according to claim 22, which comprises dyeing blends of synthetic fibres and cellulosic fibre materials, in particular polyester/cotton blends, in the presence of a disperse dye for the polyester fibres, under the dyeing conditions for polyester fibres.

24. A process for dyeing polyester/cotton blends with disperse and direct dyes, which comprises using a compound of formula (1) obtainable according to claim 1 or a mixture of compounds obtainable according to claim 18 as dye, in a one-step, single-bath process and in addition to the disperse dyes, and dyeing from an aqueous liquor at temperatures in the range from 100 to 150°C, preferably from 120 to 130°C, and at a pH of between 4 and 7.5.

## Revendications (Revendications pour l'(les) Etat(s) contractant(s) suivant(s): BE, CH, DE, FR, GB, IT, LI)

1. Composés de formule 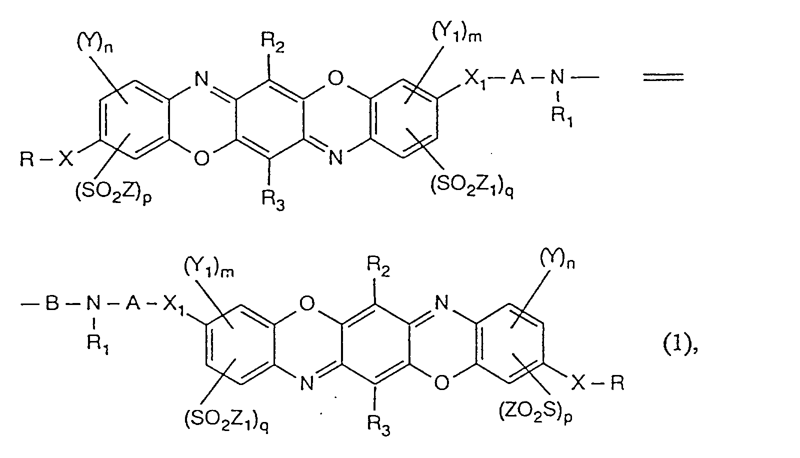 dans laquelle
R et R₁ représentent, indépendamment l'un de l'autre, un atome d'hydrogène; un résidu alkyle en C₁₋₄ non substitué ou portant un substituant hydroxy, sulfo, sulfato, chloro, cyano ou acétoxy et/ou, à l'exception du groupe méthyle, pouvant être interrompu par un -O-; un résidu cyclopentyle ou cyclohexyle non substitué ou portant de 1 à 3 substituants méthyle; un résidu phényle non substitué ou portant un ou plusieurs substituants sulfo, nitro, chloro, méthyle, méthoxy, N-méthyl-ou N-éthylamino, N,N-diméthyl- ou N,N-diéthylamino, acétylamino, propionylamino, benzoylamino, méthoxycarbonyle, éthoxycarbonyle, carboxy ou méthylsulfonyle; un résidu 1-naphtyle ou 2-naphtyle non substitué ou portant un ou plusieurs résidus sulfo, nitro et/ou chloro; ou un résidu benzyle non substitué ou portant un ou plusieurs substituants méthyle, méthoxy, sulfo et/ou chloro;
X représente une liaison directe, un groupe -O-, -S- ou -N(R₄)-où R₄ a la signification indiquée ci-dessus pour R et R₁,
X₁ représente un groupe -O-, -S- ou -N(R₄)- où R₄ a la signification indiquée ci-dessus pour R et R₁,
Y et Y₁ représentent, indépendamment l'un de l'autre, chacun un groupe alkyle en C₁₋₄, alcoxy en C₁₋₄, halogéno, sulfo, carboxy, carbamoyle, N-mono- ou N,N-di(alkyle en C₁₋₄)-carbamoyle, N-phényl-ou N,N-diphénylcarbamoyle, sulfamoyle, N-mono- ou N,N-di(alkyle en C₁₋₄)-sulfamoyle ou N-phényl- ou N,N-diphénylsulfamoyle,
Z et Z₁ représentent, indépendamment l'un de l'autre, chacun un résidu hydroxy ou alkyle en C₁₋₄,
R₂ et R₃ représentent, indépendamment l'un de l'autre, un atome d'hydrogène ou d'halogène, un groupe cyano, alkyle en C₁₋₄, alcoxy en C₁₋₄, un groupe phényle, benzyle, benzoylamino ou phénoxy éventuellement substitués par un ou des groupes alkyle en C₁₋₄, alcoxy en C₁₋₄, acétylamino, halogéno, nitro ou sulfo; un groupe sulfo, carboxy, carbamoyle, phénylcarbamoyle ou alcanoylamino en C₂₋₅,
A représente un groupe alkylène en C₂₋₆ non substitué ou portant un ou des résidus hydroxy, sulfo, sulfato, alcoxy en C₁₋₄, carboxy, cyano, halogéno, phényle, sulfophényle ou alcoxycarbonyle en C₂₋₅ et éventuellement interrompu par 1 ou 2 groupes -O-, -S-, -SO₂- ou -N(R₅)- où R₅ représente un groupe alkyle en C₁₋₄, acétyle ou un atome d'hydrogène, ou un groupe cyclopentylène ou cyclohexylène non substitué ou portant un ou plusieurs substituants alkyle en C₁₋₃; ou un résidu phénylène, biphénylène ou naphtylène non substitué ou portant un ou des substituants alkyle en C₁₋₄, alcoxy en C₁₋₄, sulfo, halogéno ou carboxy; ou un groupe (alkylène en C₁₋₆)-phénylène, phénylène-(alkylène en C₁₋₆)-phénylène, (alkylène en C₁₋₃)-phénylène-(alkylène en C₁₋₃) ou méthylène-naphtylène-méthylène, les résidus phénylène ou naphtylène ne portant pas d'autres substituants ou 1 ou 2 substituants choisis parmi les résidus sulfo, carboxy, sulfamoyle, carbamoyle, méthyle, éthyle, méthoxy, éthoxy, nitro, chloro, amino, N-méthyl- et N-éthylamino, N,N-diméthyl-et N,N-diéthylamino et phénylamino,
B représente un résidu de formule
Q-E-Q (2')
dans laquelle Q représente un groupe de formule 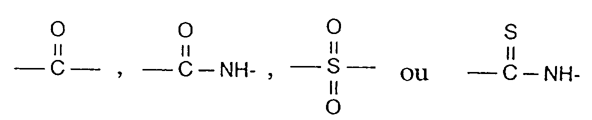 et E représente une liaison directe; un résidu alkylène en C₁₋₆ ou alcénylène en C₂₋₆ non substitué ou portant un ou plusieurs résidus hydroxy, sulfo, sulfato, méthoxy, carboxy, phényle ou sulfophényle; un résidu cyclohexylène ou (alkylène en C₁₋₂)-cyclohexylène non substitué ou portant de 1 à 3 groupes méthyle; un résidu pipérazin-1,4-diyle; thiophène-2,5-diyle; biphényl-4,4-diyle; stilbène-4,4'-diyle; phénylène ou naphtylène non substitué ou portant des substituants alkyle en C₁₋₄, alcoxy en C₁₋₄, sulfo, halogéno ou carboxy; ou un résidu (alkylène en C₁₋₃)-phénylène ou (alkylène en C₁₋₂)-phénylène-(alkylène en C₁₋₂) non substitués ou portant sur la partie phényle des substituants méthyle, méthoxy, chloro ou sulfo, et m, n, p et q valent indépendamment l'un de l'autre chacun 0 ou 1.

2. Composés conformes à la revendication 1, caractérisés en ce que R et R₁ représentent indépendamment l'un de l'autre chacun un atome d'hydrogène, un groupe alkyle en C₁₋₄, cyclohexyle, phényle ou benzyle non substitué ou portant un ou plusieurs substituants sulfo, chloro, méthyle, et/ou méthoxy.

3. Composés conformes à une des revendications 1 et 2, caractérisés en ce que R et R₁ représentent chacun un atome d'hydrogène.

4. Composés conformes à une de revendications 1 à 3, caractérisés en ce que A représente un résidu alkylène en C₂₋₄ non substitué ou portant un ou plusieurs résidus hydroxy, sulfo, sulfato, méthoxy, carboxy ou sulfophényle, un groupe -CH₂-CH₂-Z'-CH₂-CH₂- où Z' représente un groupe -O-, -S-, -SO₂-, -NH- ou -N(CH₃)-, un résidu cyclohexylène non substitué ou portant de 1 à 3 résidus méthyle, un résidu 1,3-phénylène ou 1,4-phénylène non substitué ou portant un résidu sulfo, ou un résidu (alkylène en C₁₋₃)-phénylène ou (alkylène en C₁₋₂)-phénylène-(alkylène en C₁₋₂) à groupe phénylène non substitué ou portant un ou plusieurs résidus méthyle, méthoxy, chloro ou sulfo.

5. Composés conformes à une des revendications 1 à 4 caractérisés en ce que A représente un groupe alkylène en C₂₋₄ non substitué ou portant un ou plusieurs substituants hydroxy, sulfo, sulfato, méthoxy, carboxy ou sulfophényle.

6. Composés conformes à une des revendications 1 à 5, caractérisés en ce que A représente un résidu 1,2-éthylène ou 1,2- ou 1,3-propylène non substitué ou portant un substituant hydroxy ou sulfato.

7. Composés conformes à une des revendications 1 à 6 caractérisés en ce que X représente un groupe -N(R₄)- où R₄ représente un atome d'hydrogène, un groupe alkyle en C₁₋₄, cyclohexyle, phényle ou benzyle non substitué ou portant un ou plusieurs substituants sulfo, chloro, méthyle et/ou méthoxy.

8. Composés conformes à la revendication 7, caractérisés en ce que R₄ représente un atome d'hydrogène ou un groupe alkyle en C₁₋₄, en particulier un atome d'hydrogène.

9. Composés conformes à une des revendications 1 à 8 caractérisés en ce que m et n représentent tous deux le nombre 0.

10. Composés conformes à une des revendications 1 à 9 caractérisés en ce que p et q sont tous deux égaux à 1 et Z et Z₁ représentent chacun un groupe hydroxy ou alkyle en C₁₋₄, en particulier un groupe hydroxy.

11. Composés conformes à une des revendications 1 à 10, caractérisés en ce que R₂ et R₃ représentent chacun un atome d'hydrogène, de fluor, de chlore ou de brome, un groupe méthyle, méthoxy, acétylamino, phénoxy ou cyano.

12. Composés conformes à une des revendications 1 à 11, caractérisés en ce que R₂ et R₃ représentent chacun un atome de chlore.

13. Composés conformes à une des revendications 1 à 12, caractérisés en ce que B est un élément pontant de formule 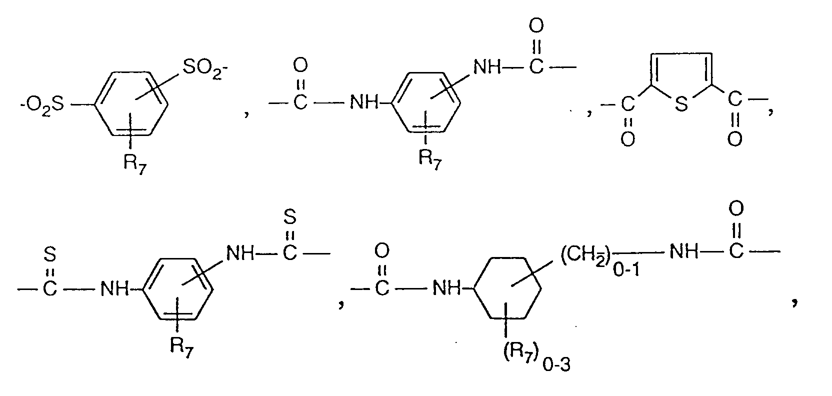 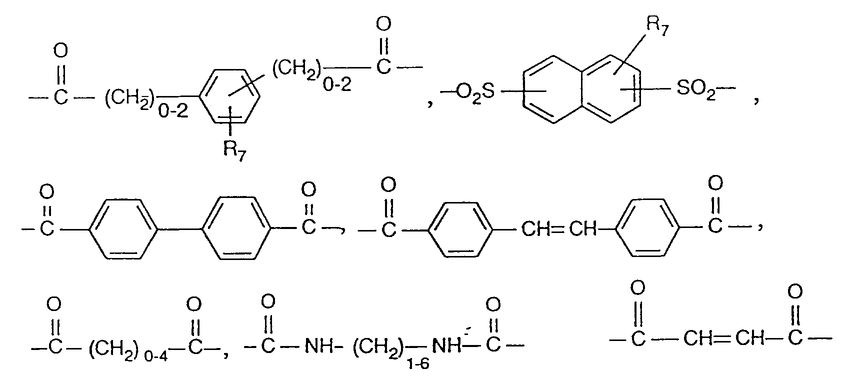 où R₇ représente un groupe sulfo, méthyle, méthoxy, chloro, carboxy ou, de préférence, un atome d'hydrogène.

14. Composés conforme à une des revendications 1 à 3, caractérisés en ce que B représente un résidu de formule

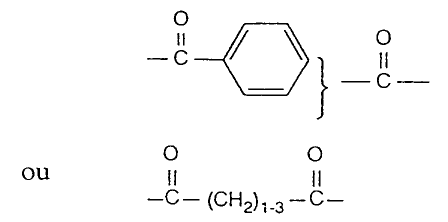

15. Composés de formule (1) conformes à la revendication 1, dans lesquels R et R₁ représentent indépendamment l'un de l'autre un atome d'hydrogène; un groupe alkyle en C₁₋₄ non substitué ou portant un ou plusieurs résidus hydroxy, sulfo, sulfato, chloro, cyano ou acétoxy et/ou, à l'exception du groupe méthyle, pouvant être interrompu par un groupe -O-; un résidu cyclopentyle ou cyclohexyle non substitué ou portant de 1 à 3 substituants méthyle; un résidu phényle non substitué ou portant un ou plusieurs substituants sulfo, nitro, chloro, méthyle, méthoxy, N-méthyl- ou N-éthylamino, N,N-diméthyl- ou N,N-diéthylamino, acétylamino, propionylamino, benzoylamino, méthoxycarbonyle, éthoxycarbonyle, carboxy ou méthylsulfonyle; un groupe 1-naphtyle ou 2-naphtyle non substitué ou portant des groupes sulfo, nitro et/ou chloro; ou encore un groupe benzyle non substitué ou portant des substituants méthyle, méthoxy, sulfo et/ou chloro;
A représente un résidu alkylène en C₂₋₄ non substitue ou portant un résidu hydroxy, sulfo, sulfato, méthoxy, carboxy ou sulfophényle, un groupe -CH₂-CH₂-Z'-CH₂-CH₂- où Z' représente un groupe -O-, -S-, -SO₂-, -NH- ou -N(CH₃)-, un résidu cyclohexylène non substitué ou portant de 1 à 3 résidus méthyle, un résidu 1,3-phénylène ou 1,4-phénylène non substitué ou portant un résidu sulfo, ou un résidu (alkylène en C₁₋₃)-phénylène ou (alkylène en C₁₋₂)-phénylène-(alkylène en C₁₋₂) à groupe phénylène non substitué ou portant un ou plusieurs résidus méthyle, méthoxy, chloro ou sulfo;
X et X₁ représentent, indépendamment l'un de l'autre, un groupe -N(R₄)- où R₄ représente un atome d'hydrogène, un groupe alkyle en C₁₋₄, cyclohexyle, phényle ou benzyle non substitués ou portant des résidus sulfo, chloro, méthyle et/ou méthoxy,
Y et Y₁ représentent indépendamment l'un de l'autre chacun un groupe méthoxy, méthyle, sulfo ou chloro;
n et m représentent de préférence le nombre 0 ou 1;
Z et Z₁ représentent, indépendamment l'un de l'autre, un groupe hydroxy, méthyle ou éthyle,
p et q sont tous deux égaux à 1;
R₂ et R₃ représentent chacun un atome d'hydrogène, de fluor, de chlore, de brome, un groupe méthyle, méthoxy, acétylamino, phénoxy ou cyano.

16. Composés conformes à la revendication 1 de formule 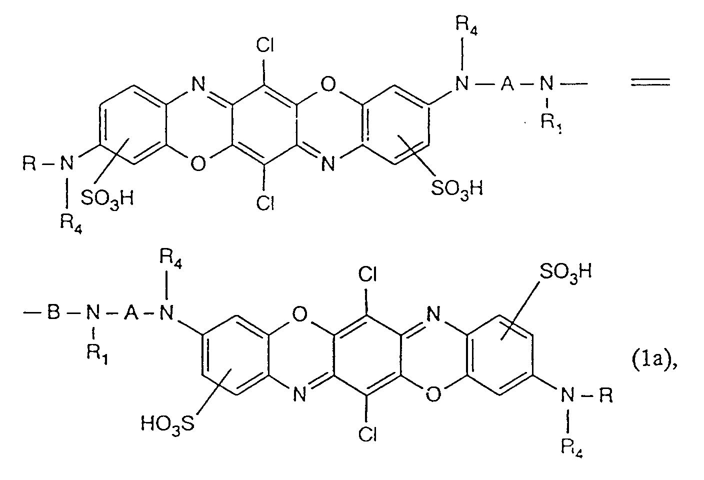 dans lesquelles R et R₁ représentent, indépendamment l'un de l'autre, un atome d'hydrogène, un groupe alkyle en C₁₋₄, cyclohexyle, phényle ou benzyle non substitués ou portant des substituants sulfo, chloro, méthyle et/ou méthoxy,
A représente un résidu alkylène en C₂₋₄ non substitué ou portant un ou plusieurs substituants hydroxy, sulfo, sulfato, méthoxy, carboxy ou sulfophényle, R₄ représente un atome d'hydrogène, un groupe méthyle ou éthyle et B un résidu de formule 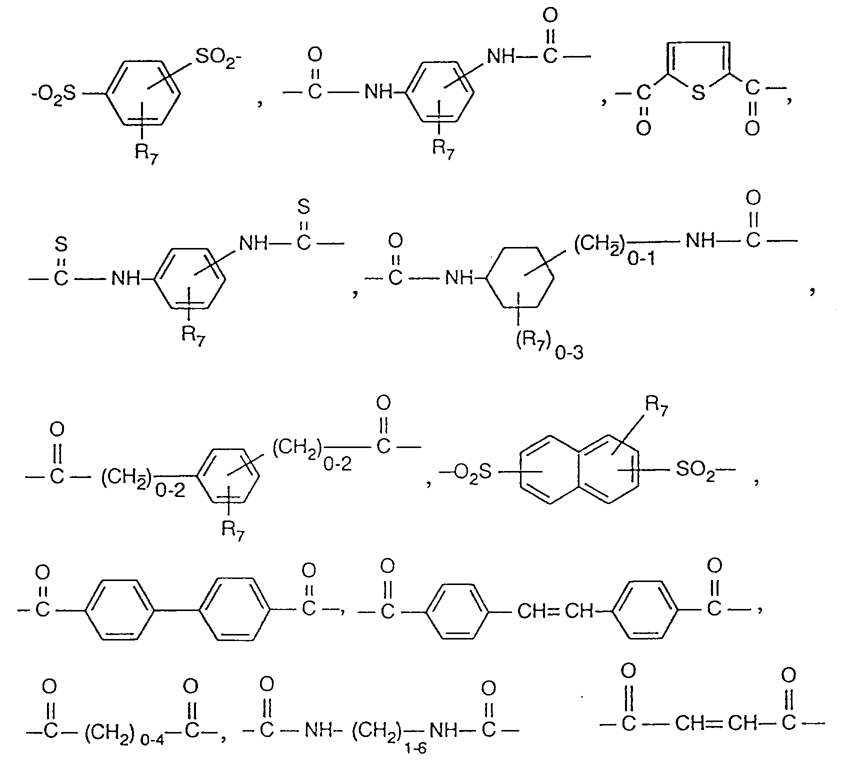 où R₇ représente un groupe sulfo, méthyle, méthoxy, chloro, carboxy ou, de préférence, un atome d'hydrogène.

17. Composés conformes à la revendication 1 de formule 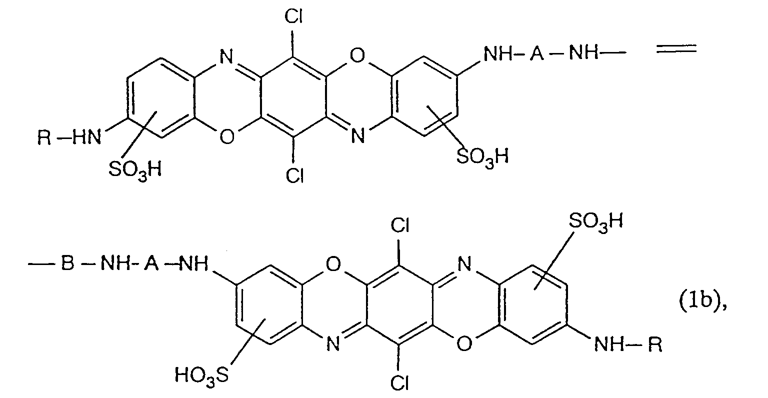 où R représente un groupe méthyle, éthyle, benzyle ou en particulier un atome d'hydrogène, A représente un résidu 1,2-éthylène ou 1,2- ou 1,3-propylène non substitué ou portant un substituant hydroxy ou sulfato et B représente un résidu de formule

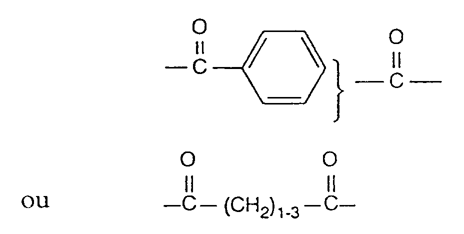

18. Procédé de préparation de composés de formule (1), caractérisé en ce que l'on effectue une réaction de condensation entre un composé de formule 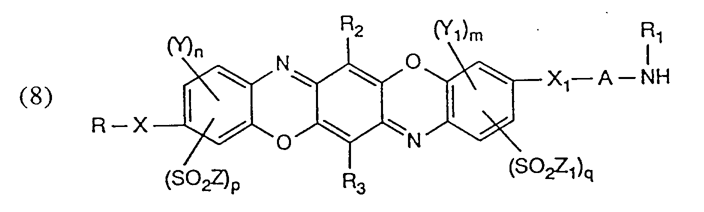 et un composé de formule
T-B-T (9)
dans lesquelles A, B, R, R₁, R₂, R₃, X, X₁, Y, Y₁, Z, Z₁, m, n, p et q ont chacun la signification indiquée dans la revendication 1 et T représente un atome d'halogène, de préférence un atome de chlore.

19. Mélanges de colorants que l'on peut obtenir par réaction de condensation d'un composé de formule (8) conforme à la revendication 18 et d'un composé de formule 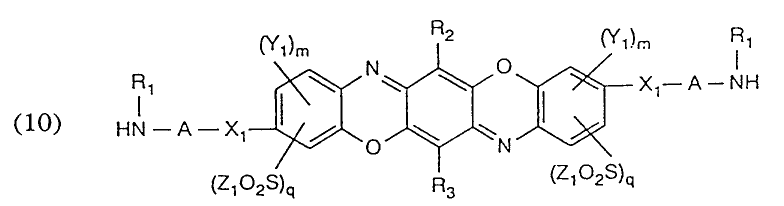 dans laquelle A, R₁, R₂, R₃, X₁, Y₁, Z, m et q ont la signification indiquée dans la revendication 1, avec un composé de formule (9) conforme à la revendication 18.

20. Mélanges de colorants conformes à la revendication 19, caractérisés en ce que l'on utilise les composés de formule (8) et (10) en un rapport en poids de 90 : 10 à 60 : 40, de préférence de 80 : 20 à 65 : 35.

21. Utilisation des composés de formule (1) ainsi que des mélanges de composés que l'on peut obtenir en faisant réagir entre eux les composés de formule (8), (9) et (10) conformes à la revendication 19 en tant que colorants pour la teinture et l'impression de matériaux fibreux contenant des atomes d'azote, en particulier de matériaux fibreux cellulosiques.

22. Utilisation conforme à la revendication 21, caractérisée en ce que l'on teint des mélanges de fibres contenant des fibres synthétiques et des matériaux fibreux cellulosiques, en particulier des tissus mixtes de type polyester/coton, en présence d'un colorant de dispersion pour les fibres de polyester dans les conditions de teinture de fibres de polyester.

23. Procédé de teinture de tissus mixtes en polyester/coton avec des colorants de dispersion et des colorants directs, caractérisé en ce que l'on utilise dans un procédé à étape unique et à bain unique en plus des colorants de dispersion les composés de formule (1) conformes à la revendication 1 ou les mélanges de composés que l'on peut obtenir conformément à la revendication 19, en tant que colorant, et en ce que la teinture se fait dans des bains aqueux à des températures allant de 100 à 150 °C, de préférence de 120 à 130 °C et à un pH allant de 4 à 7,5.

## Revendications (Revendications pour l'(les) Etat(s) contractant(s) suivant(s): ES)

1. Procédé de préparation de composés de formule 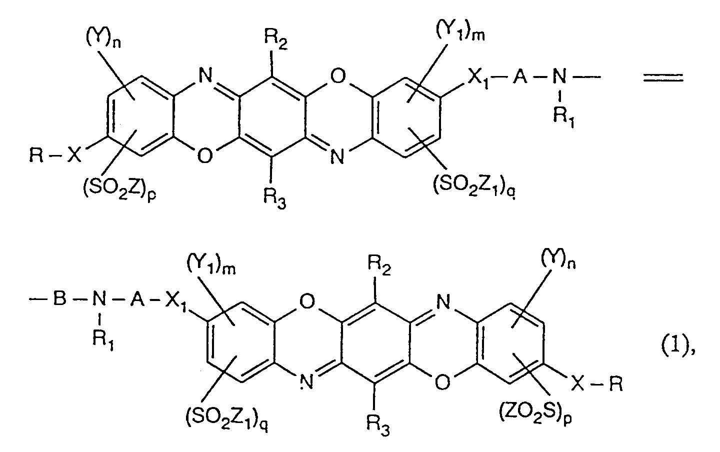 dans laquelle
R et R₁ représentent, indépendamment l'un de l'autre, un atome d'hydrogène: un résidu alkyle en C₁₋₄ non substitué ou portant un substituant hydroxy, sulfo, sulfato, chloro, cyano ou acétoxy et/ou, à l'exception du groupe méthyle, pouvant être interrompu par un -O-; un résidu cyclopentyle ou cyclohexyle non substitué ou portant de 1 à 3 substituants méthyle; un résidu phényle non substitué ou portant un ou plusieurs substituants sulfo, nitro, chloro, méthyle, méthoxy, N-méthyl-ou N-éthylamino, N,N-diméthyl- ou N,N-diéthylamino, acétylamino, propionylamino, benzoylamino, méthoxycarbonyle, éthoxycarbonyle, carboxy ou méthylsulfonyle; un résidu 1-naphtyle ou 2-naphtyle non substitué ou portant un ou plusieurs résidus sulfo, nitro et/ou chloro; ou un résidu benzyle non substitué ou portant un ou plusieurs substituants méthyle, méthoxy, sulfo et/ou chloro;
X représente une liaison directe, un groupe -O-, -S- ou -N(R₄)-où R₄ a la signification indiquée ci-dessus pour R et R₁,
X₁ représente un groupe -O-, -S- ou -N(R₄)- où R₄ a la signification indiquée ci-dessus pour R et R₁,
Y et Y₁ représentent, indépendamment l'un de l'autre, chacun un groupe alkyle en C₁₋₄, alcoxy en C₁₋₄, halogéno, sulfo, carboxy, carbamoyle, N-mono- ou N,N-di(alkyle en C₁₋₄)-carbamoyle, N-phényl-ou N,N-diphénylcarbamoyle, sulfamoyle, N-mono- ou N,N-di(alkyle en C₁₋₄)-sulfamoyle ou N-phényl- ou N,N-diphénylsulfamoyle,
Z et Z₁ représentent, indépendamment l'un de l'autre, chacun un résidu hydroxy ou alkyle en C₁₋₄,
R₂ et R₃ représentent, indépendamment l'un de l'autre, un atome d'hydrogène ou d'halogène, un groupe cyano, alkyle en C₁₋₄, alcoxy en C₁₋₄, un groupe phényle, benzyle, benzoylamino ou phénoxy éventuellement substitués par un ou des groupes alkyle en C₁₋₄, alcoxy en C₁₋₄, acétylamino, halogéno, nitro ou sulfo; un groupe sulfo, carboxy, carbamoyle, phénylcarbamoyle ou alcanoylamino en C₂₋₅,
A représente un groupe alkylène en C₂₋₆ non substitué ou portant un ou des résidus hydroxy, sulfo, sulfato, alcoxy en C₁₋₄, carboxy, cyano, halogéno, phényle, sulfophényle ou alcoxycarbonyle en C₂₋₅ et éventuellement interrompu par 1 ou 2 groupes -O-, -S-, SO₂- ou -N(R₅)- où R₅ représente un groupe alkyle en C₁₋₄, acétyle ou un atome d'hydrogène, ou un groupe cyclopentylène ou cyclohexylène non substitué ou portant un ou plusieurs substituants alkyle en C₁₋₃; ou un résidu phénylène, biphénylène ou naphtylène non substitué ou portant un ou des substituants alkyle en C₁₋₄, alcoxy en C₁₋₄, sulfo, halogéno ou carboxy; ou un groupe (alkylène en C₁₋₆)-phénylène, phénylène-(alkylène en C₁₋₆)-phénylène, (alkylène en C₁₋₃)-phénylène-(alkylène en C₁₋₃) ou méthylène-naphtylène-méthylène, les résidus phénylène ou naphtylène ne portant pas d'autres substituants ou 1 ou 2 substituants choisis parmi les résidus sulfo, carboxy, sulfamoyle, carbamoyle, méthyle, éthyle, méthoxy, éthoxy, nitro, chloro, amino, N-méthyl- et N-éthylamino, N,N-diméthyl-et N,N-diéthylamino et phénylamino,
B représente un résidu de formule
Q-E-Q (2')
dans laquelle Q représente un groupe de formule 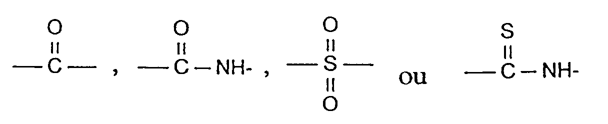 et E représente une liaison directe; un résidu alkylène en C₁₋₆ ou alcénylène en C₂₋₆ non substitué ou portant un ou plusieurs résidus hydroxy, sulfo, sulfato, méthoxy, carboxy, phényle ou sulfophényle; un résidu cyclohexylène ou (alkylène en C₁₋₂)-cyclohexylène non substitué ou portant de 1 à 3 groupes méthyle; un résidu pipérazin-1,4-diyle; thiophène-2,5-diyle; biphényl-4,4-diyle; stilbène-4,4'-diyle; phénylène ou naphtylène non substitué ou portant des substituants alkyle en C₁₋₄, alcoxy en C₁₋₄, sulfo, halogéno ou carboxy; ou un résidu (alkylène en C₁₋₃)-phénylène ou (alkylène en C₁₋₂)-phénylène-(alkylène en C₁₋₂) non substitués ou portant sur la partie phényle des substituants méthyle, méthoxy, chloro ou sulfo, et m, n, p et q valent indépendamment l'un de l'autre chacun 0 ou 1,
caractérisé en ce que l'on effectue une réaction de condensation entre un composé de formule 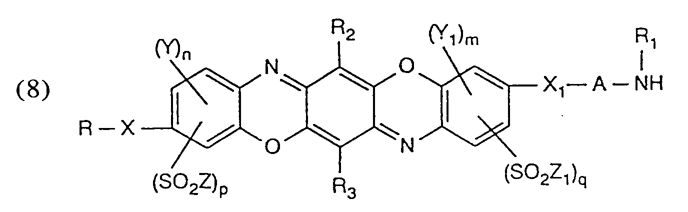 et un composé de formule
T-B-T (9)
dans lesquelles A, B, R, R₁, R₂, R₃, X, X₁, Y, Y₁, Z, Z₁, m, n, p et q ont chacun la signification indiquée dans la revendication 1 et T représente un atome d'halogène, de préférence un atome de chlore.

2. Procédé conforme à la revendication 1, caractérisé en ce que l'on utilise un composé de formule (8) dans lequel R et R₁ représentent indépendamment l'un de l'autre chacun un atome d'hydrogène, un groupe alkyle en C₁₋₄, cyclohexyle, phényle ou benzyle non substitué ou portant un ou plusieurs substituants sulfo, chloro, méthyle, et/ou méthoxy.

3. Procédé conforme à une des revendications 1 et 2, caractérisé en ce que l'on utilise un composé de formule (8) dans lequel R et R₁ représentent chacun un atome d'hydrogène.

4. Procédé conforme à une des revendications 1 à 3, caractérisé en ce que l'on utilise un composé de formule (8) dans lequel A représente un résidu alkylène en C₂₋₄ non substitué ou portant un ou plusieurs résidus hydroxy, sulfo, sulfato, méthoxy, carboxy ou sulfophényle, un groupe - CH₂-CH₂-Z'-CH₂-CH₂- où Z' représente un groupe -O-, -S-, -SO₂-, -NH-ou -N(CH₃)-, un résidu cyclohexylène non substitué ou portant de 1 à 3 résidus méthyle, un résidu 1,3-phénylène ou 1,4-phénylène non substitué ou portant un résidu sulfo, ou un résidu (alkylène en C₁₋₃)-phénylène ou (alkylène en C₁₋₂)-phénylène-(alkylène en C₁₋₂) à groupe phénylène non substitué ou portant un résidu méthyle, méthoxy, chloro ou sulfo.

5. Procédé conforme à une des revendications 1 à 4, caractérisé en ce que l'on utilise un composé de formule (8) dans lequel A représente un groupe alkylène en C₂₋₄ non substitué ou portant un ou plusieurs substituants hydroxy, sulfo, sulfato, méthoxy, carboxy ou sulfophényle.

6. Procédé conforme à une des revendications 1 à 5, caractérisé en ce que l'on utilise un composé de formule (8) dans lequel A représente un résidu 1,2-éthylène ou 1,2- ou 1,3-propylène non substitué ou portant un substituant hydroxy ou sulfato.

7. Procédé conforme à une des revendications 1 à 6, caractérisé en ce que l'on utilise un composé de formule (8) dans lequel X représente un groupe -N(R₄)- où R₄ représente un atome d'hydrogène, un groupe alkyle en C₁₋₄, cyclohexyle, phényle ou benzyle non substitué ou portant un ou plusieurs substituants sulfo, chloro, méthyle et/ou méthoxy.

8. Procédé conforme à la revendication 7, caractérisé en ce que l'on utilise un composé de formule (8) dans lequel R₄ représente un atome d'hydrogène ou un groupe alkyle en C₁₋₄, en particulier un atome d'hydrogène.

9. Procédé conforme à une des revendications 1 à 8, caractérisé en ce que l'on utilise un composé de formule (8) dans lequel m et n représentent tous deux le nombre 0.

10. Procédé conforme à une des revendications 1 à 9, caractérisé en ce que l'on utilise un composé de formule (8) dans lequel p et q sont tous deux égaux à 1 et Z et Z₁ représentent chacun un groupe hydroxy ou alkyle en C₁₋₄, en particulier un groupe hydroxy.

11. Procédé conforme à une des revendications 1 à 10, caractérisé en ce que l'on utilise un composé de formule (8) dans lequel R₂ et R₃ représentent chacun un atome d'hydrogène, de fluor, de chlore ou de brome, un groupe méthyle, méthoxy, acétylamino, phénoxy ou cyano.

12. Procédé conforme à une des revendications 1 à 11, caractérisé en ce que l'on utilise un composé de formule (8) dans lequel R₂ et R₃ représentent chacun un atome de chlore.

13. Procédé conforme à une des revendications 1 à 12, caractérisé en ce que l'on utilise un composé de formule (9) dans lequel B est un élément pontant de formule 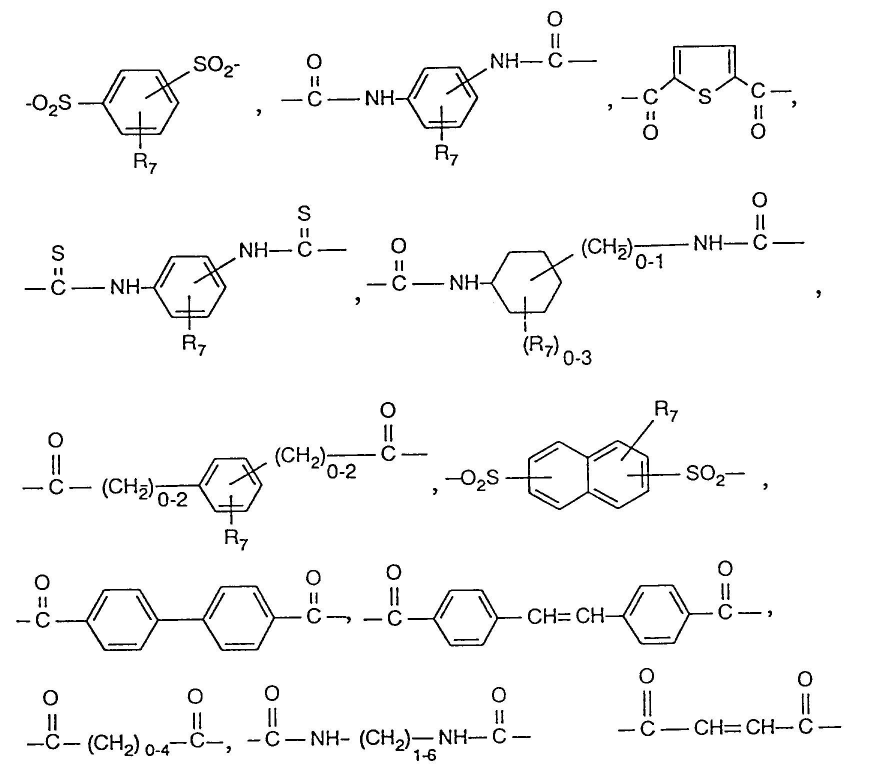 où R₇ représente un groupe sulfo, méthyle, méthoxy, chloro, carboxy ou, de préférence, un atome d'hydrogène.

14. Procédé conforme à une des revendications 1 à 13, caractérisé en ce que l'on utilise un composé de formule (9) dans lequel B représente un résidu de formule

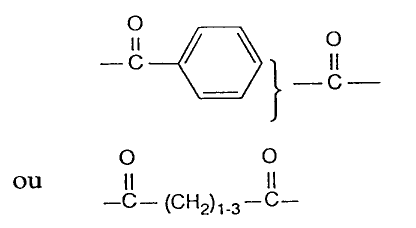

15. Procédé conforme à la revendication 1, caractérisé en ce que l'on utilise un composé de formule (8) dans lequel R et R₁ représentent indépendamment l'un de l'autre un atome d'hydrogène; un groupe alkyle en C₁₋₄ non substitué ou portant un ou plusieurs résidus hydroxy, sulfo, sulfato, chloro, cyano ou acétoxy et/ou, à l'exception du groupe méthyle, pouvant être interrompu par un groupe -O-; un résidu cyclopentyle ou cyclohexyle non substitué ou portant de 1 à 3 substituants méthyle; un résidu phényle non substitué ou portant un ou plusieurs substituants sulfo, nitro, chloro, méthyle, méthoxy, N-méthyl- ou N-éthylamino, N,N-diméthyl- ou N,N-diéthylamino, acétylamino, propionylamino, benzoylamino, méthoxycarbonyle, éthoxycarbonyle, carboxy ou méthylsulfonyle; un groupe 1-naphtyle ou 2-naphtyle non substitué ou portant des groupes sulfo, nitro et/ou chloro; ou encore un groupe benzyle non substitué ou portant des substituants méthyle, méthoxy, sulfo et/ou chloro;
A représente un résidu alkylène en C₂₋₄ non substitué ou portant un résidu hydroxy, sulfo, sulfato, méthoxy, carboxy ou sulfophényle, un groupe -CH₂-CH₂-Z'-CH₂-CH₂- où Z' représente un groupe -O-, -S-, -SO₂-, -NH- ou -N(CH₃)-, un résidu cyclohexylène non substitué ou portant de 1 à 3 résidus méthyle, un résidu 1,3-phénylène ou 1,4-phénylène non substitué ou portant un résidu sulfo, ou un résidu (alkylène en C₁₋₃)-phénylène ou (alkylène en C₁₋₂)-phénylène-(alkylène en C₁₋₂) à groupe phénylène non substitué ou portant un ou plusieurs résidus méthyle, méthoxy, chloro ou sulfo;
X et X₁ représentent, indépendamment l'un de l'autre, un groupe -N(R₄)- où R₄ représente un atome d'hydrogène, un groupe alkyle en C₁₋₄, cyclohexyle, phényle ou benzyle non substitués ou portant des résidus sulfo, chloro, méthyle et/ou méthoxy,
Y et Y₁ représentent indépendamment l'un de l'autre chacun un groupe méthoxy, méthyle, sulfo ou chloro;
n et m représentent indépendamment chacun le nombre 0 ou 1;
Z et Z₁ représentent, indépendamment l'un de l'autre, un groupe hydroxy, méthyle ou éthyle,
p et q sont tous deux égaux à 1;
R₂ et R₃ représentent chacun un atome d'hydrogène, de fluor, de chlore, de brome, un groupe méthyle, méthoxy, acétylamino, phénoxy ou cyano.

16. Procédé conforme à la revendication 1, caractérisé en ce que l'on condense un composé de formule 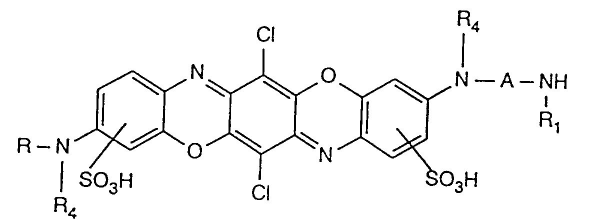 dans lesquelles R et R₁ représentent, indépendamment l'un de l'autre, un atome d'hydrogène, un groupe alkyle en C₁₋₄, cyclohexyle, phényle ou benzyle non substitués ou portant des substituants sulfo, chloro, méthyle et/ou méthoxy,
A représente un résidu alkylène en C₂₋₄ non substitué ou portant un ou plusieurs substituants hydroxy, sulfo, sulfato, méthoxy, carboxy ou sulfophényle, R₄ représente un atome d'hydrogène, un groupe méthyle ou éthyle,
avec un composé de formule (9) dans lequel B représente un résidu de formule 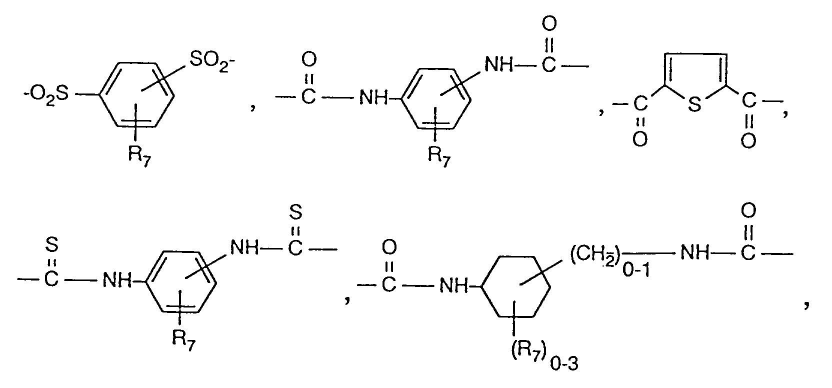 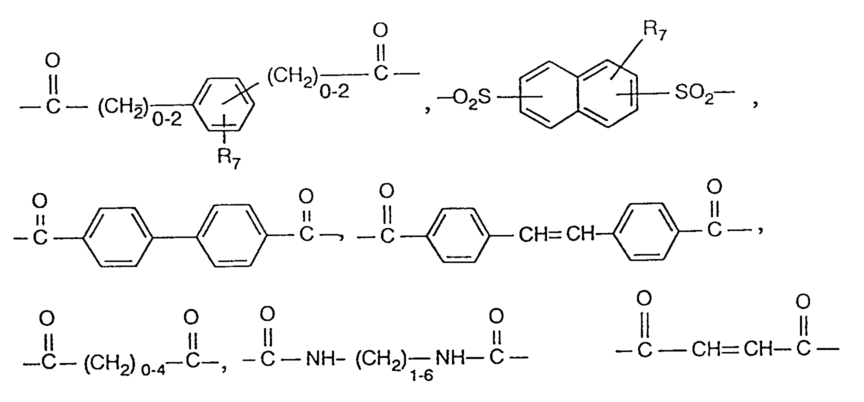 où R₇ représente un groupe sulfo, méthyle, méthoxy, chloro, carboxy ou, de préférence, un atome d'hydrogène.

17. Procédé conforme à la revendication 1, caractérisé en ce que l'on condense un composé de formule 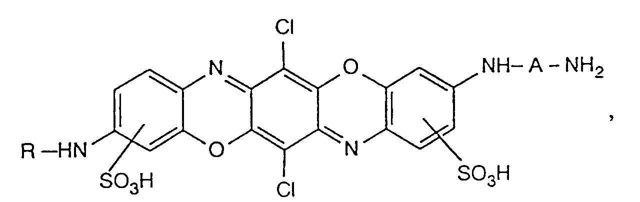 où R représente un groupe méthyle, éthyle, benzyle ou en particulier un atome d'hydrogène, A représente un résidu 1,2-éthylène ou 1,2- ou 1,3-propylène non substitué ou portant un substituant hydroxy ou sulfato avec un composé de formule (9) dans lequel B représente un résidu de formule

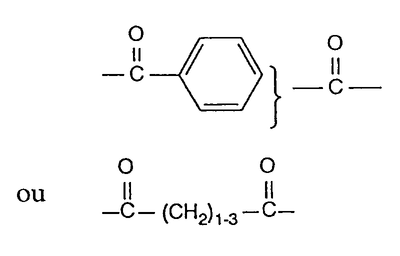

18. Procédé de préparation de mélanges de colorants, caractérisé en ce que l'on condense un composé de formule (8) conforme à la revendication 1 et un composé de formule 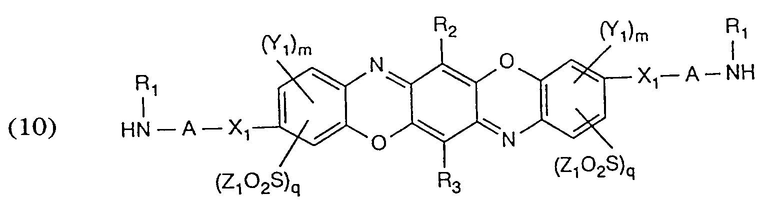 dans laquelle A, R₁, R₂, R₃, X₁, Y₁, Z, m et q ont la signification indiquée dans la revendication 1, avec un composé de formule (9) conforme à la revendication 1.

19. Procédé de préparation de mélanges de colorants conforme à la revendication 18, caractérisé en ce que l'on utilise les composés de formule (8) et (10) en un rapport en poids de 90 : 10 à 60 : 40, de préférence de 80 : 20 à 65 : 35.

20. Composés de formule 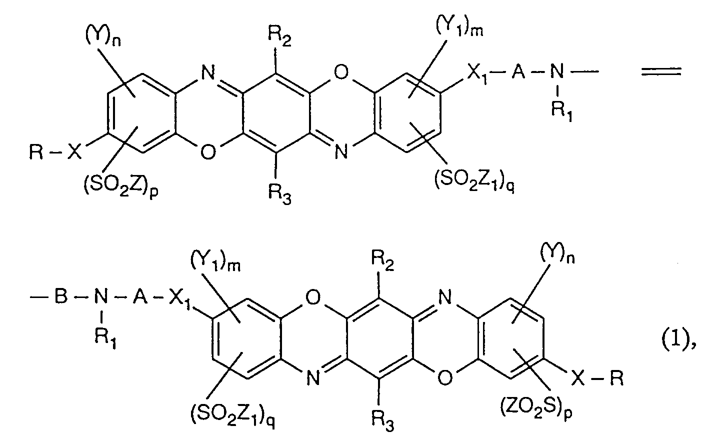 dans laquelle
R et R₁ représentent, indépendamment l'un de l'autre, un atome d'hydrogène; un résidu alkyle en C₁₋₄ non substitué ou portant un substituant hydroxy, sulfo, sulfato, chloro, cyano ou acétoxy et/ou, à l'exception du groupe méthyle, pouvant être interrompu par un -O-; un résidu cyclopentyle ou cyclohexyle non substitué ou portant de 1 à 3 substituants méthyle; un résidu phényle non substitué ou portant un ou plusieurs substituants sulfo, nitro, chloro, méthyle, méthoxy, N-méthyl-ou N-éthylamino, N,N-diméthyl- ou N,N-diéthylamino, acétylamino, propionylamino, benzoylamino, méthoxycarbonyle, éthoxycarbonyle, carboxy ou méthylsulfonyle; un résidu 1-naphtyle ou 2-naphtyle non substitué ou portant un ou plusieurs résidus sulfo, nitro et/ou chloro; ou un résidu benzyle non substitué ou portant un ou plusieurs substituants méthyle, méthoxy, sulfo et/ou chloro;
X représente une liaison directe, un groupe -O-, -S- ou -N(R₄)-où R₄ a la signification indiquée ci-dessus pour R et R₁,
X₁ représente un groupe -O-, -S- ou -N(R₄)- où R₄ a la signification indiquée ci-dessus pour R et R₁,
Y et Y₁ représentent, indépendamment l'un de l'autre, chacun un groupe alkyle en C₁₋₄, alcoxy en C₁₋₄, halogéno, sulfo, carboxy, carbamoyle, N-mono- ou N,N-di(alkyle en C₁₋₄)-carbamoyle, N-phényl-ou N,N-diphénylcarbamoyle, sulfamoyle, N-mono- ou N,N-di(alkyle en C₁₋₄)-sulfamoyle ou N-phényl- ou N,N-diphénylsulfamoyle,
Z et Z₁ représentent, indépendamment l'un de l'autre, chacun un résidu hydroxy ou alkyle en C₁₋₄,
R₂ et R₃ représentent, indépendamment l'un de l'autre, un atome d'hydrogène ou d'halogène, un groupe cyano, alkyle en C₁₋₄, alcoxy en C₁₋₄, un groupe phényle, benzyle, benzoylamino ou phénoxy éventuellement substitués par un ou des groupes alkyle en C₁₋₄, alcoxy en C₁₋₄, acétylamino, halogéno, nitro ou sulfo; un groupe sulfo, carboxy, carbamoyle, phénylcarbamoyle ou alcanoylamino en C₂₋₅,
A représente un groupe alkylène en C₂₋₆ non substitué ou portant un ou des résidus hydroxy, sulfo, sulfato, alcoxy en C₁₋₄, carboxy, cyano, halogéno, phényle, sulfophényle ou alcoxycarbonyle en C₂₋₅ et éventuellement interrompu par 1 ou 2 groupes -O-, -S-, SO₂- ou -N(R₅)- où R₅ représente un groupe alkyle en C₁₋₄, acétyle ou un atome d'hydrogène, ou un groupe cyclopentylène ou cyclohexylène non substitué ou portant un ou plusieurs substituants alkyle en C₁₋₃; ou un résidu phénylène, biphénylène ou naphtylène non substitué ou portant un ou des substituants alkyle en C₁₋₄, alcoxy en C₁₋₄, sulfo, halogéno ou carboxy; ou un groupe (alkylène en C₁₋₆)-phénylène, phénylène-(alkylène en C₁₋₆)-phénylène, (alkylène en C₁₋₃)-phénylène-(alkylène en C₁₋₃) ou méthylène-naphtylène-méthylène, les résidus phénylène ou naphtylène ne portant pas d'autres substituants ou 1 ou 2 substituants choisis parmi les résidus sulfo, carboxy, sulfamoyle, carbamoyle, méthyle, éthyle, méthoxy, éthoxy, nitro, chloro, amino, N-méthyl- et N-éthylamino, N,N-diméthyl-et N,N-diéthylamino et phénylamino,
B représente un résidu de formule
Q-E-Q (2')
dans laquelle Q représente un groupe de formule 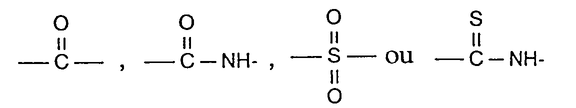 et E représente une liaison directe; un résidu alkylène en C₁₋₆ ou alcénylène en C₂₋₆ non substitué ou portant un ou plusieurs résidus hydroxy, sulfo, sulfato, méthoxy, carboxy, phényle ou sulfophényle; un résidu cyclohexylène ou (alkylène en C₁₋₂)-cyclohexylène non substitué ou portant de 1 à 3 groupes méthyle; un résidu pipérazin-1,4-diyle; thiophène-2,5-diyle; biphényl-4,4-diyle; stilbène-4,4'-diyle; phénylène ou naphtylène non substitué ou portant des substituants alkyle en C₁₋₄, alcoxy en C₁₋₄, sulfo, halogéno ou carboxy; ou un résidu (alkylène en C₁₋₃)-phénylène ou (alkylène en C₁₋₂)-phénylène-(alkylène en C₁₋₂) non substitués ou portant sur la partie phényle des substituants méthyle, méthoxy, chloro ou sulfo, et m, n, p et q valent indépendamment l'un de l'autre chacun 0 ou 1.

21. Mélanges de colorants que l'on peut obtenir par condensation d'un composé de formule (8) conforme à la revendication 1 et d'un composé de formule 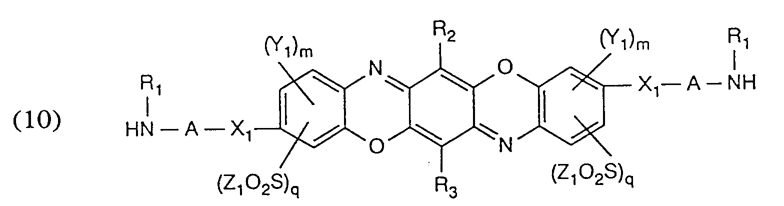 dans laquelle A, R₁, R₂, R₃, X₁, Y₁, Z, m et q ont la signification indiquée dans la revendication 1, avec un composé de formule (9) conforme à la revendication 1.

22. Utilisation des composés de formule (1) que l'on peut obtenir conformément à la revendication 1 ainsi que des mélanges de composés que l'on peut obtenir en faisant réagir entre eux les composés de formule (8), (9) et (10) conformément à la revendication 18, en tant que colorants pour la teinture et l'impression de matériaux fibreux contenant des atomes d'azote, en particulier de matériaux fibreux cellulosiques.

23. Utilisation conforme à la revendication 22, caractérisée en ce que l'on teint des mélanges de fibres contenant des fibres synthétiques et des matériaux fibreux cellulosiques, en particulier des tissus mixtes de type polyester/coton, en présence d'un colorant de dispersion pour les fibres de polyester dans les conditions de teinture de fibres de polyester.

24. Procédé de teinture de tissus mixtes en polyester/coton avec des colorants de dispersion et des colorants directs, caractérisé en ce que l'on utilise dans un procédé à étape unique et à bain unique, en plus des colorants de dispersion, les composés de formule (1) conformes à la revendication 1 ou les mélanges de composés que l'on peut obtenir conformément à la revendication 18, en tant que colorants, et en ce que la teinture se fait dans des bains aqueux à des températures allant de 100 à 150 °C, de préférence de 120 à 130 °C et à un pH allant de 4 à 7,5.
